# FASCICULE DE BREVET EUROPEEN

(11) **EP 2 260 113 B1**
(45) Date de publication et mention de la délivrance du brevet: **11.07.2012**
(21) Numéro de dépôt: 09754074.4
(22) Date de dépôt: 30.03.2009
(51) Int. Cl.: C12Q 1/68

(54) **PROCEDE ET METHODES DE DIAGNOSTIC DE LA MALADIE D'ALZHEIMER**
VORGEHENSWEISE UND VERFAHREN FÜR DIE DIAGNOSE VON MORBUS ALZHEIMER
PROCESS AND METHOD FOR DIAGNOSING ALZHEIMER'S DISEASE

(30) Priorité: 28.03.2008 FR 0852042; 11.04.2008 US 44097 P
(43) Date de publication de la demande: 15.12.2010
(73) Titulaire: EXONHIT SA, 75013 Paris (FR)
(72) Inventeur: JARRIGE-LE PRADO, Anne-Charlotte, F-75014 Paris (FR); BEURDELEY, Pascale, F-94120 Fontenay Sous Bois (FR); PALLARES, Diego, F-75014 Paris (FR); ROUET, Fabien, 69290 Craponne (FR); BRACCO, Laurent, F-92250 La Garenne Colombes (FR)
(74) Mandataire: Becker, Philippe
(86) Numéro de dépôt international: PCT/FR2009/050530
(87) Numéro de publication internationale: WO 2009/144424

(56) Documents cités:
- WO-A-2004/112589
- WO-A-2005/020784
- WO-A-2006/020269
- WO-A-2009/074331
- FR-A- 2 900 936
- BEURDELEY P ET AL: "P1-343: Discovery of blood gene expression biomarkers in Alzheimer's disease using the human genome-wide splice array" ALZHEIMER'S & DEMENTIA: THE JOURNAL OF THE ALZHEIMER'SASSOCIATION, ELSEVIER, NEW YORK, NY, US, vol. 4, no. 4, 1 juillet 2008 (2008-07-01), page T319, XP023173856 ISSN: 1552-5260 [extrait le 2008-07-01]
- MAES ET AL: "Transcriptional profiling of Alzheimer blood mononuclear cells by microarray" NEUROBIOLOGY OF AGING, TARRYTOWN, NY, US, vol. 28, no. 12, 12 octobre 2007 (2007-10-12), pages 1795-1809, XP022295299 ISSN: 0197-4580
- RAY SANDIP ET AL: "Classification and prediction of clinical Alzheimer's diagnosis based on plasma signaling proteins" NATURE MEDICINE, vol. 13, no. 11, novembre 2007 (2007-11), pages 1359-1362, XP002544276 ISSN: 1078-8956
- WILMOT ET AL: "Translational gene mapping of cognitive decline" NEUROBIOLOGY OF AGING, TARRYTOWN, NY, US, vol. 29, no. 4, 3 mars 2008 (2008-03-03), pages 524-541, XP022509457 ISSN: 0197-4580

## Description

La présente demande concerne des méthodes et compositions utilisables pour la détection de la maladie d'Alzheimer chez les mammifères, en particulier les humains. Elle décrit notamment des marqueurs sériques de la maladie d'Alzheimer et leurs utilisations dans des méthodes de diagnostic. Elle concerne également des outils et/ou kits utilisables pour la mise en oeuvre de ces méthodes (réactifs, sondes, amorces, anticorps, puces, cellules, etc.), leur préparation et leurs utilisations. L'invention est utilisable pour détecter la présence ou l'évolution de la maladie d'Alzheimer chez les mammifères, y compris en phase précoce.

La maladie d'Alzheimer représente la principale cause de démence et la maladie neurodégénérative la plus fréquente. Cette maladie, d'évolution progressive, est caractérisée par la perte de mémoire et par une dégradation des aptitudes au langage, à l'orientation et au jugement. La nature des symptômes, souvent confondus avec les désagréments physiologiques liés à la vieillesse, leur sévérité ainsi que l'âge de leurs apparitions, varient selon les individus. Ceci contribue à la difficulté d'établir un diagnostic aux stades précoces de la maladie.

L'examen de cerveaux de patients atteints de cette maladie révèle une perte de neurones de l'hippocampe, centre important de la mémoire, et du cortex cérébral, impliqué dans le raisonnement, le langage et la mémoire. Les neurones cholinergiques sont particulièrement affectés par cette déplétion.

Une autre anomalie majeure observée dans les cerveaux des malades atteints de la maladie d'Alzheimer est l'accumulation d'agrégats intracellulaires et extracellulaires de protéines. Des agrégats neurofibrillaires intracellulaires de la protéine tau semblent bien corrélés avec la gravité de la démence. Des plaques séniles formées par agrégation intra et extracellulaire de peptide beta-amyloïde caractérisent des régions d'altérations de neurones et de cellules gliales.

Il est cependant remarquable de noter que ces zones d'agrégation ne correspondent pas aux sites de la déplétion en synapses caractéristique du déclin des fonctions cognitives.

Les études génétiques menées sur les formes familiales ont montré que 4 gènes sont associés au développement de la maladie. L'APP (Amyloid Precursor Protein ; précurseur du peptide beta amyloide), les présénilines 1 et 2 (PS1 et PS2) et l'apolipoprotéine E (ApoE). Bien que des mutations ou polymorphismes dans chacun de ces gènes aboutissent à une production accrue de peptide beta amyloïde, les mécanismes qui président aux pertes synaptiques et neuronales restent mal connus. A cet égard, plusieurs hypothèses et mécanismes semblent ainsi coexister, qui impliquent différents phénomènes:
1 Des phénomènes purement cérébraux impliquant les neurones et les cellules gliales:
   - le stress oxydatif, qui peut être induit notamment par le peptide beta amyloïde et modulé par le métabolisme du cholestérol;
   - des modifications de flux calciques et l'excitotoxicité.
2 Des phénomènes inflammatoires et immunitaires réactionnels ;
3 Une altération des hormones sexuelles ;
4 L'hypothyroïdisme et des défauts dans la régulation des signaux insuliniques.

Par conséquent, la maladie d'Alzheimer serait caractérisée par une altération de différents systèmes d'intégration régulant l'homéostasie, l'atteinte de certains neurones entraînant à la fois une réaction inflammatoire impliquant le système immunitaire et des modifications des régulations endocriniennes. Ces dernières ont en retour un impact sur l'activité et la viabilité d'autres neurones et sur les fonctions immunitaires, ces réactions en cascade soulignant le rôle non seulement de la neurodégénérescence mais aussi des régulations hormonales et de la réponse immunitaire dans la progression de la maladie d'Alzheimer.

De marqueurs seriques de la maladie d'Alzheimer sont décrit dans FR 2 900 936, Olivier C. Maes et al.: "Transcriptional profiling of Alzheiemr blood mononuclear cells by microarray", Neurobiology of Aging 28 (2007) 1795-1809, WO 2005/ 020784, WO 2006/020269, WO 2004/112589 et Ray et al.: "Classification and prediction of clinical Alzheimer's diagnosis based on plasma signaling proteins", nature medicine, 13(11) 1359-1362.

Il n'existe actuellement aucune signature robuste et spécifique de la maladie d'Alzheimer, notamment à partir d'échantillon sanguin, susceptible de permettre d'établir un diagnostic de cette pathologie, notamment des différents stades de l'évolution de la maladie. La mise à disposition d'un test de diagnostic efficace, notamment précoce, permettrait aux patients d'être pris en charge dès le début de la maladie, et de bénéficier ainsi d'un traitement plus efficace et plus adapté, notamment par des inhibiteurs d'acétylcholinestérase tels que la galantamine, le donepezil et la rivastigmine, dans des conditions optimales.

La présente invention apporte une réponse à ce besoin. L'invention décrit notamment l'identification de marqueurs sériques de la maladie d'Alzheimer, permettant la mise au point de diagnostics efficaces et prédictifs de la présence de cette maladie. L'invention décrit ainsi l'identification de signatures moléculaires spécifiquement ou préférentiellement exprimées dans le sang de patients atteints de la maladie d'Alzheimer, résultant notamment de l'expression complexe de certains gènes liée à des épissages alternatifs. Notamment l'identification des séquences 1-5578, qui sont présentes dans des ARN de cellules sanguines de sujets humains, et qui sont caractéristiques, en combinaisons, de la maladie d'Alzheimer est décrite. L'invention peut donc proposer, pour la première fois, des outils et méthodes de diagnostic, de la maladie d'Alzheimer, basés sur une mesure, dans le sang de sujets, de l'expression de plusieurs gènes. La présence d'une dérégulation dans l'expression de tels gènes permet de confirmer la présence de cette pathologie chez un sujet.

Un objet de l'invention réside ainsi dans une méthode pour détecter (in vitro ou ex vivo) la présence de la maladie d'Alzheimer chez un mammifère, comprenant la détermination de la présence, dans un échantillon biologique du mammifère, de préférence dans un échantillon (dérivé) de sang, d'une altération dans plusieurs gènes ou ARN comprenant une séquence choisie parmi SEQ ID Nos : 1 à 5578, la présence d'une telle altération étant indicative de la présence ou du risque de développer la maladie d'Alzheimer chez ce mammifère.

Également décrite est une méthode pour détecter (in vitro ou ex vivo) la capacité de développer la maladie d'Alzheimer chez un mammifère atteint de Troubles Cognitifs Légers ou à des stades asymptomatique d'Alzheimer (Mild Cognitive Impairements), notamment de type hippocampique ou amnésique, comprenant la détermination de la présence, dans un échantillon biologique du mammifère, de préférence dans un échantillon (dérivé) de sang, d'une altération dans plusieurs gènes ou ARN comprenant une séquence choisie parmi SEQ ID Nos : 1 à 5578, la présence d'une telle altération étant indicative du risque de développer la maladie d'Alzheimer chez ce mammifère

Un autre objet de la description concerne une méthode pour évaluer ou suivre la réponse à un traitement de la maladie d'Alzheimer, comprenant une étape de mesure de l'expression d'un ou, de préférence, de plusieurs gènes ou ARN comprenant une séquence choisie parmi SEQ ID Nos : 1 à 5578 avant et/ou au cours du traitement, et une comparaison de l'expression ainsi mesurée à celle mesurée à un stade antérieur du traitement ou au traitement.

Une amélioration aux méthodes de traitement de la maladie d'Alzheimer, l'amélioration consistant à mesurer l'expression d'un ou, de préférence, de plusieurs gènes ou ARN comprenant une séquence choisie parmi SEQ ID Nos : 1 à 5578, chez un sujet, avant et/ou pendant le traitement est décrite. La mesure de l'expression permet d'adapter le traitement en fonction de l'évolution de la pathologie. Le traitement est typiquement un traitement par des inhibiteurs d'acétylcholinestérase, tels que la galantamine, le donepezil et la rivastigmine.

Un autre objet de l'invention concerne l'utilisation d'un inhibiteur d'acétylcholinestérase, tel que la galantamine, le donepezil et la rivastigmine, pour la préparation d'un médicament pour traiter la maladie d'Alzheimer chez un patient présentant une dérégulation de l'expression d'un gène (au moins) tel que défini précédemment.

L'altération dans un gène ou ARN désigne au sens de l'invention (i) toute altération de l'expression, à savoir en particulier une dérégulation dans les niveaux d'expression (e.g., de transcription ou de traduction), une dérégulation de l'épissage, conduisant par exemple à l'apparition de formes épissées particulières ou à une modification de la quantité (relative) ou du rapport entre les différentes formes d'épissage, de même que (ii) toute altération dans la structure de la protéine produite (apparition ou disparition de formes tronquées, allongées, mutées, etc.).

Comme il sera décrit dans la suite du texte, la présente demande décrit l'identification de dérégulations de l'épissage parmi certains gènes dans le sang de patients atteints de la maladie d'Alzheimer. Toute molécule ou technique permettant de mesurer l'expression de ces gènes dans le sang peut être mise en oeuvre dans le cadre de la présente invention, telles que des amorces nucléotidiques, des sondes nucléotidiques ou des anticorps spécifiques, qui peuvent être en suspension ou sous forme immobilisée, comme il sera décrit en détails dans la suite du texte.

Ainsi, un autre objet de la présente demande concerne un produit comprenant un support sur lequel est immobilisé
un ensemble de (groupes de) sondes comprenant au moins:
- une sonde comprenant tout ou partie de la séquence SEQ ID NO : 2363 ou de sa séquence complémentaire;
- une sonde comprenant tout ou partie de la séquence SEQ ID NO : 2364 ou de sa séquence complémentaire;
- une sonde comprenant tout ou partie de la séquence SEQ ID NO : 5307 ou de sa séquence complémentaire;
- une sonde comprenant tout ou partie de la séquence SEQ ID NO : 5426 ou de sa séquence complémentaire;
- une sonde comprenant tout ou partie de la séquence SEQ ID NO : 4218 ou de sa séquence complémentaire;
- une sonde comprenant tout ou partie de la séquence SEQ ID NO : 5063 ou de sa séquence complémentaire.
- une "partie" de séquence désignant une région de 15 à 50 nucléotides consécutifs de cette séquence.

Également fourni est un produit comprenant un support sur lequel est immobilisé au moins un ligand d'un polypeptide codé par un gène ou un ARN tel que défini ci-dessus. De préférence, le produit comprend au moins 5, 10, 20, 30, 40, 50, 60 ou plus ligands de polypeptides différents choisis parmi les polypeptides mentionnés ci-dessus.

Un autre objet de la présente demande concerne un kit comprenant un compartiment ou conteneur comprenant au moins
un ensemble de (groupes de) sondes comprenant au moins:
- une sonde comprenant tout ou partie de la séquence SEQ ID NO : 2363 ou de sa séquence complémentaire;
- une sonde comprenant tout ou partie de la séquence SEQ ID NO : 2364 ou de sa séquence complémentaire;
- une sonde comprenant tout ou partie de la séquence SEQ ID NO : 5307 ou de sa séquence complémentaire;
- une sonde comprenant tout ou partie de la séquence SEQ ID NO : 5426 ou de sa séquence complémentaire;
- une sonde comprenant tout ou partie de la séquence SEQ ID NO : 4218 ou de sa séquence complémentaire;
- une sonde comprenant tout ou partie de la séquence SEQ ID NO : 5063 ou de sa séquence complémentaire.
- une "partie" de séquence désignant une région de 15 à 50 nucléotides consécutifs de cette séquence.
Le kit peut comprendre par ailleurs des réactifs pour une réaction d'hybridation ou immunologique, ainsi que, le cas échéant, des contrôles et/ou instructions.

Un autre objet de l'invention concerne l'utilisation d'un produit ou kit tel que défini ci-dessus pour la détection de la maladie d'Alzheimer chez un sujet mammifère, de préférence un sujet humain.

Est également fournie l'utilisation d'un produit ou kit tel que défini ci-dessus pour la détermination du risque de développer la maladie d'Alzheimer chez un sujet mammifère, de préférence un sujet humain, en particulier chez un sujet atteint de Troubles Cognitifs Légers ou à des stades asymptomatique d'Alzheimer, en particulier de type hippocampique ou amnésique.

Un autre objet de l'invention concerne l'utilisation d'un produit ou kit tel que défini ci-dessus pour discriminer des sujets atteints de la maladie d'Alzheimer de sujets atteints de Troubles Cognitifs Légers.

L'utilisation d'un produit ou kit tel que défini ci-dessus pour la détermination de la réponse àun traitement de la maladie d'Alzheimer ou pour la sélection de sujets susceptibles de répondre de façon efficace à un traitement est également décrite

Également décrite est une méthode de détection ou dépistage de la maladie d'Alzheimer, comprenant la détermination de la présence d'une ou plusieurs molécules cibles choisies parmi :
a) les acide nucléiques comprenant au moins une paire de séquences définies dans le tableau 2 ou un fragment de celles-ci ayant au moins 15, de préférence au moins 16, 17, 18, 19, 20, 25 ou 30 bases consécutives,
b) les acides nucléiques ayant une séquence complémentaire d'une séquence selon a),
c) les analogues fonctionnels d'acides nucléiques selon a) ou b), ou
d) les polypeptides codés par les acides nucléiques selon a) à c).

Également fourni est un acide nucléique isolé comprenant une séquence choisie parmi SEQ ID Nos : 1 à 5578 ou un fragment de celle-ci ayant au moins 15, 16, 17, 18, 19 ou 20 bases consécutives, ou une séquence complémentaire de celles-ci. De préférence, l'acide nucléique de l'invention ne comprend pas la séquence complète d'un gène ou ARN naturel. De préférence, il ne comprend pas plus de 500 bases, de préférence pas plus de 400 ou 300 bases. L'acide nucléique est typiquement synthétique, c'est-à-dire produit par voie non naturelle (recombinante, in vitro, synthèse chimoique, etc.).

### Marqueurs sériques de la maladie d'Alzheimer

La présente invention repose sur la mise en évidence et la caractérisation d'événements biologiques sériques caractéristiques de la maladie d'Alzheimer chez un patient humain. Ces événements constituent des biomarqueurs, dont la détection chez un patient permet, de préférence en combinaison, de déterminer, même à un stade précoce la présence d'une telle maladie

. En outre, les marqueurs sont également utilisables pour mesurer la réponse à un traitement, et/ou pour sélectionner des médicaments candidats. Les combinaisons de marqueurs décrites peuvent permettre de distinguer la maladie d'Alzheimer des autres pathologies neurodégénératives.

Les événements biologiques identifiés correspondent typiquement à des modifications dans la régulation de l'expression de gènes. Il peut s'agir d'une inhibition partielle ou totale de l'expression de gènes ou d'ARN, ou de certaines formes de gènes ou d'ARN, d'une augmentation de l'expression de gènes ou de certaines formes de gènes ou d'ARN, de l'apparition ou de la disparition de formes d'épissages de gènes, etc.

Également fournie est la détection, dans un échantillon, d'une ou plusieurs molécules cibles choisies avantageusement parmi :
a) les acides nucléiques comprenant une séquence choisie parmi SEQ ID NOs: 1-5578, ou un fragment distinctif de ceux-ci ayant au moins 15, de préférence au moins 16, 17, 18, 19, 20, 25 ou 30 bases consécutives,
b) les acides nucléiques ayant une séquence complémentaire d'une séquence selon a),
c) les analogues fonctionnels d'acides nucléiques selon a) ou b), ou
d) les polypeptides codés par les acides nucléiques selon a) à c).

Le terme « analogue fonctionnel » désigne de préférence un variant polymorphique des séquences SEQ ID NO : 1 - 5578 présent parmi la population humaine. Dans la plupart des cas, ces polymorphismes sont représentés par des variations ponctuelles au niveau d'une base, même si d'autres configurations polymorphiques existent également. Ces analogues peuvent être identifiés par toute technique connue de l'homme du métier, notamment en considération des séquences fournies dans la demande et des noms des gènes correspondants. Le terme analogue inclut également les séquences provenant d'une autre espèce de mammifère. En effet, les gènes correspondant aux SEQ ID NO : 1 - 5578 sont des gènes humains, et ces séquences constituent des marqueurs efficaces et adaptés à la détection de la maladie d'Alzheimer chez les patients humains. Néanmoins, pour une application des méthodes de l'invention à d'autres espèces de mammifères, il est généralement préférable d'utiliser des analogues fonctionnels de ces séquences, caractérisés dans l'espèce considérée..

Dans un mode de réalisation qui est également fournie la méthode comprend la détermination de la présence (ou de l'absence ou d'une variation de niveau d'expression) d'au moins un acide nucléique selon a) à c).

Dans un mode de réalisation qui est également fournie la méthode est utilisée pour détecter la maladie d'Alzheimer chez un sujet humain et comprend la détermination de la présence (ou de l'absence ou d'une variation de niveau d'expression) d'au moins un acide nucléique selon a) à c).

Dans une variante particulière de mise en oeuvre, la méthode décrite comprend la détermination combinée de la présence ou absence ou quantité (relative) d'au moins 5, 10, 15, 20, 30, 40, 50, 60, 70 ou plus des molécules cibles telles que définies ci-dessus. La détermination "combinée" désigne le fait qu'un profil d'hybridation (ou une signature) impliquant plusieurs marqueurs est déterminé. La détermination combinée est typiquement réalisée de manière simultanée, c'est-à-dire par mesure globale d'un profil d'expression. Néanmoins, la détermination combinée peut également être effectuée par mesures en parallèle ou séquentielle de plusieurs marqueurs, conduisant à l'identification d'un profil. la méthode décrite permet en effet d'établir et de déterminer un profil d'hybridation (ou une signature) sur un ensemble de marqueurs, afin d'évaluer la présence ou le risque de développer la maladie d'Alzheimer chez un mammifère. Le profil d'hybridation est typiquement réalisé en utilisant une combinaison de plusieurs marqueurs choisis parmi les cibles indiquées ci-dessus, par exemple contenant l'ensemble de ces cibles.

Dans un mode de mise en oeuvre particulier, la méthode décrite comprend la détermination de la présence (ou de l'absence ou de la quantité (relative)), dans un échantillon biologique du mammifère, d'au moins 5 molécules cibles distinctes choisies parmi celles définies ci-dessus, de préférence d'au moins 10.

Dans des modes préférés de mise en oeuvre, la méthode également décrite comprend la détermination combinée de la présence (ou de l'absence ou de la quantité (relative)), dans un échantillon biologique du mammifère, de sous-ensembles particuliers de molécules cibles choisies parmi celles définies ci-dessus. De tels sous-ensembles, décrits dans les exemples, sont particulièrement adaptés à la détection, notamment en phase précoce, de la présence de la maladie d'Alzheimer chez des patients à partir d'un échantillon de sang total.

Ainsi, dans un mode de mise en oeuvre particulier, la méthode de l'invention comprend la détermination combinée de la présence (ou de l'absence ou de la quantité (relative)), dans un échantillon biologique du mammifère, des acides nucléiques de l'ensemble d'un panel de cibles (ou signatures) comprenant
un ensemble de (groupes de) sondes comprenant au moins:
- une sonde comprenant tout ou partie de la séquence SEQ ID NO : 2363 ou de sa séquence complémentaire;
- une sonde comprenant tout ou partie de la séquence SEQ ID NO : 2364 ou de sa séquence complémentaire;
- une sonde comprenant tout ou partie de la séquence SEQ ID NO : 5307 ou de sa séquence complémentaire;
- une sonde comprenant tout ou partie de la séquence SEQ ID NO : 5426 ou de sa séquence complémentaire;
- une sonde comprenant tout ou partie de la séquence SEQ ID NO : 4218 ou de sa séquence complémentaire;
- une sonde comprenant tout ou partie de la séquence SEQ ID NO : 5063 ou de sa séquence complémentaire.
- une "partie" de séquence désignant une région de 15 à 50 nucléotides consécutifs de cette séquence.

Ainsi, dans un mode particulier, la méthode de l'invention comprend la détermination combinée de la présence (ou de l'absence ou de la quantité (relative)), dans un échantillon biologique du mammifère, de l'ensemble des acides nucléiques d'un des Panel 1 comprenant les sequences représentées dans le Tableau 1, colonnes G à BN, respectivement, ou un fragment distinctif de celles-ci ayant au moins 15, de préférence au moins 16, 17, 18, 19, 20, 25 ou 30 bases consécutives, ou ayant une séquence complémentaire de celles-ci et/ou des analogues fonctionnels de ceux-ci provenant d'autres espèces, et/ou des polypeptides codés par ces acides nucléiques. Les exemples fournis dans la présente demande montrent en effet que ce panel de marqueurs permet de détecter de manière prédictive la présence, de la maladie d'Alzheimer. Dans un mode particulier, la méthode comprend en outre la détection d'une ou plusieurs des autres molécules cibles telles que définies précédemment.

Également fournie est une méthode qui comprend la détermination de la présence (ou de l'absence ou de la quantité (relative)), dans un échantillon biologique du mammifère, des acides nucléiques comprenant respectivement les séquences représentées dans SEQ ID NOs: 1-5578 ou un fragment distinctif de celles-ci ayant au moins 15, de préférence au moins 16, 17, 18, 19, 20, 25 ou 30 bases consécutives, ou des acides nucléiques ayant une séquence complémentaire de celles-ci.

Un autre objet particulier de l'invention réside ainsi dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes spécifique des molécules cibles suivantes:
a) les acides nucléiques comprenant les séquences représentées dans le PANEL 1 tel que définis ci-avant, ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b) provenant d'une autre espèce,
pour obtenir un profil d'hybridation, le profil d'hybridation étant caractéristique de la présence de la maladie d'Alzheimer chez ce mammifère.

Un autre objet particulier de l'invention réside dans une méthode pour détecter le risque de développer la maladie d'Alzheimer chez un mammifère présentant des troubles cognitifs légers, ou asymptomatique, ou pour discriminer des patients atteints d'Alzheimer de sujets atteints de troubles cognitifs légers, comprenant la mise en contact, dans des conditions permettant une hybridation entre séquences complémentaires, des acides nucléiques issus d'un échantillon de sang du mammifère et d'un ensemble de sondes, l'ensemble de sondes contenant au moins une sonde comprenant tout ou une partie de chacune des séquences nucléiques du panel suivant, ou de leur séquence complémentaire:
- ensemble 1 : 6PS
pour obtenir un profil d'hybridation, le profil d'hybridation étant caractéristique de la présence de la maladie d'Alzheimer chez ce mammifère.

Un autre objet de l'invention réside dans l'utilisation d'un ensemble de sondes, l'ensemble de sondes comprenant au moins une sonde comprenant tout ou partie de chacune des séquences nucléiques du panel suivant, ou de leur séquence complémentaire :
- ensemble 1 : 6PS
pour détecter la présence de la maladie d'Alzheimer chez un sujet présentant des troubles cognitifs légers, ou asymptomatique, ou pour discriminer des sujets atteints d'Alzheimer de sujets atteints de troubles cognitifs légers.

Également fournie est une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins une sonde comprenant tout ou partie de chacune des séquences nucléiques du panel suivant, ou de leur séqeunce complémentaire :
- ensemble 3 : 18PS
pour obtenir un profil d'hybridation, le profil d'hybridation étant caractéristique de la présence ou du risque de développer la maladie d'Alzheimer chez ce mammifère.

L'utilisation d'un ensemble de sondes, l'ensemble de sondes comprenant au moins une sonde comprenant tout ou partie de chacune des séquences nucléiques du panel suivant, ou de leur séquence complémentaire :
- ensemble 3 : 18PS pour détecter la présence ou le risque de développer la maladie d'Alzheimer chez un sujet est décrite.

Un autre objet de l'invention réside dans une méthode pour détecter la présence de la maladie d'Alzheimer chez un mammifère, ou pour discriminer des patients atteints d'Alzheimer de sujets atteints de troubles cognitifs légers ou de sujets controles, la méthode comprenant la détection, à partir d'un échantillon de sang du mammifère, d'une variation dans les niveaux d'acides nucléiques complémentaires d'un ensemble de sondes, l'ensemble de sondes comprenant au moins une sonde comprenant tout ou partie de chacune des séquences nucléiques du de panel suivant, ou de leur séquence complémentaire :
- ensemble 1 : 6PS
une variation étant étant caractéristique de la présence ou du risque de développer la maladie d'Alzheimer chez ce mammifère.

Également décrite est la définition de panels supplémentaires, comprenant au moins certains marqueurs tels que définis précédemment, qui peuvent éventuellement être combinés à d'autres marqueurs. De tels panels peuvent être obtenus en testant la présence ou non de ces marqueurs dans des échantillons de patients, pour définir d'autres combinaisons prédictives, le cas échéant spécifiques de pathologies particulières.

### Méthodes de détection d'une altération dans un gène

Comme indiqué précédemment, une altération dans un gène ou ARN désigne au sens de l'invention (i) toute altération de l'expression, à savoir en particulier une dérégulation dans les niveaux d'expression (e.g., de transcription ou de traduction), une dérégulation de l'épissage, conduisant par exemple à l'apparition de formes épissées particulières ou à une modification de la quantité (relative) de ou du rapport entre différentes formes d'épissage, de même que (ii) toute altération dans la structure de la protéine produite (apparition ou disparition de formes tronquées, allongées, mutées, etc.).

Différentes techniques permettant la détection d'une espèce d'acide nucléique dans un échantillon sont utilisables dans la présente invention, comme par exemple le Northern Blot, l'hybridation sélective, l'utilisation de supports revêtus d'oligonucléotides sondes, l'amplification d'acide nucléique comme par exemple par RT-PCR, PCR quantitative ou ligation-PCR, etc. Ces méthodes peuvent comprendre l'utilisation d'une sonde nucléique (par exemple un oligonucléotide) capable de détecter sélectivement ou spécifiquement l'acide nucléique cible dans l'échantillon. L'amplification peut être réalisée selon différentes méthodes connues en soi de l'homme du métier, telles que la PCR, la LCR, l'amplification médiée par transcription (TMA), l'amplification par déplacement de brin (SDA), NASBA, l'emploi d'oligonucléotides spécifiques d'allèles (ASO), l'amplification spécifique d'allèle, le Southern blot, l'analyse conformationnelle SSCA, l'hybridation in situ (e.g., FISH), la migration sur gel, l'analyse d'hétéroduplexes, etc. Si nécessaire, la quantité d'acide nucléique détectée peut être comparée à une valeur de référence, par exemple une valeur médiane ou moyenne observée chez des patients qui ne sont pas atteints de la maladie d'Alzheimer, ou à une valeur mesurée en parallèle dans un échantillon témoin. Ainsi, il est possible de mettre en évidence une variation de niveaux d'expression.

Selon un mode préféré de mise en oeuvre, la méthode comprend la détection de la présence ou de l'absence ou de la quantité (relative) d'un acide nucléique selon a) à c) par hybridation sélective ou amplification sélective.

L'hybridation sélective est typiquement réalisée en utilisant des sondes nucléiques, de préférence immobilisées sur un support, tel qu'un support solide ou semi-solide présentant au moins une surface, plane ou non, permettant l'immobilisation de sondes nucléiques. De tels supports sont par exemple une lame, bille, membrane, filtre, colonne, plaque, etc. Ils peuvent être réalisés en tout matériau compatible, comme notamment du verre, silice, plastique, fibre, métal, polymère, etc. Les sondes nucléiques peuvent être tout acide nucléique (ADN, ARN, PNA, etc.), de préférence simple-brin, comprenant une séquence spécifique d'une molécule cible telle que définie en a) à c) ci-dessus. Les sondes comprennent typiquement de 5 à 400 bases, de préférence de 8 à 200, plus préférentiellement moins de 100, et encore plus préférentiellement moins de 75, 60, 50, 40 ou même 30 bases. Les sondes peuvent être des oligonucléotides synthétiques, produits sur la base des séquences SEQ ID NO : 1-5578 (target séquences) décrits selon des techniques de synthèse classique. De tels oligonucléotides comportent typiquement de 10 à 50 bases, de préférence de 20 à 40, par exemple 25 bases environ. Dans un mode particulièrement avantageux, de manière à améliorer le signal détecté, on utilise plusieurs oligonucléotides (ou sondes) différents définis à partir d'une même séquence cible (target sequence) pour détecter la même molécule cible (transcrit issu de l'amplification de l'ARN du patient) au cours de l'hybridation. Il peut s'agir d'oligonucléotides spécifiques de régions différentes de la même séquence cible, ou centrés différemment sur une même région. On utilise avantageusement des probesets comprenant 1-3 sondes, qui peuvent être chevauchantes ou non, en tout ou en partie, et qui sont spécifiques de la même molécule cible. On peut également utiliser des couples de sondes, dont un membre est parfaitement apparié à la séquence cible, et un autre présente un mésappariement, permettant ainsi d'estimer le bruit de fond. Les sondes peuvent être conçues pour s'hybrider à une région d'un exon ou d'un intron, ou à une région de jonction de type exon-exon, exon-intron ou intron-intron. Ainsi, les sondes permettent de mettre en évidence et de distinguer différentes formes d'épissage d'un gène.

L'utilisation des sondes dont la séquence comprend tout ou une partie d'une séquence nucléique choisie parmi SEQ ID NOs : 1-5578 ou d'une séquence complémentaire de celles-ci également décrite. Dans un mode préféré, on utilise des sondes nucléiques ayant une longueur comprise entre 15 et 50 bases, plus préférentiellement entre 15 et 40 bases, et dont la séquence est identique à un fragment d'une séquence choisie parmi SEQ ID NO : 1-5578 ou d'une séquence complémentaire de celles-ci.

Dans un mode on utilise des probesets, c'est-à-dire des jeux de 1-3 sondes comprenant chacune une partie, chevauchante ou non, de la même séquence nucléique choisie parmi SEQ ID NOs : 1-5578.

Les sondes peuvent être synthétisées préalablement puis déposées sur le support, ou synthétisées directement in situ, sur le support, selon des méthodes connues en soi de l'homme du métier. Les sondes peuvent également être fabriquées par des techniques génétiques, par exemple par amplification, recombinaison, ligation, etc.

Les sondes ainsi définies sont décrits, ainsi que leurs utilisations (essentiellement in vitro) pour la détection de la maladie d'Alzheimer chez un sujet.

L'hybridation peut être réalisée dans des conditions classiques, connues de l'homme du métier et ajustables par celui-ci (Sambrook, Fritsch, Maniatis (1989) Molecular Cloning, Cold Spring Harbor Laboratory Press). En particulier, l'hybridation peut être réalisée dans des conditions de stringence élevée, moyenne ou faible, selon le niveau de sensibilité recherché, la quantité de matériel disponible, etc. Par exemple, des conditions appropriées d'hybridation incluent une température comprise entre 55 et 63°C pendant 2 à 18 heures. D'autres conditions d'hybridation, adaptées à des supports de haute densité, sont par exemple une température d'hybridation entre 45 et 55°C. Après l'hybridation, différents lavages peuvent être réalisés pour éliminer les molécules non-hybridées, typiquement dans des tampons SSC comprenant du SDS, tels que un tampon comprenant 0,1 à 10 X SSC et 0,5-0,01% SDS. D'autres tampons de lavage contenant du SSPE, du MES, du NaCl ou du EDTA peuvent également être utilisés.

Dans un mode de mise en oeuvre typique, les acides nucléiques (ou les puces ou supports) sont pré-hybridés dans un tampon d'hybridation (Rapid Hybrid Buffer, Amersham) contenant typiquement 100 µg/ml d'ADN de sperme de saumon à 65°C pendant 30 min. Les acides nucléiques de l'échantillon sont ensuite mis en contact avec les sondes (typiquement appliqués sur le support ou la puce) à 65°C pendant 2 à 18 heures. De préférence, les acides nucléiques de l'échantillon sont marqués au préalable, par tout marquage connu (radioactif, enzymatique, fluorescent, luminescent, etc.). Les supports sont ensuite lavés dans un tampon 5X SSC, 0,1% SDS à 65°C pendant 30 min, puis dans un tampon 0.2X SSC, 0,1% SDS. Le profil d'hybridation est analysé selon des techniques classiques, comme par exemple en mesurant le marquage sur le support au moyen d'un instrument adapté (par exemple InstantImager, Packard Instruments). Les conditions de l'hybridation peuvent naturellement être ajustées par l'homme du métier, par exemple en modifiant la température d'hybridation et/ou la concentration saline du tampon ainsi que par ajout de substances auxiliaires comme le formamide ou de l'ADN simple brin.

Également fournie est une méthode pour détecter la présence ou le risque de développer la maladie d'Alzheimer chez un mammifère, ou pour évaluer la réponse à un traitement contre la maladie d'Alzheimer, comprenant la mise en contact, dans des conditions permettant une hybridation entre séquences complémentaires, des acides nucléiques issus d'un échantillon de sang du mammifère et d'un ensemble de sondes spécifique des molécules cibles identifiées précédemment pour obtenir un profil d'hybridation, le profil d'hybridation étant caractéristique de la présence ou du risque de développer la maladie d'Alzheimer chez ce mammifère, ou de l'efficacité du traitement.

Un objet particulier de l'invention réside ainsi dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes spécifique des molécules cibles suivantes au moins:
a) les acides nucléiques comprenant les séquences du panel 1 défini précédemment, ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b) provenant d'une autre espèce,
pour obtenir un profil d'hybridation, le profil d'hybridation étant caractéristique de la présence ou du risque de développer la maladie d'Alzheimer chez ce mammifère.

Dans des modes particuliers de mise en oeuvre, les procédés de l'invention utilisent en outre d'autres molécules cibles et/ou d'autres sondes, notamment les sous-ensembles de molécules cibles mentionnés dans la présente demande.

Ainsi, une autre méthode pour détecter la présence ou le risque de développer la maladie d'Alzheimer chez un mammifère est décrite comprenant la mise en contact, dans des conditions permettant une hybridation entre séquences complémentaires, des acides nucléiques issus d'un échantillon de sang du mammifère et d'un ensemble de sondes spécifique d'au moins deux molécules distinctes choisies parmi les cibles suivantes:
a) les acides nucléiques comprenant les séquences représentées dans SEQ ID NOs: 1-5578 ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b) provenant d'une autre espèce,
pour obtenir un profil d'hybridation, le profil d'hybridation étant caractéristique de la présence ou du risque de développer la maladie d'Alzheimer chez ce mammifère.

Un autre objet particulier de l'invention réside dans une méthode pour détecter la présence de la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins une sonde comprenant tout ou une partie de chacune des séquences nucléiques du panels suivants, ou de leur séquence complémentaire :
- ensemble 1 : 6PS
pour obtenir un profil d'hybridation, le profil d'hybridation étant caractéristique de la présence de la maladie d'Alzheimer chez ce mammifère.

Comme expliqué plus en détails dans la partie expérimentale (voir exemple 11), l'ensemble 1 comprend ainsi au moins les 6 sondes suivantes :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.

Comme indiqué précédement, le terme « partie » désigne avantageusement une région de 15 à 50 nucléotides consécutifs.

Dans un mode particulier, l'ensemble 1 comprend :
- de 1 à 3 sondes, chevauchantes ou non, comprenant chacune tout ou une partie de la séquence SEQ ID NO : 2363 ou de sa séquence complémentaire ;
- de 1 à 3 sondes, chevauchantes ou non, comprenant chacune tout ou une partie de la séquence SEQ ID NO : 2364 ou de sa séquence complémentaire ;
- de 1 à 3 sondes, chevauchantes ou non, comprenant chacune tout ou une partie de la séquence SEQ ID NO : 5307 ou de sa séquence complémentaire ;
- de 1 à 3 sondes, chevauchantes ou non, comprenant chacune tout ou une partie de la séquence SEQ ID NO : 5426 ou de sa séquence complémentaire ;
- de 1 à 3 sondes, chevauchantes ou non, comprenant chacune tout ou unepartie de la séquence SEQ ID NO : 4218 ou de sa séquence complémentaire ; et
- de 1 à 3 sondes, chevauchantes ou non, comprenant chacune tout ou une partie de la séquence SEQ ID NO : 5063 ou de sa séquence complémentaire.

Il est entendu que l'ensemble 1 peut comprendre, en plus des 6 sondes ou groupes de sondes (probesets) cités, d'autres sondes ou probesets, comprenant par exemple une séquence choisie parmi SEQ ID NO : 1-5578 et/ou parmi d'autres séquences.
Les ensembles 2-4 peuvent être définis comme l'ensemble 1 ci-dessus. Les sondes de base constituant ces ensembles sont données dans l'exemple 11.

Un autre objet réside dans une méthode pour discriminer des patients atteints d'Alzheimer de sujets atteints de troubles cognitifs légers, comprenant la mise en contact dans des conditions permettant une hybridation entre séquences complémentaires, des acides nucléiques issus d'un échantillon de sang du mammifère et d'un ensemble de sondes. l'ensemble de sondes comprenant une pluralité de sondes dont au moins une sonde comprenant tout ou partie de chacune des séquences nucléiques du panel suivant, ou de leur séquence complémentaire :
- ensemble 1 : 6PS
pour obtenir un profil d'hybridation, le profil d'hybridation étant caractéristique de la présence de la maladie d'Alzheimer chez ce mammifère.

Un autre objet de l'invention réside dans l'utilisation d'un ensemble de (groupes de) sondes l'ensemble contenant une pluralité de (groupes de) sondes dont au moins une sonde (ou un groupe de sondes) comprenant tout ou une partie de chacune des séquences nucléiques du panel suivant, ou de leur séquence complémentaire:
- ensemble 1 : 6PS
pour détecter la présence de la maladie d'Alzheimer chez un sujet présentant des troubles cognitifs légers, ou asymptomatique, ou pour discriminer des sujets atteints d'Alzheimer de sujets atteints de troubles cognitifs légers.

La possibilité de distinguer, au sein de patients présentant des troubles cognitifs légers (MCI) ou asymptomatiques, ceux atteints d'Alzheimer est particulièrement importante car elle permet de mettre en place rapidement le traitement le mieux adapté. 30-40% des sujets présentant des troubles de type MCI vont évoluer vers un AD. La possibilité d'identifier ces sujets permet, dès le stade MCI, de mettre en place les protocoles thérapeutiques efficaces pour AD, sans devoir attendre l'évolution de la maladie.

Également décrite est une méthode pour détecter le risque de développer la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes. l'ensemble de sondes comprenant au moins une sonde comprenant tout ou partie de chacune des séquences nucléiques du panel suivant, ou de leur séquence complémentaire :
- ensemble 3 : 18PS
pour obtenir un profil d'hybridation, le profil d'hybridation étant caractéristique de la présence ou du risque de développer la maladie d'Alzheimer chez ce mammifère.

Également décrite est l'utilisation d'un ensemble de (groupes de) sondes comprenant au moins une sonde (ou un groupe de sondes) comprenant tout ou partie de chacune des séquences nucléiques du panel suivant, ou de leur séquence complémentaire :
- ensemble 3 : 18PS
pour détecter la présence ou le risque de développer la maladie d'Alzheimer chez un sujet.

Un autre objet de l'invention réside dans une méthode pour détecter la présence de la maladie d'Alzheimer chez un mammifère, ou pour discriminer des patients atteints d'Alzheimer de sujets atteints de troubles cognitifs légers ou de sujets controles, comprenant la détection, à partir d'un échantillon de sang du mammifère, d'une variation dans les niveaux d'acides nucléiques complémentaires d'un ensemble de sondes, l'ensemble de sondes comprenant au moins une sonde comprenant tout ou partie de chacune des séquences nucléiques du panel suivant, ou de leur séquence complémentaire :
- ensemble 1 : 6PS
une variation étant caractéristique de la présence ou du risque de développer la maladie d'Alzheimer chez ce mammifère.

Le profil d'hybridation peut être comparé à un ou plusieurs profils de référence, notamment un profil de référence caractéristique de sujets sains et/ou de sujets atteints de la maladie d'Alzheimer, la comparaison permettant de déterminer la probabilité ou le risque que le patient testé soit atteint de la maladie d'Alzheimer. Typiquement, la comparaison est réalisée au moyen de programmes informatiques connus en soi de l'homme du métier.

L'amplification sélective est de préférence réalisée en utilisant une amorce ou une paire d'amorces permettant l'amplification de tout ou partie d'un des acides nucléiques cibles dans l'échantillon, lorsque celui-ci y est présent. L'amorce peut être spécifique d'une séquence cible telle que définie précédemment selon SEQ ID NO : 1-5578, ou d'une région flanquant la séquence cible dans un acide nucléique de l'échantillon. L'amorce comprend typiquement un acide nucléique simple-brin, d'une longueur comprise avantageusement entre 5 et 50 bases, de préférence entre 5 et 30. Une telle amorce constitue un autre objet de la présente demande, ainsi que son utilisation (essentiellement in vitro) pour la détection de la maladie d'Alzheimer chez un sujet. Les amorces peuvent être conçues pour s'hybrider à une région d'un exon ou d'un intron, ou à une région de jonction de type exon-exon, exon-intron ou intron-intron. Ainsi, les amorces permettent de mettre en évidence et de distinguer différentes formes d'épissage d'un gène.

A cet égard, est décrite également l'utilisation d'une amorce nucléotidique ou d'un ensemble d'amorces nucléotidiques permettant l'amplification de tout ou partie d'un ou, de préférence plusieurs gènes ou ARN comprenant une séquence cible selon SEQ ID NO : 1-5578, pour détecter la présence ou le risque de développer la maladie d'Alzheimer chez un mammifère, ou pour évaluer la réponse àun traitement contre de la maladie d'Alzheimer chez.un mammifère, tout particulièrement chez un être humain.

Une méthode pour détecter la présence de la maladie d'Alzheimer chez un mammifère, comprenant la mise en contact, dans des conditions permettant une amplification, des acides nucléiques issus d'un échantillon de sang du mammifère et d'un ensemble d'amorces spécifique d'au moins deux molécules distinctes choisies parmi les cibles suivantes:
a) les acides nucléiques comprenant les séquences représentées dans SEQ ID NOs: 1-5578 ou un fragment distinctif de celles-ci ayant au moins 15, de préférence au moins 16, 17, 18, 19, 20, 25 ou 30 bases consécutives, et/ou
b) les acides nucléiques ayant une séquence complémentaire de séquences selon a), et/ou
c) les analogues fonctionnels des acides nucléiques selon a) ou b) provenant d'une autre espèce,
pour obtenir un profil d'amplification, le profil d'amplification étant caractéristique de la présence ou du risque de développer la maladie d'Alzheimer chez ce mammifère est également décrite.

### Détection d'une altération dans un polypeptide

Dans un autre mode de réalisation, la méthode comprend la détermination de la présence ou de la quantité (relative) d'un polypeptide codé par un gène tel que défini précédemment. La mise en évidence ou le dosage d'un polypeptide dans un échantillon peut être réalisée par toute technique connue en soi, comme notamment au moyen d'un ligand spécifique, par exemple un anticorps ou un fragment ou dérivé d'anticorps. De préférence, le ligand est un anticorps spécifique du polypeptide, ou un fragment d'un tel anticorps (par exemple un Fab, Fab', CDR, etc.), ou un dérivé d'un tel anticorps (par exemple un anticorps simple-chaîne, ScFv). Le ligand est typiquement immobilisé sur un support, tel qu'une lame, bille, colonne, plaque, etc. La présence ou la quantité du polypeptide cible dans l'échantillon peut être détectée par mise en évidence d'un complexe entre la cible et le ligand, par exemple en utilisant un ligand marqué, en utilisant un deuxième ligand de révélation marqué, etc. Des techniques immunologiques utilisables et bien connues sont les techniques ELISA, RIA, etc. Si nécessaire, la quantité de polypeptide détectée peut être comparée à une valeur de référence, par exemple une valeur médiane ou moyenne observée chez des patients qui ne sont pas atteints de la maladie d'Alzheimer, ou à une valeur mesurée en parallèle dans un échantillon témoin. Ainsi, il est possible de mettre en évidence une variation de niveaux d'expression.

Des anticorps spécifiques des polypeptides cibles peuvent être produits par des techniques conventionnelles, notamment par immunisation d'un animal non-humain avec un immunogène comprenant le polypeptide (ou un fragment immunogène de celui-ci), et récupération des anticorps (polyclonaux) ou des cellules productrices (pour produire des monoclonaux). Des techniques de production d'anticorps poly- ou monoclonaux, de fragments ScFv, d'anticorps humains ou humanisés sont décrites par exemple dans Harlow et al., Antibodies: A laboratory Manual, CSH Press, 1988 ; Ward et al., Nature 341 (1989) 544 ; Bird et al., Science 242 (1988) 423 ; WO94/02602 ; US5,223,409 ; US5,877,293 ; WO93/01288. L'immunogène peut être fabriqué par synthèse, ou par expression, dans un hôte approprié, d'un acide nucléique cible tel que défini ci-avant. Un tel anticorps, monoclonal ou polyclonal, ainsi que ses dérivés ayant la même spécificité antigénique, constituent également un objet de la présente demande, de même que leur utilisation pour détecter la maladie d'Alzheimer.

Des modifications de l'expression et/ou de la structure des protéines peuvent aussi être détectées au moyen des techniques connues en soi de l'homme du métier et impliquant la spectroscopie de masse, plus généralement regroupées sous le nom d'analyse protéomique, afin de détecter des signatures spécifiques du sang des patients atteints de la maladie d'Alzheimer.

La méthode décrite est applicable à tout échantillon biologique du mammifère testé, en particulier tout échantillon comportant des acides nucléiques ou des polypeptides. On peut citer avantageusement un échantillon de sang, plasma, plaquette, salive, urine, selles, etc., plus généralement tout tissu, organe ou, avantageusement, fluide biologique comportant des acides nucléiques ou des polypeptides.

Dans un mode de mise en oeuvre préféré et particulièrement avantageux, l'échantillon est un échantillon dérivé du sang, par exemple un échantillon de sang, de sérum ou de plasma. L'invention découle en effet de l'identification de marqueurs sanguins de la maladie d'Alzheimer, et permet donc une détection de cette pathologie sans biopsie tissulaire, mais uniquement à partir de prélèvements sanguins.

L'échantillon peut être obtenu par toute technique connue en soi, par exemple par prélèvement, par des techniques non invasives, à partir de collections ou banques d'échantillons, etc. L'échantillon peut par ailleurs être pré-traité pour faciliter l'accessibilité des molécules cibles, par exemple par lyse (mécanique, chimique, enzymatique, etc.), purification, centrifugation, séparation, etc. L'échantillon peut également être marqué, pour faciliter la détermination de la présence des molécules cibles (marquage fluorescent, radioactif, luminescent, chimique, enzymatique, etc.). Les acides nucléiques de l'échantillon peuvent par ailleurs être séparés, traités, enrichis, purifiés, rétro-transcrits, amplifiés, fragmentés, etc. Dans un mode particulier, les acides nucléiques de l'échantillon sont les ou des ARN, notamment les ou des ARNm de l'échantillon. Dans un mode tout particulier, les acides nucléiques sont le produit d'amplification des ARN, notamment des ARNm ; ou les/des ADNc préparés à partir des ARN, notamment des ARNm de l'échantillon.

Dans un mode de mise en oeuvre préféré, l'échantillon biologique est un échantillon de sang total, c'est-à-dire n'ayant pas subi d'étape de séparation, qui peut être éventuellement dilué.

L'invention est applicable à tout mammifère, de préférence les humains. La méthode de invention est particulièrement utile pour la détection de la maladie d'Alzheimer, notamment de la présence de la maladie d'Alzheimer chez l'être humain. Ainsi, les données fournies dans les exemples montrent que l'invention permet de détecter la présence de la maladie d'Alzheimer avec une sensibilité supérieure à 95% et une spécificité supérieure à 95%.

Également décrite dans la présente demande est une méthode pour détecter la présence, l'évolution ou le risque de développer la maladie d'Alzheimer chez un sujet humain, comprenant la détermination combinée de la présence (ou de l'absence ou de la quantité (relative)), dans un échantillon biologique du sujet humain, de molécules cibles choisies parmi:
a) les acides nucléiques comprenant une séquence choisie parmi SEQ ID NO: 1-5578 ou un fragment de celles-ci ayant au moins 15, de préférence au moins 16, 17, 18, 19, 20, 25 ou 30 bases consécutives,
b) les acides nucléiques ayant une séquence complémentaire d'une séquence selon a), et
c) les polypeptides codés par les acides nucléiques selon a) ou b).

De préférence, la méthode comprend la détermination combinée de la présence, absence ou quantité de 5, 10, 20, 30, 40, 50 ou 60 molécules cibles telles que définies ci-dessus.

Également décrite est une méthode pour détecter la présence, l'évolution ou le risque de développer la maladie d'Alzheimer chez un sujet humain, comprenant la mise en contact d'un échantillon biologique du sujet contenant des acides nucléiques avec un produit comprenant un support sur lequel sont immobilisés des acides nucléiques comprenant une séquence complémentaire et/ou spécifique d'une ou, de préférence, plusieurs molécules cibles choisies parmi (i) les acides nucléiques comprenant une séquence choisie parmi SEQ ID NO: 1-5578 ou un fragment de celles-ci ayant au moins 15, de préférence au moins 16, 17, 18, 19, 20, 25 ou 30 bases consécutives et (ii) les acides nucléiques ayant une séquence complémentaire d'une séquence selon (i), et le profil indiquant la présence, le degré de sévérité / avancement ou le risque de développer la maladie d'Alzheimer chez ledit sujet humain. De préférence, le produit comprend des acides nucléiques distincts comprenant une séquence complémentaire et/ou spécifique d'au moins 5, 10, 20, 30, 40, 50, 60 ou plus gènes ou ARNs différents tels que mentionnés ci-dessus.

Également fournie est un produit comprenant un support sur lequel sont immobilisés des acides nucléiques comprenant une séquence complémentaire et/ou spécifique d'une ou, de préférence, plusieurs molécules cibles choisies parmi (i) les acides nucléiques comprenant une séquence choisie parmi SEQ ID NO: 1-5578 ou un fragment de celles-ci ayant au moins 15, de préférence au moins 16, 17, 18, 19, 20, 25 ou 30 bases consécutives et (ii) les acides nucléiques ayant une séquence complémentaire d'une séquence selon (i). De préférence, le produit comprend des acides nucléiques distincts comprenant une séquence complémentaire et/ou spécifique d'au moins 5, 10, 20, 30, 40, 50, 60 ou plus gènes ou ARN tels que définis précédemment.

Également décrite est un produit comprenant un support sur lequel est immobilisé au moins un, de préférence plusieurs, acides nucléiques comprenant une séquence choisie parmi SEQ ID NO: 1-5578, ou un analogue fonctionnel de celles-ci. De préférence, le produit comprend au moins 5, 10, 20, 30, 40, 50, 60 ou plus acides nucléiques différents choisis parmi les acides nucléiques mentionnés ci-dessus.

Un autre objet de la présente demande concerne un produit comprenant un support sur lequel est immobilisé un ensemble de sondes, l'ensemble de sondes comprenant une pluralité de sondes dont au moins une sonde comprenant tout ou partie de chacune des séquences nucléiques du panel suivant, ou de leur séquence complémentaire :
- ensemble 1 : 6PS,

Également fournie est un produit comprenant un support sur lequel est immobilisé au moins un ligand d'un polypeptide codé par un acide nucléique cible tel que défini ci-dessus, c'est-à-dire un acide nucléique comprenant une séquence choisie parmi SEQ ID NO: 1-5578, un fragment distinctif de celles-ci ayant au moins 15, de préférence au moins 16, 17, 18, 19, 20, 25 ou 30 bases consécutives, un acide nucléique ayant une séquence complémentaire de celles-ci ou un analogue fonctionnel de celles-ci. De préférence, le produit comprend au moins 5, 10, 20, 30, 40, 50, 60 ou plus ligands de polypeptides différents choisis parmi les polypeptides mentionnés ci-dessus.

Le support peut être tout support solide ou semi-solide présentant au moins une surface, plane ou non (c'est-à-dire en 2 ou 3 dimensions), permettant l'immobilisation d'acides nucléiques ou de polypeptides. De tels supports sont par exemple une lame, bille, membrane, filtre, colonne, plaque, etc. Ils peuvent être réalisés en tout matériau compatible, comme notamment du verre, silice, plastique, fibre, métal, polymère, polystyrène, téflon, etc. Les réactifs peuvent être immobilisés sur la surface du support par des techniques connues, ou, dans le cas des acides nucléiques, synthétisés directement in situ sur le support. Des techniques d'immobilisation incluent l'adsorption passive (Inouye et al., J. Clin. Microbiol. 28 (1990) 1469), la liaison covalente. Des techniques sont décrites par exemple dans WO90/03382, WO99/46403 Les réactifs immobilisés sur le support peuvent être ordonnés selon un schéma pré-établi, pour faciliter la détection et l'identification des complexes formés, et selon une densité variable et adaptable.

Le produit comprend un pluralité d'oligonucléotides synthétiques, d'une longueur comprise entre 5 et 100 bases, spécifiques de plusieurs gènes ou ARNs tels que définis précédemment.

Les produits de l'invention comprennent typiquement des molécules contrôle, permettant d'étalonner et/ou normaliser les résultats.

Également décrit est un produit comprenant un support sur lequel sont immobilisés des acides nucléiques comprenant tout ou partie des séquences choisies parmi SEQ ID NO: 5579 - 21559. Ces séquences représentent des sondes spécifiques des SEQ ID NO : 1 - 5578. Un tel produit pourra avantageusement incorporer des acides nucléiques choisis pour leur caractère non discriminant de population de patients atteints de la maladie d'Alzheimer, de tels acides nucléiques servant de contrôles de normalisation du produit. Ces acides nucléiques peuvent correspondre à tout ou partie des séquences choisies parmi SEQ ID NO : 21560 - 51087.

Un autre objet de la présente demande concerne un kit comprenant un compartiment ou conteneur comprenant au moins un, de préférence plusieurs, acides nucléiques comprenant de une séquence complémentaire et/ou spécifique de plusieurs gènes ou ARNs tels que définis précédemment De préférence, le produit comprend au moins 5, 10, 20, 30, 40, 50, 60 ou plus acides nucléiques et/ou ligands différents choisis parmi les acides nucléiques et ligands mentionnés ci-dessus. Dans un mode particulier de mise en oeuvre, le produit comprend chacun des acides nucléiques de séquence SEQ ID NO: 1-5578 ou un ligand pour chacun des polypeptides cibles tels que définis ci-dessus. Dans un autre mode particulier de mise en oeuvre, le produit comprend chacun des acides nucléiques de séquence SEQ ID NO : 5579 - 51087. Le kit peut comprendre par ailleurs des réactifs pour une réaction d'hybridation ou immunologique, ainsi que, le cas échéant, des contrôles et/ou instructions.

Un autre objet de l'invention concerne l'utilisation d'un produit ou kit tel que défini ci-dessus pour la détection de la maladie d'Alzheimer chez un sujet mammifère, de préférence un sujet humain.

Également décrite est un acide nucléique de séquence choisie parmi SEQ ID NO : 1-5578, ou un fragment distinctif de celles-ci comprenant au moins 15 bases consécutives, de préférence au moins 16, 17, 18, 19, 20, 25 ou 30, ou un acide nucléique ayant une séquence complémentaire de celles-ci, ou un analogue fonctionnel de celles-ci. L'invention concerne également un vecteur de clonage ou d'expression comportant ces acides nucléiques, ainsi que toute cellule recombinante comprenant un tel vecteur ou acide nucléique.

L'utilisation d'un acide nucléique comprenant une séquence choisie parmi SEQ ID NO: 1-5578, ou un fragment distinctif de celles-ci comprenant au moins 15 bases consécutives, de préférence au moins 16, 17, 18, 19, 20, 25 ou 30, ou un acide nucléique ayant une séquence complémentaire de celles-ci, ou un analogue fonctionnel de celles-ci, pour la détection (essentiellement in vitro) de la maladie d'Alzheimer chez un sujet mammifère est également décrite.

Selon un exemple particulier de mise en oeuvre de l'invention, on prélève un échantillon de sang d'un mammifère à tester. L'échantillon de sang est éventuellement traité de manière à rendre les acides nucléiques plus accessibles, et ceux-ci sont marqués. Les acides nucléiques sont ensuite appliqués sur un produit tel que défini ci-avant et le profil d'hybridation est déterminé, permettant de diagnostiquer la présence ou non de la maladie d'Alzheimer chez le sujet. La méthode de l'invention est simple, pratiquée ex vivo, et permet la détection précoce de la maladie d'Alzheimer à partir d'un échantillon de sang.

D'autres aspects et avantages de la présente invention apparaîtront à la lecture des exemples qui suivent, qui doivent être considérés comme illustratifs et non limitatifs.

### LEGENDE DES FIGURES

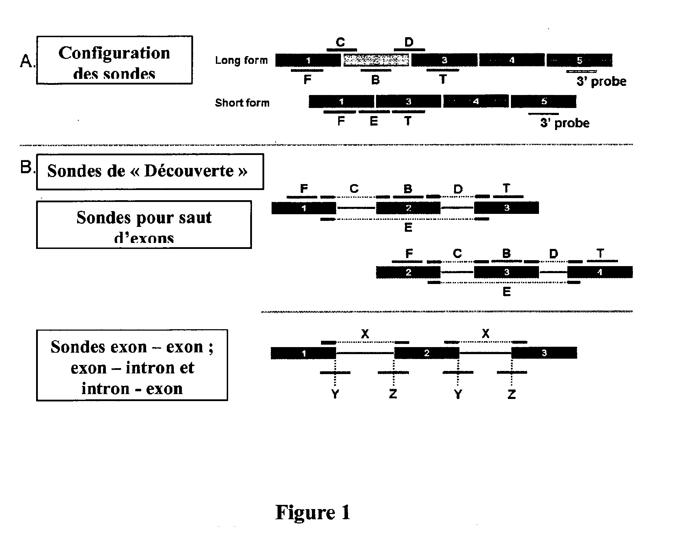

**Figure 1**. Configuration des sondes présentes sur le microarray GWSA. A) Les sondes exons de type B, F et T et les sondes de jonction de type C, D et E permettent de couvrir de façon optimale les événements d'épissage. B) Des sondes capables de couvrir tout saut d'exon unique sont également présentes avec la même configuration. De même, des sondes de jonction exon - exon (X), exon - intron (Z) et intron -exon (Y) sont également présentes.

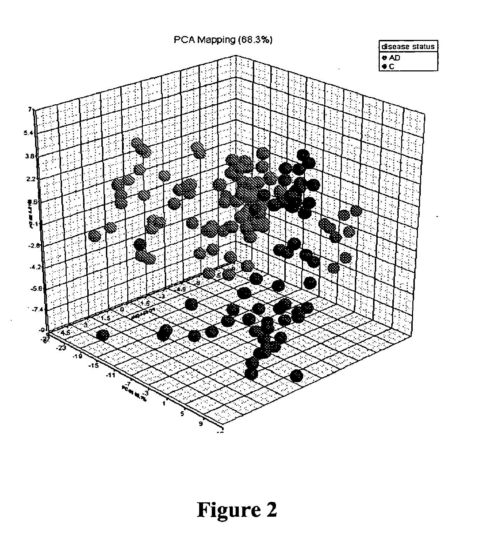

**Figure 2**. Analyse par Composante Principale des patients AD et des contrôles à partir de la signature SVM du panel 50.

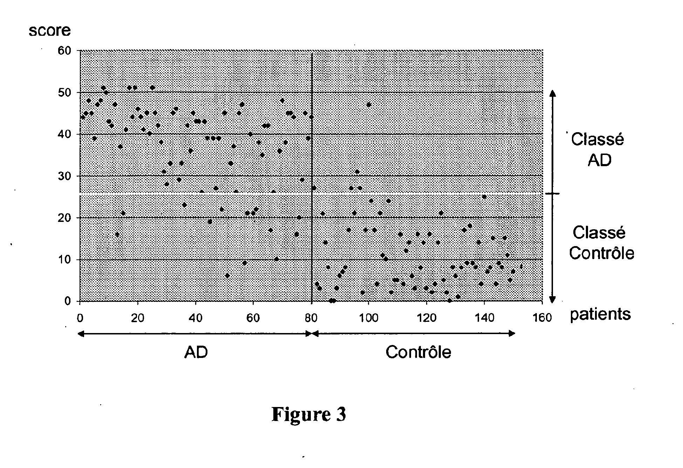

**Figure 3**. Scores de classification obtenus à partir de 51 paires de probe sets issues des gènes PTPRC, SORL1 et PSEN1. Patients AD : n° 1 à 80. Individus contrôles : n° 81 à 150. Le score maximal est de 51. La ligne blanche horizontale matérialise la valeur 25,5. Tout patient dont le score est supérieur à cette valeur est classé en AD. Tout patient dont le score est inférieur à cette valeur est classé en contrôle.

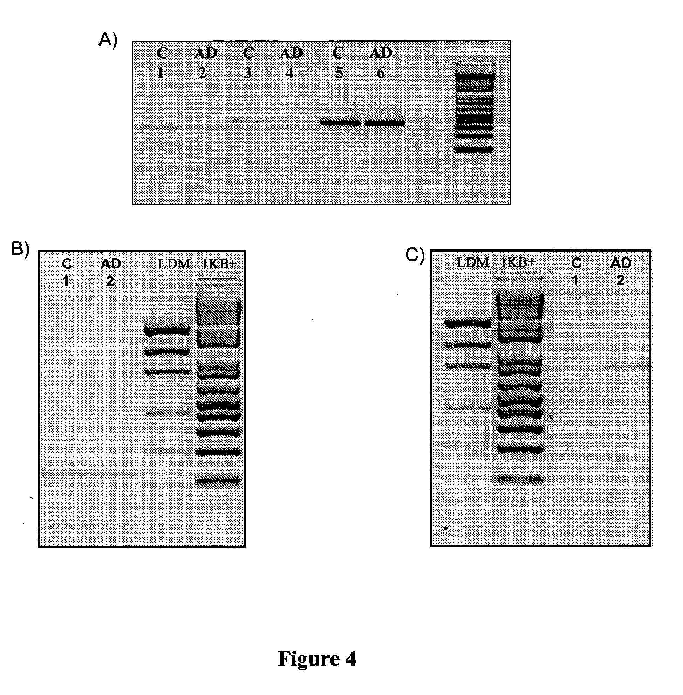

**Figure 4**. Essais de RT-PCR. A) Gène PTPRC. 1-2 : Amorces dans exon 2 et intron 5 ; 3-4 : Amorces dans exon 4 et intron 5 ; 5-6 : Amorces tubuline (contrôle positif). B) Gène SORL1. 1-2 : Amorces dans intron 41 et exon 42. C) Gène PSEN1. 1-2 : Amorces dans exon alternatif représenté par l'EST AK122722.1, situé entre les exons 3 et 4 et dans exon 4.

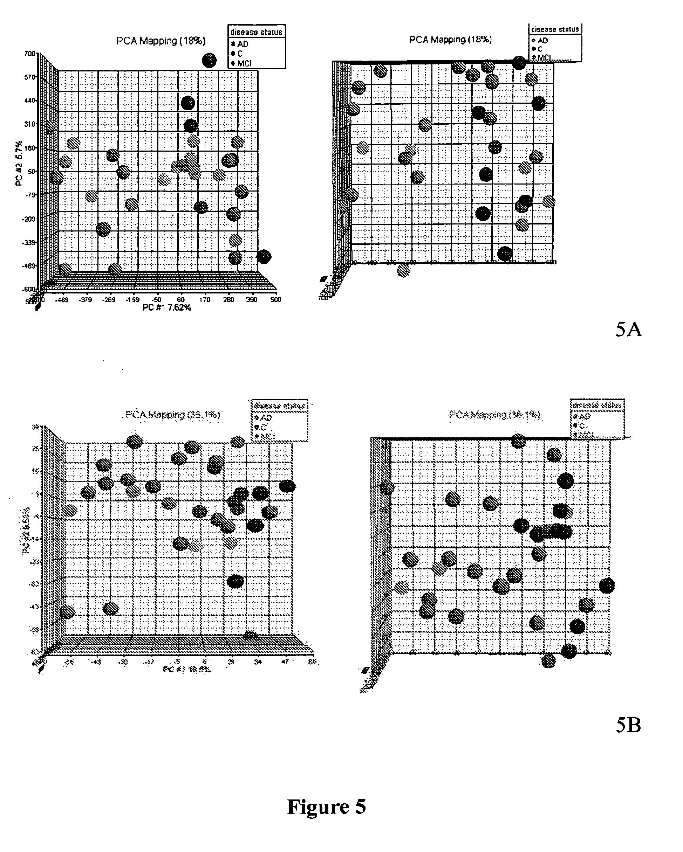

**Figure 5**. Analyse par Composante Principale des patients AD, MCI et C à partir de l'ensemble des groupes de sondes présent sur la lame GWSA (Fig. 5A) ou d'une sous sélection constituée de 5562 groupes de sondes parmi les 5578 (Fig. 5B).

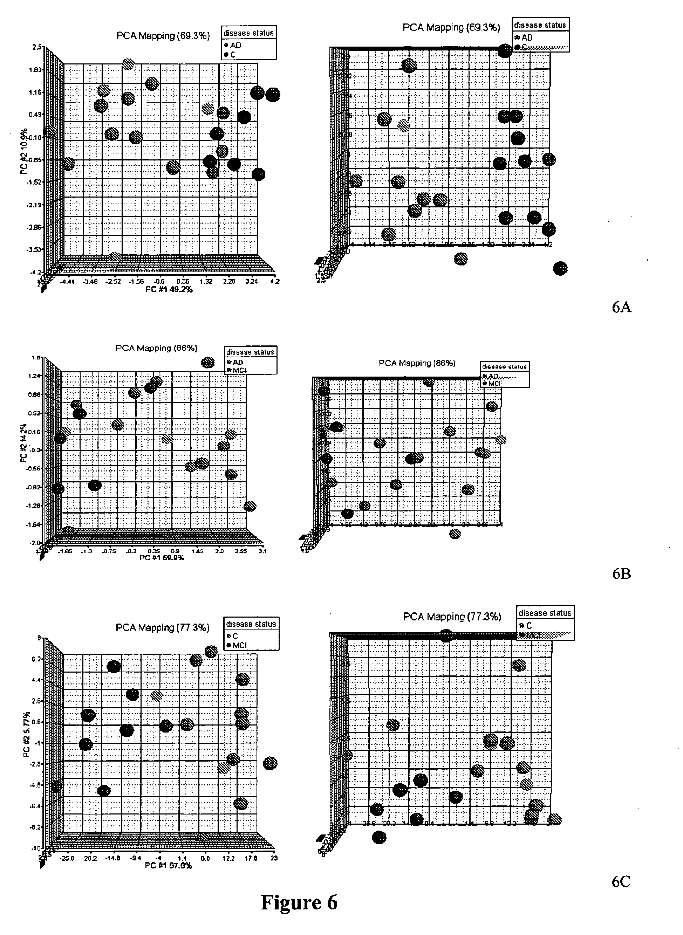

**Figure 6**. Visualisation de la répartition des patients par Analyse par Composante Principale des patients MCI, AD ou C à partir des sous-ensembles de groupes de sondes (= probe sets,.PS) spécifiques sélectionnés. Fig. 6A, discrimination entre patients AD et C à partir d'un ensemble de 18 sondes ; Fig. 6B, discrimination entre patients MCI et AD à partir d'un ensemble de 6 sondes ; Fig. 6C, discrimination entre patients AD et C à partir d'un ensemble de 373 sondes.

### LEGENDE DES TABLEAUX

**Tableau 1**. Tableau associé aux 5578 séquences cibles (SEQ ID NO : 1-5578) et aux différents panels ou sous-groupes. Colonne A : Références GWSA des séquences cibles. Colonne B : SEQ ID NO. Colonne C : Numéro Gene ID (Genbank). Colonnes D à F : Séquences cibles présentes (1) dans les paires des groupes TSP 1300 SS, TSP NN et TSP N. Colonnes G à BD. Panels 1 à 50. Colonnes BE à BG. Meilleures séquences cibles issues des analyses ANOVA. Colonne BH. Cibles associées aux nouvelles isoformes de RT- PRC, SORL1 et PSEN1. Colonnes BI à BN : Panels 55 à 60.

**Tableau 2**. Tableau associé aux meilleures paires de probesets. Colonne A :Références GWSA des paires. Colonne B : Référence GWSA du premier probe set dans chaque paire. Colonne C : Référence GWSA du deuxième probe set dans chaque paire. Colonne D : SEQ ID NO du premier probe set. Colonne E : SEQ ID NO du deuxième probe set. Colonne F : SEQ ID NO des deux probe sets présents dans la paire (séparés par un « _ »). Colonne G : Score des paires de TSP 1300 SS. Colonne H : Score des paires de TSP NN. Colonne I : Score des paires de TSP N.

### Exemple 1: Identification de marqueurs sériques de la maladie d'Alzheimer

### 1.1. Caractéristiques des échantillons biologiques

Les exemples présentés ci après ont été réalisés initialement à partir de 150 échantillons sanguins (5 ml de sang total, prélevé dans deux tubes PaxGene). Ces échantillons regroupaient 80 patients diagnostiqués d'une maladie d'Alzheimer probable selon les critères du DSM IV. Ces patients correspondaient à un age moyen de 72,9 ans (écart-type : 5,5 ans).Ces patients présentaient un score MMSE (Mini-Mental State Examination) inférieur à 20 (score moyen de 13,7 ; écart-type de 6,7) et un score GDS (Global Deterioration Scale) supérieur ou égal à 4. Par ailleurs 70 sujets, d'âge comparable aux patients AD, déclarés non déménts après un examen clinique ont également été recrutés (age moyen de 68,2 ans avec un écart-type de 6,3 ans ; MMSE moyen de 29,8 avec un écart-type de 0,4).

### 1.2. Extraction des ARN totaux de l'échantillon sanguin

Les prélèvements sanguins ont été collectés directement dans des tubes PAXGene^{™} Blood RNA (PreAnalytix, Hombrechtikon, CH). Après l'étape de prélèvement de l'échantillon sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXGene Blood RNA® (PreAnalytix) en respectant les recommandations du fabriquant. Brièvement, les tubes ont été centrifugés (15 min, 3000 g) afin d'obtenir un culot d'acides nucléiques. Ce culot a été lavé et repris dans un tampon contenant de la protéinase K nécessaire à la digestion des protéines (10 min à 55°C). Une nouvelle centrifugation (5 min, 19 000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen, Hilden, Allemagne). La qualité et la quantité des ARN totaux extraits sont évaluées par réalisation d'électrophorégrammes à l'aide du Bioanalyser 2100 d'Agilent en utilisant le kit RNA 600 NanoChip (Agilent Technologies, Santa Clara, CA). Seuls les échantillons remplissant les critères de qualité ont été utilisés pour des analyses ultérieures.

### 1.3. Le micro-array GWSA (Genome Wide SpliceArray)

La détection et la quantification de l'expression exhaustive des transcrits par microarray nécessite l'utilisation d'une configuration de sondes particulière. Tout couple ARN messager de référence / variant d'épissage peut être modélisé en tant que Isoforme longue / Isoforme courte (Figure 1A). Ainsi, un variant d'épissage associé à un saut d'exon sera l'isoforme courte par rapport au variant de référence. Un variant d'épissage comportant un nouvel exon ou une rétention d'intron sera l'isoforme longue par rapport au variant de référence. Les autres événements d'épissage alternatifs (utilisations de sites cryptiques 5' ou 3' d'épissage) peuvent également être modélisés de la même manière.

Le jeu de sondes nécessaire à la mesure de l'expression de variants d'épissage est également indiqué sur la figure lA. Ce jeu est composé de trois sondes « exoniques » traditionnelles F, T et B et de trois sondes de jonction de type exon-exon ou exon-intron C, D et E. Les sondes F et T mesurent l'expression des deux isoformes, les sondes B, C et D l'expression de l'isoforme longue et la sonde E l'expression de la forme courte.

Afin de concevoir toutes ces sondes, il est nécessaire d'identifier les événements d'épissage correspondant aux gènes humains, puis les régions « cibles » à partir desquelles seront dessinées les sondes. Les séquences cibles correspondant aux sondes de jonction C, D et E sont définies par une longueur de 30 nucléotides, 15 nucléotides de part et d'autre de la jonction. Il est ainsi possible de « couvrir » toute jonction par des sondes de 25 nucléotides par exemple de type : 11/14, 12/13, 13/12 ( le signe / représentant la zone de jonction).

La puce GWSA est un microarray capable de fournir une couverture très complète de l'expression du génome humain en prenant en compte l'existence des variants d'épissage. 20,649 gènes humains ont été sélectionnés puis analysés pour identifier des événements d'épissage connus et potentiels associés. Plus de 90% de ces gènes pouvaient être associés à de tels événements. Des sondes telles que décrites ci-dessus ont été dessinées à partir des quelques 140,000 événement d'épissage identifiés. Par ailleurs, l'utilisation de sondes spécifiques d'événements d'épissages correspondant à des sauts uniques d'exons permet également de prédire l'existence de tels événements (Figure 1B). De plus, l'incorporation de sondes de jonction couvrant chaque région exon - exon, exon - intron et intron -exon de chaque gène permet également de caractériser des évenements d'épissages affectant les extrémités 5' et 3' des exons (Figure 1B). Trois sondes différentes ont été conçues par séquence cible et regroupées en « Probe Set ». Les valeurs d'expression des sondes individuelles sont consolidées en une valeur de Probe Set. Parfois, le Probe Set ne contiendra que 2 voire 1 sonde si les restrictions sur la séquence cible étaient trop contraignantes. Le GWSA est un microarray à façon construit sur la plateforme GeneChip® d'Affymetrix à une résolution de 5 microns. Le GWSA a passé un contrôle qualité selon les normes MAQC (Microarray Quality Control ; Shi et al., Nat Biotechnol. 2006; 24(9): 1151-61).

### 1.4. Amplification de l'ARN

50 ng d'ARN total ont servi de matrice pour la synthèse de cibles à l'aide du kit WT-Ovation^{™} Pico RNA Amplification System (NuGen, San Carlos, CA). Le module FL-Ovation^{™} cDNA Biotin Module (NuGen, San, Carlos, CA) a ensuite été utilisé pour réaliser la fragmentation de 5 µg de cDNA amplifié ainsi que le marquage à la biotine. Les différentes étapes ont été réalisées en suivant les instructions du fabricant. Chaque série d'amplification/fragmentation/marquage contenait autant d'échantillons correspondant à des patients AD et contrôle.

La qualité et la quantité des ADN complémentaires extraits sont évaluées par réalisation d'électrophorégrammes à l'aide du Bioanalyser 2100 d'Agilent en utilisant le kit RNA 600 NanoChip (Agilent Technologies, Santa Clara, CA).

### 1.5. Hybridation sur la lame GWSA

5 µg de cDNA amplifiés et marqués à la biotine sont utilisés par hybridation. Les méthodes standard recommandées par Affymetrix (Affymetrix, Santa Clara, CA) ont été appliquées pour l'hybridation des cibles sur le microarray GWSA. Les puces à ADN ont ensuite été lavées et l'hybridation spécifique révélée en suivant les recommandations d'Affymetrix. Les signaux d'hybridation ont été détectés à l'aide du scanner GeneChip^{R}3000 7G.

### 1.6. Extraction des données et normalisation

Les fichiers .CEL obtenus après scanning des lames ont ensuite été importés dans Partek Genomic Suites^{™} (Partek Incorporated, St Louis, MI) pour une normalisation quantile des lames, un ajustement du bruit de fond en fonction la composition en GC des oligonucléotides sondes, une correction du bruit de fond par RMA et une consolidation des valeurs d'expression mesurée pour chaque oligonucléotide en probe set.

Les données ont ensuite été filtrées par rapport aux valeurs d'expression des probe sets, le seuil de niveau d'expression a été fixé à 3.8 (échelle log 2) en se basant sur la fréquence de distribution des valeurs d'expression sur l'ensemble des lames.

### 1.7. Analyse des données pour sélection de signatures et des sondes de normalisation

### 1.7.1 Sélection des signatures à partir d'expression absolue de sondes.

Un premier groupe de 100 individus (53 AD et 47 contrôles) a été crée parmi les 150 patients de la cohorte pour l'apprentissage de signature(s) discriminante(s) par des analyses de clustering supervisé en utilisant des algorithmes de classification binaire tels que Support Vector Machine (SVM), K-Nearest Neighbor (KNN) et Linear Discriminant Analysis (LDA) et des validations croisées (cross validation) avec des partitions de 10 (Partek Genomic Suites^{™}). Des tests non paramétriques (1-way et 2-ways ANOVA) ont été réalisés pour l'analyse des valeurs d'expression obtenues par probe set et servent d'outil de filtrage pour la sélection de variables qui intégreront la signature. Une permière signature de 170 marqueurs a ainsi été caractérisée et évaluée sur un groupe de patients test indépendant (cf 8.1). Par la suite 4 autres groupes d'apprentissage ont également été crées pour moyenner un effet éventuel du choix spécifique d'un premier groupe d'apprentissage. Ainsi, 5 groupes de 100 patients (53 AD and 47 Contrôles) parmi les 150 patients de la cohorte ont été constitués pour l'apprentissage de signature(s) discriminante(s) par des analyses de clustering supervisé en utilisant des algorithmes de classification binaire tels que Support Vector Machine (SVM), K-Nearest Neighbor (KNN) et Linear Discriminant Analysis (LDA) et des validations croisées (cross validation) avec des partitions de 10 (Partek Genomic Suites^{™} ). Des tests non paramétriques (1-way et 2-ways ANOVA) ont été réalisés pour l'analyse des valeurs d'expression obtenues par probe set et servent d'outil de filtrage pour la sélection de variables qui intégreront la signature. Le modèle de classification optimal par groupe d'apprentissage est ensuite appliqué au groupe test correspondant constitué de 50 patients (27 AD and 23 Contrôles) pour l'identification des performances de la signature à savoir, la spécificité (% de bon classement des patients contrôles) et la sensibilité (% de bon classement des patients AD).

3770 modèles ont été testés sur chaque groupe d'apprentissage, les performances de chaque modèle ont été évaluées sur l'ensemble des groupes d'apprentissage. Les modèles ont ensuite été classés par rapport à la moyenne des performances sur les 5 groupes. Les signatures présentant les moyennes de performances les plus élevées ont été sélectionnées pour discriminer entre les patients atteints de la maladie d'Alzheimer et les patients contrôles non déments.

### 1.7.2 Sélection des signatures à partir d'expression relative de paires de sondes.

Ce type d'analyse appelé TSP consiste à selectionner des probe sets en paires dont l'expression relative est inversée entre les deux groupes qui sont comparés, AD et Contrôle (Geman et al., 2004 Stat Appl Genet Mol Biol 3 : Article 19). L'utilisation de k paires de probe sets est une variante dénommée k-TSP (Tan et al., 2005 Bioinformatics 21 : 3896-3904). Pour chaque gène, une liste de toutes les paires de probe sets est créée. Les niveaux d'expression de chaque probe set dans une paire sont comparés par échantillon/Microarray. La probabilité pour que le premier probe set ait un niveau d'expression supérieur au second est calculée pour chacun des deux groupes, AD et Contrôle. La différence de ces deux probabilités permet de définir un score. La « Top-Scoring-Pair » (TSP) ou les "k-TOP-Scoring-Pairs » correspondent au meilleur ou aux k-meilleurs scores. Une analyse par permutation est ensuite effectuée 1000 fois en définissant deux groupes au hasard à partir des mêmes 150 individus. Le score maximum obtenu par une paire parmi ces 1000 permutations peut ainsi être comparé au meilleurs scores obtenus à partir des groupes AD et Contrôle réels, permettant de porter un jugement sur la relevance statistique de tels scores.

### 1.7.3 Sélection des sondes de normalisation.

Les probe sets de normalisation remplissent plusieurs conditions :
- ils ne sont pas statistiquement significatifs d'une différence entre AD et Contrôles
- leur variance est faible avant et après normalisation
- la distribution de leurs intensités est comparable à l'ensemble de la lame après normalisation

Les probes sets statistiquement significatifs d'une différence entre les patients AD et les patients contrôles avec un False Discovery Rate de 0.01 ont été éliminés des fichiers normalisé et non normalisé contenant les valeurs d'expression des probe sets correspondant aux 150 patients filtrés avec une valeur seuil de 3.8 (log2).

La variance a ensuite été calculée pour chaque probe set à partir des fichiers normalisés et non normalisés. Les probe sets communs aux deux fichiers et présentant les valeurs de variance les plus faibles ont été retenus.

Ces probe sets ont été répertoriés par classes d'intensités en fonction de la distribution des intensités mesurées sur le fichier normalisé et filtré. Les probe sets ont ensuite été sélectionnés dans chaque classe d'intensité par rapport aux valeurs de p-value décroissantes issues du test statistique ANOVA.

### Exemple 2. Mise en évidence de profils d'expression pour le diagnostic de la maladie d'Alzheimer à partir d'échantillon sanguins

### 2.1. Mise en évidence d'un profil d'expression permettant de discriminer les patients Contrôles non déméntes (CND) des patients atteints de la maladies d'Alzheimer (AD).

L'expression de l'ensemble du transcriptome humain, représentant environ 21,000 gènes, a été analysée et comparée entre des patients AD et CND à l'aide du microarray GWSA. L'ensemble des analyses mentionnées dans les paragraphes 7.1 et 7.2 a permis dé mettre en évidence un ensemble de 5578 séquences cibles, pertinents selon l'invention (cf tableau 1, SEQ ID Nos: 1-5578).

Ces séquences sont composées en partie de trois groupes de probe sets provenant d'analyses ANOVA à un ou deux paramètres (1-way or 2-way).

Plus précisement il s'agit du groupe ou panel 51 (tableau 1) représenté par SEQ ID No : 16; 18; 19; 21; 22; 38; 39; 40; 41; 42; 43; 44; 45; 46; 47; 48; 62; 67; 73; 79; 80; 83; 84; 86; 88; 89; 94; 108; 109; 110; 113; 119; 125; 126; 127; 128; 143; 150; 156; 157; 164; 165; 175; 178; 179; 180; 182; 187; 190; 191; 213; 260; 261; 262; 263; 271; 272; 275; 281; 284; 294; 295; 308; 310; 311; 312; 313; 314; 321; 322; 323; 327; 337; 341; 343; 347; 348; 350; 351; 353; 355; 356; 357; 362; 366; 370; 372; 376; 377; 378; 379; 388; 394; 396; 399; 400; 402; 404; 405; 406; 408; 409; 410; 413; 416; 421; 423; 425; 426; 441; 442; 443; 444; 445; 446; 448; 449; 450; 451; 452; 453; 454; 455; 456; 457; 458; 462; 465; 466; 467; 468; 469; 470; 471; 472; 473; 474; 475; 501; 502; 503; 513; 514; 515; 519; 520; 523; 524; 528; 532; 533; 535; 537; 540; 541; 543; 547; 556; 565; 566; 567; 568; 572; 581; 582; 583; 590; 592; 595; 600; 602; 603; 606; 607; 608; 609; 613; 618; 620; 634; 635; 636; 637; 641; 646; 647; 654; 655; 656; 657; 659; 660; 663; 667; 669; 676; 684; 686; 687; 688; 695; 696; 704; 705; 706; 707; 709; 710; 713; 720; 731; 732; 737; 739; 742; 754; 760; 769; 775; 776; 778; 782; 783; 784; 785; 786; 787; 790; 796; 808; 809; 810; 811; 826; 833; 836; 839; 852; 857; 867; 868; 891; 912; 913; 914; 915; 916; 922; 923; 924; 925; 927; 928; 930; 93 1; 932; 933; 934; 935; 936; 940; 948; 950; 951; 952; 956; 957; 976; 977; 978; 979; 980; 982; 983; 984; 985; 986; 988; 1014; 1015; 1016; 1017; 1019; 1020; 1029; 1030; 1031; 1037; 1040; 1041; 1042; 1046; 1049; 1075; 1076; 1077; 1078; 1079; 1080; 1095; 1096; 1100; 1101; 1106; 1108; 1109; 1110; 1112; 1113; 1114; 1115; 1116; 1121; 1140; 1145; 1148; 1149; 1152; 1154; 1156; 1158; 1163; 1164; 1165; 1167; 1169; 1171; 1172; 1173; 1175; 1178; 1179; 1186; 1187; 1191; 1207; 1215; 1230; 1239; 1241; 1260; 1277; 1278; 1286; 1294; 1304; 1309; 1332; 1333; 1354; 1355; 1356; 1357; 1358; 1359; 1361; 1366; 1395; 1396; 1408; 1415; 1416; 1420; 1422; 1428; 1439; 1445; 1472; 1473; 1474; 1484; 1487; 1489; 1490; 1493; 1498; 1509; 1510; 1512; 1513; 1515; 1516; 1517; 1518; 1520; 1521; 1525; 1526; 1527; 1528; 1529; 1530; 1532; 1533; 1535; 1560; 1562; 1563; 1566; 1575; 1576; 1577; 1578; 1579; 1580; 1581; 1582; 1583; 1584; 1589; 1597; 1598; 1599; 1600; 1619; 1621; 1623; 1627; 1634; 1635; 1639; 1640; 1649; 1651; 1652; 1653; 1669; 1670; 1671; 1672; 1673; 1675; 1680; 1689; 1694; 1696; 1697; 1709; 1710; 1713; 1717; 1721; 1722; 1725; 1729; 1732; 1734; 1741; 1742; 1743; 1744; 1747; 1748; 1749; 1753; 1756; 1762; 1763; 1764; 1765; 1770; 1771; 1772; 1773; 1774; 1775; 1776; 1779; 1780; 1781; 1785; 1787; 1788; 1790; 1791; 1792; 1793; 1794; 1800; 1801; 1802; 1805; 1824; 1825; 1826; 1827; 1828; 1829; 1830; 1831; 1833; 1834; 1837; 1839; 1840; 1844; 1845; 1847; 1851; 1855; 1856; 1857; 1858; 1861; 1862; 1910; 1913; 1914; 1915; 1916; 1917; 1918; 1919; 1935; 1938; 1942; 1946; 1947; 1948; 1950; 1952; 1953; 1956; 1961; 1966; 1969; 1971; 1972; 1976; 1977; 1978; 1983; 1984; 1995; 1996; 1999; 2000; 2002; 2012; 2020; 2021; 2025; 2041; 2045; 2047; 2050; 2051; 2060; 2063; 2064; 2087; 2088; 2090; 2093; 2097; 2098; 2099; 2102; 2109; 2113; 2127; 2128; 2129; 2130; 2132; 2135; 2136; 2143; 2144; 2159; 2160; 2161; 2164; 2166; 2177; 2178; 2182; 2204; 2214; 2215; 2216; 2217; 2235; 2241; 2242; 2243; 2267; 2268; 2270; 2271; 2272; 2278; 2279; 2283; 2305; 2306; 2311; 2312; 2313; 2314; 2315; 2316; 2317; 2318; 2319; 2321; 2322; 2323; 2324; 2325; 2326; 2327; 2328; 2331; 2332; 2336; 2341; 2343; 2344; 2346; 2347; 2348; 2361; 2362; 2368; 2371; 2372; 2373; 2375; 2376; 2377; 2379; 2380; 2381; 2382; 2383; 2384; 2389; 2391; 2392; 2395; 2396; 2397; 2399; 2405; 2406; 2407; 2408; 2409; 2410; 2411; 2412; 2413; 2414; 2415; 2417; 2418; 2441; 2443; 2444; 2445; 2446; 2447; 2451; 2456; 2458; 2460; 2465; 2469; 2472; 2486; 2493; 2494; 2520; 2521; 2524; 2526; 2527; 2528; 2529; 2530; 2531; 2532; 2533; 2534; 2536; 2538; 2539; 2543; 2556; 2563; 2577; 2578; 2579; 2582; 2583; 2584; 2586; 2587; 2588; 2589; 2590; 2591; 2592; 2600; 2601; 2603; 2606; 2612; 2613; 2616; 2617; 2618; 2636; 2648; 2658; 2660; 2676; 2679; 2680; 2684; 2691; 2692; 2694; 2695; 2696; 2697; 2698; 2699; 2700; 2701; 2702; 2703; 2725; 2727; 2728; 2740; 2741; 2742; 2743; 2762; 2763; 2765; 2770; 2771; 2772; 2773; 2775; 2776; 2783; 2791; 2793; 2795; 2796; 2800; 2803; 2804; 2809; 2810; 2828; 2843; 2844; 2850; 2851; 2852; 2853; 2860; 2862; 2863; 2865; 2867; 2871; 2872; 2874; 2879; 2886; 2890; 2892; 2893; 2894; 2895; 2897; 2898; 2899; 2902; 2904; 2905; 2906; 2907; 2909; 2910; 2914; 2915; 2916; 2917; 2919; 2920; 2922; 2924; 2925; 2930; 2933; 2934; 2935; 2936; 2937; 2938; 2939; 2940; 2941; 2943; 2944; 2945; 2946; 2947; 2950; 2951; 2953; 2954; 2983; 2991; 2997; 3018; 3028; 3035; 3036; 3037; 3041; 3042; 3043; 3055; 3056; 3057; 3061; 3065; 3073; 3074; 3075; 3076; 3085; 3086; 3089; 3102; 3105; 3108; 3109; 3110; 3111; 3118; 3150; 3161; 3164; 3165; 3166; 3167; 3175; 3188; 3191; 3194; 3195; 3196; 3205; 3207; 3215; 3219; 3221; 3223; 3224; 3225; 3226; 3233; 3234; 3235; 3252; 3260; 3270; 3273; 3277; 3280; 3285; 3286; 3297; 3298; 3301; 3302; 3303; 3307; 3308; 3310; 3311; 3312; 3314; 3315; 3322; 3323; 3324; 3326; 3330; 3335; 3341; 3344; 3345; 3346; 3349; 3350; 3358; 3360; 3361; 3362; 3366; 3373; 3377; 3378; 3379; 3380; 3384; 3406; 3409; 3410; 3411; 3412; 3413; 3420; 3421; 3427; 3428; 3434; 3442; 3443; 3452; 3461; 3474; 3480; 3483; 3487; 3489; 3490; 3491; 3495; 3500; 3505; 3507; 3509; 3517; 3521; 3523; 3524; 3532; 3534; 3536; 3539; 3541; 3542; 3553; 3558; 3559; 3562; 3563; 3564; 3572; 3574; 3576; 3577; 3578; 3579; 3581; 3582; 3583; 3586; 3587; 3588; 3595; 3596; 3597; 3598; 3599; 3600; 3601; 3602; 3607; 3608; 3609; 3610; 3615; 3627; 3628; 3629; 3632; 3639; 3646; 3649; 3650; 3657; 3671; 3673; 3696; 3705; 3707; 3708; 3709; 3732; 3733; 3743; 3748; 3751; 3752; 3753; 3758; 3761; 3762; 3772; 3773; 3775; 3776; 3777; 3778; 3783; 3789; 3790; 3793; 3796; 3799; 3806; 3808; 3819; 3820; 3849; 3853; 3855; 3856; 3857; 3859; 3862; 3863; 3864; 3871; 3884; 3889; 3890; 3912; 3913; 3915; 3919; 3920; 3921; 3929; 3931; 3932; 3934; 3937; 3938; 3939; 3940; 3943; 3944; 3950; 3951; 3952; 3953; 3955; 3956; 3958; 3961; 3962; 3963; 3964; 3965; 3969; 3973; 3974; 3977; 3990; 3991; 3992; 3995; 4001; 4004; 4005; 4006; 4009;.4010; 4011; 4012; 4014; 4015; 4051; 4053; 4054; 4063; 4064; 4070; 4078; 4091; 4092; 4093; 4095; 4096; 4097; 4111; 4115; 4119; 4121; 4154; 4158; 4170; 4171; 4175; 4193; 4198; 4200; 4201; 4202; 4206; 4207; 4208; 4209; 4219; 4221; 4222; 4227; 4228; 4229; 4230; 4232; 4234; 4255; 4260; 4266; 4280; 4282; 4292; 4293; 4296; 4297; 4298; 4299; 4300; 4306; 4307; 4308; 4309; 4312; 4316; 4327; 4330; 4331; 4336; 4337; 4340; 4341; 4342; 4343; 4344; 4345; 4346; 4347; 4348; 4349; 4350; 4351; 4352; 4357; 4358; 4364; 4365; 4378; 4379; 4380; 4386; 4398; 4399; 4400; 4426; 4430; 4431; 4432; 4435; 4438; 4469; 4472; 4475; 4476; 4482; 4488; 4492; 4499; 4505; 4506; 4532; 4547; 4548; 4549; 4550; 4551; 4554; 4555; 4556; 4558; 4559; 4582; 4592; 4593; 4594; 4595; 4603; 4607; 4609; 4611; 4612; 4613; 4617; 4618; 4619; 4621; 4623; 4624; 4629; 4656; 4661; 4662; 4663; 4666; 4671; 4672; 4674; 4675; 4676; 4677; 4678; 4679; 4684; 4693; 4694; 4695; 4698; 4710; 4713; 4718; 4732; 4733; 4734; 4735; 4738; 4744; 4749; 4765; 4768; 4769; 4771; 4779; 4780; 4781; 4782; 4783; 4789; 4790; 4798; 4799; 4805; 4808; 4809; 4810; 4812; 4813; 4816; 4823; 4824; 4826; 4827; 4828; 4831; 4833; 4851; 4856; 4859; 4860; 4861; 4862; 4865; 4867; 4868; 4869; 4873; 4874; 4876; 4883; 4884; 4896; 4905; 4908; 4909; 4910; 4917; 4920; 4921; 4922; 4923; 4924; 4926; 4927; 4928; 4929; 4932; 4933; 4934; 4935; 4938; 4943; 4944; 4946; 4948; 4949; 4951; 4952; 4953; 4954; 4955; 4957; 4958; 4960; 4962; 4964; 4966; 4967; 4970; 4973; 4980; 4985; 4987; 4998; 5000; 5001; 5004; 5011; 5012; 5015; 5016; 5017; 5018; 5020; 5022; 5024; 5025; 5026; 5029; 5030; 5031; 5037; 5038; 5039; 5041; 5042; 5043; 5044; 5045; 5046; 5047; 5048; 5052; 5053; 5055; 5057; 5058; 5061; 5067; 5069; 5080; 5084; 5096; 5098; 5099; 5100; 5101; 5104; 5109; 5111; 5112; 5116; 5122; 5133; 5134; 5135; 5136; 5137; 5138; 5139; 5140; 5144; 5145; 5146; 5154; 5155; 5157; 5158; 5159; 5160; 5163; 5165; 5166; 5167; 5174; 5175; 5180; 5182; 5186; 5188; 5190; 5191; 5195; 5196; 5197; 5198; 5199; 5200; 5202; 5203; 5204; 5206; 5209; 5210; 5211; 5212; 5221; 5231; 5238; 5239; 5244; 5245; 5246; 5247; 5254; 5257; 5265; 5269; 5271; 5288; 5290; 5317; 5325; 5329; 5335; 5336; 5337; 5353; 5365; 5370; 5372; 5374; 5376; 5380; 5383; 5384; 5396; 5407; 5408; 5409; 5410; 5411; 5412; 5413; 5414; 5415; 5425; 5428; 5439; 5440; 5441; 5443; 5465; 5466; 5469; 5494; 5495; 5499; 5500; 5518; 5520; 5521; 5522; 5523; 5524; 5525; 5528; 5530; 5531; 5532; 5533; 5534; 5535; 5537; 5538; 5539; 5540; 5553; 5555; 5564; 5569; 5575; 5576; 5577

Tous ces probe sets présentent une p-value (AD vs Contrôle) inférieure à 3,19 e⁻⁰⁸

Il s'agit également d'un second groupe ou panel 52 représenté par les SEQ ID No : 1; 6; 8; 28; 59; 60; 61; 83; 90; 91; 121; 133; 181; 182; 224; 311; 314; 321; 323; 331; 339; 340; 342; 343; 354; 356; 397; 417; 418; 420; 429; 446; 449; 453; 455; 473; 522; 538; 596; 598; 600; 601; 604; 611; 613; 636; 638; 661; 690; 696; 720; 724; 738; 742; 762; 788; 845; 852; 926; 939; 990; 1050; 1146; 1150; 1151; 1152; 1153; 1154; 1160; 1185; 1203; 1206; 1214; 1215; 1237; 1239; 1242; 1322; 1326; 1357; 1394; 1397; 1398; 1413; 1414; 1422; 1489; 1490; 1501; 1511; 1531; 1539; 1552; 1559; 1588; 1592; 1609; 1637; 1641; 1650; 1658; 1671; 1675; 1683; 1692; 1694; 1695; 1699; 1702; 1711; 1723; 1729; 1730; 1731; 1740; 1741; 1742; 1743; 1744; 1747; 1749; 1756; 1789; 1800; 1806; 1831; 1832; 1833; 1834; 1836; 1837; 1850; 1853; 1859; 1939; 1951; 1964; 1972; 1985; 1994; 1995; 1999; 2022; 2023; 2024; 2038; 2060; 2061; 2062; 2068; 2073; 2078; 2081; 2086; 2090; 2091; 2111; 2116; 2117; 2118; 2119; 2120; 2121; 2122; 2123; 2124; 2125; 2126; 2127; 2128; 2129; 2130; 2132; 2133; 2143; 2144; 2146; 2147; 2148; 2169; 2187; 2190; 2191; 2192; 2195; 2196; 2209; 2284; 2351; 2352; 2353; 2358; 2360; 2365; 2366; 2367; 2369; 2370; 2371; 2372; 2374; 2375; 2378; 2380; 2382; 2394; 2399; 2402; 2411; 2412; 2435; 2454; 2458; 2459; 2461; 2462; 2465; 2469; 2472; 2488; 2489; 2490; 2518; 2528; 2529; 2532; 2533; 2534; 2536; 2538; 2542; 2543; 2544; 2545; 2546; 2548; 2556; 2557; 2558; 2559; 2561; 2563; 2564; 2575; 2585; 2590; 2642; 2649; 2652; 2659; 2679; 2684; 2692; 2695; 2703; 2708; 2709; 2710; 2714; 2724; 2725; 2727; 2729; 2732; 2740; 2745; 2790; 2811; 2813; 2865; 2887; 2888; 2900; 2902; 2908; 2921; 2928; 2941; 2942; 2943; 2944; 2975; 2976; 2983; 3001; 3014; 3018; 3023; 3043; 3045; 3057; 3092; 3104; 3171; 3172; 3173; 3192; 3206; 3211; 3216; 3221; 3243; 3271; 3286; 3301; 3304; 3312; 3325; 3333; 3356; 3365; 3384; 3387; 3388; 3389; 3427; 3429; 3483; 3487; 3491; 3500; 3520; 3526; 3533; 3554; 3559; 3574; 3580; 3600; 3673; 3685; 3686; 3688; 3690; 3692; 3706; 3741; 3751; 3763; 3782; 3805; 3806; 3807; 3808; 3809; 3810; 3811; 3812; 3813; 3819; 3820; 3821; 3822; 3823; 3857; 3859; 3862; 3867; 3904; 3906; 3909; 3920; 3930; 3936; 3940; 3950; 3958; 3961; 3985; 4012; 4013; 4015; 4019; 4076; 4091; 4093; 4094; 4096; 4124; 4130; 4200; 4265; 4295; 4297; 4343; 4345; 4350; 4351; 4352; 4398; 4428; 4434; 4442; 4468; 4471; 4476; 4478; 4479; 4493; 4494; 4497; 4498; 4501; 4510; 4515; 4525; 4546; 4583; 4584; 4591; 4592; 4598; 4603; 4659; 4676; 4706; 4710; 4733; 4737; 4744; 4780; 4793; 4797; 4799; 4804; 4806; 4807; 4860; 4879; 4885; 4887; 4888; 4889; 4890; 4891; 4892; 4895; 4896; 4897; 4898; 4899; 4905; 4907; 4918; 4944; 4955; 4968; 4969; 4975; 4976; 4977; 4978; 4979; 4982; 4999; 5000; 5028; 5031; 5059; 5080; 5125; 5144; 5156; 5170; 5188; 5195; 5196; 5198; 5206; 5221; 5237; 5238; 5245; 5246; 5247; 5249; 5254; 5257; 5285; 5289; 5305; 5320; 5333; 5363; 5371; 5379; 5380; 5383; 5398; 5416; 5421; 5450; 5498; 5501; 5529; 5534; 5535; 5538; 5555; 5562

Tous ces probe sets présentent une p-value (AD vs Contrôle) inférieure à 1,17 e⁻¹²

Il s'agit ensuite d'un troisième groupe ou panel 53 représenté par les SEQ ID No : 15; 17; 20; 25; 26; 50; 51; 52; 58; 66; 75; 87; 98; 99; 101; 103; 105; 114; 115; 116; 117; 118; 120; 166; 167; 171; 173; 216; 278; 285; 300; 325; 338; 354; 365; 371; 397; 398; 403; 407; 424; 459; 473; 476; 482; 506; 509; 510; 511; 516; 517; 518; 521; 527; 536; 539; 569; 570; 571; 591; 593; 597; 598; 599; 614; 617; 619; 629; 661; 664; 666; 668; 677; 689; 690; 692; 693; 694; 711; 721; 722; 726; 751; 768; 777; 788; 789; 791; 842; 869; 872; 893; 894; 895; 896; 905; 921; 1032; 1033; 1043; 1044; 1045; 1070; 1147; 1155; 1157; 1159; 1161; 1162; 1168; 1170; 1174; 1185; 1218; 1220; 1225; 1226; 1229; 1237; 1238; 1239; 1240; 1242; 1253; 1254; 1258; 1259; 1268; 1270; 1346; 1348; 1349; 1362; 1364; 1365; 1401; 1417; 1421; 1432; 1440; 1491; 1494; 1495; 1496; 1522; 1524; 1561; 1588; 1616; 1617; 1618; 1622; 1630; 1632; 1654; 1667; 1668; 1674; 1691; 1692; 1700; 1701; 1718; 1744; 1767; 1784; 1786; 1798; 1807; 1808; 1809; 1810; 1811; 1812; 1813; 1814; 1815; 1816; 1834; 1838; 1841; 1843; 1853; 1859; 1912; 1920; 1951; 1986; 2001; 2008; 2009; 2010; 2011; 2023; 2042; 2085; 2096; 2106; 2107; 2108; 2110; 2111; 2112; 2114; 2115; 2128; 2134; 2137; 2139; 2140; 2141; 2143; 2144; 2148; 2158; 2165; 2167; 2168; 2203; 2207; 2218; 2226; 2246; 2247; 2248; 2249; 2250; 2251; 2252; 2253; 2254; 2255; 2256; 2258; 2260; 2261; 2263; 2264; 2265; 2266; 2269; 2280; 2281; 2307; 2310; 2320; 2329; 2340; 2345; 2350; 2393; 2401; 2411; 2421; 2436; 2442; 2450; 2462; 2517; 2543; 2555; 2560; 2594; 2599; 2602; 2619; 2620; 2621; 2622; 2632; 2633; 2638; 2695; 2714; 2727; 2788; 2789; 2816; 2817; 2818; 2822; 2823; 2824; 2825; 2827; 2831; 2838; 2869; 2870; 2887; 2888; 2889; 2896; 2900; 2903; 2932; 2943; 2979; 2982; 2985; 2994; 3005; 3053; 3066; 3087; 3106; 3107; 3153; 3156; 3157; 3158; 3159; 3160; 3180; 3185; 3186; 3200; 3211; 3217; 3218; 3222; 3266; 3268; 3278; 3279; 3281; 3304; 3305; 3309; 3313; 3317; 3338; 3374; 3375; 3381; 3430; 3462; 3502; 3506; 3508; 3518; 3519; 3520; 3522; 3525; 3527; 3530; 3531; 3535; 3540; 3567; 3573; 3623; 3624; 3625; 3626; 3670; 3672; 3685; 3713; 3734; 3759; 3771; 3782; 3786; 3804; 3814; 3815; 3816; 3824; 3826; 3828; 3829; 3830; 3842; 3843; 3844; 3847; 3848; 3851; 3870; 3872; 3895; 3896; 3897; 3898; 3954; 3960; 3966; 3967; 3968; 3972; 3975; 3976; 3978; 3987; 3993; 4008; 4094; 4096; 4112; 4125; 4164; 4172; 4184; 4185; 4192; 4223; 4233; 4269; 4281; 4359; 4370; 4371; 4375; 4381; 4388; 4398; 4419; 4425; 4427; 4428; 4429; 4433; 4470; 4481; 4483; 4493; 4495; 4502; 4507; 4509; 4553; 4575; 4581; 4610; 4622; 4658; 4660; 4664; 4665; 4688; 4692; 4711; 4712; 4714; 4716; 4722; 4791; 4792; 4857; 4865; 4872; 4959; 4961; 4965; 4974; 4981; 4988; 5019; 5021; 5023; 5027; 5049; 5060; 5065; 5066; 5087; 5107; 5108; 5110; 5119; 5123; 5141; 5164; 5173; 5176; 5181; 5183; 5184; 5185; 5187; 5189; 5198; 5206; 5238; 5241; 5270; 5272; 5273; 5338; 5350; 5391; 5417; 5429; 5434; 5435; 5513; 5529; 5578

Tous ces probe sets présentent une p-value (AD vs Contrôle) inférieure à 6,70 e⁻¹⁵

Les inventeurs ont ensuite étudié l'expression simultanée de probe sets associés à des sousensemble de ces 5578 séquences pour obtenir des profils d'expression relevant de la maladie d'Alzheimer. Un groupe d'apprentissage randomisé de 100 individus (53 AD et 47 NDC) et un algorithme de classification de type SVM a permis d'identifier une signature composée de 170 Probe Sets correspondant au panel 50 (Tableau 1) et au SEQ ID NO : 20; 25; 26; 51; 52; 58; 87; 98; 100; 101; 102; 103; 104; 105; 115; 166; 173; 174; 216; 278; 354; 374; 382; 397; 398; 407; 511; 518; 569; 570; 571; 614; 629; 664; 666; 668; 693; 711; 721; 725; 751; 757; 842; 869; 895; 987; 989; 1033; 1161; 1218; 1229; 1237; 1238; 1240; 1242; 1258; 1259; 1270; 1287; 1346; 1347; 1348; 1362; 1408; 1522; 1590; 1654; 1668; 1694; 1701; 1767; 1786; 1798; 1808; 1809; 1811; 1812; 1813; 1815; 1843; 1859; 1920; 1986; 2008; 2009; 2010; 2011; 2023; 2085; 2110; 2111; 2115; 2218; 2226; 2247; 2256; 2263; 2269; 2281; 2350; 2393; 2411; 2436; 2620; 2788; 2789; 2900; 2943; 2978; 2979; 2993; 2994; 3050; 3103; 3158; 3217; 3266; 3282; 3375; 3508; 3524; 3527; 3531; 3538; 3540; 3552; 3625; 3685; 3713; 3782; 3841; 3898; 3957; 3960; 3966; 3975; 3993; 4007; 4093; 4094; 4096; 4194; 4223; 4370; 4381; 4429; 4433; 4470; 4481; 4483; 4493; 4553; 4560; 4688; 4714; 4716; 4722; 5049; 5107; 5108; 5181; 5183; 5184; 5185; 5198; 5206; 5254;5391;5468;5549

Les performances de cette signature ont été évaluées sur un ensemble TEST d'individus n'ayant pas participé à la définition de cette signature. Ce groupe TEST comportait 27 AD et 23 NDC. Les performances obtenues sont : une sensiblité de 100% et une spécificité de 96%, ie, 27 AD sur 27 ont été bien classés en AD par la signature du panel 50 et 22 NDC sur 23 ont été bien classé en NDC par la signature du panel 50.Une analyse par composante principale permet de visualiser la séparation du groupe AD du groupe contrôle (Figure 2). Les 170 marqueurs de cette signature correspondent à 133 gènes. 31 des 170 probe sets sont spécifiquement associés à des événements d'épissage, principalement des nouveaux exons et des sauts d'exons. Des expériences de RT-PCR ont permis de confirmer la présence de certains de ces transcrits générés par épissage alternatif.

Le répertoire de certains des 133 gènes fournit une base moléculaire précise, non prédictible à certains phénomènes décrits dans la maladie d'Alzheimer : activation des macrophages, voie de signalisation TGF-beta, stress oxydatif, immunité innée, inflamation, réorganisation du cytosquelette, implication des « lipid rafts » au travers notamment des voies de signalisation du cholesterol et des sphingolipides, et implication du système ubiquitine-protéasome.

### 2.2. Caractérisation de sous-panels du panel 50 et performances associées

Les inventeurs ont également étudié l'expression simultanée de sous-ensembles de 100, 50 et 25 probe sets à partir des 170 probe sets associés au panel 50.

### 2.2.1. Caractérisation d'un panel de 100 marqueurs (PANEL 49)

Les inventeurs ont mis en évidence une combinaison de 100 marqueurs, basée sur les séquences SEQ ID Nos : 20; 25; 26; 51; 52; 58; 87; 98; 100; 101; 102; 103; 104; 105; 115; 166; 173; 174; 216; 278; 354; 374; 382; 397; 398; 407; 511; 518; 569; 570; 571; 614; 629; 664; 666; 668; 693; 711; 721; 725; 751; 757; 842; 869; 895; 987; 989; 1033; 1161; 1218; 1229; 1237; 1238; 1240; 1242; 1258; 1259; 1270; 1287; 1346; 1347; 1348; 1362; 1408; 1522; 1590; 1654; 1668; 1694; 1701; 1767; 1786; 1798; 1808; 1809; 1811; 1812; 1813; 1815; 1843; 1859; 1920; 1986; 2008; 2009; 2010; 2011; 2023; 2085; 2110; 2111; 2115; 2218; 2226; 2247; 2256; 2263; 2269; 2281; 2350 (voir tableau 1).

Cette combinaison produit une sensibilité de 81% et une spécificité de 91% sur le groupe TEST.

### 2.2.2. Caractérisation d'un panel de 50 marqueurs (PANEL 48)

Les inventeurs ont mis en évidence une combinaison de 50 marqueurs, basée sur les séquences SEQ ID Nos : 20; 25; 26; 51; 52; 58; 87; 98; 100; 101; 102; 103; 104; 105; 115; 166; 173; 174; 216; 278; 354; 374; 382; 397; 398; 407; 511; 518; 569; 570; 571; 614; 629; 664; 666; 668; 693; 711; 721; 725; 751; 757; 842; 869; 895; 987; 989; 1033; 1161; 1218 (voir tableau 1).

Cette combinaison produit une sensibilité de 70% et une spécificité de 87% sur le groupe TEST.

### 2.2.3. Caractérisation d'un panel de 25 marqueurs (PANEL 47)

Les inventeurs ont mis en évidence une combinaison de 25 marqueurs, basée sur les séquences SEQ ID Nos : 20; 25; 26; 51; 52; 58; 87; 98; 100; 101; 102; 103; 104; 105; 115; 166; 173; 174; 216; 278; 354; 374; 382; 397; 398 (voir tableau 1).

Cette combinaison produit une sensibilité de 89% et une spécificité de 61% sur le groupe TEST.

Ces éléments indiquent que ces sous-signatures gardent un pouvoir diagnostique qui est néanmoins inférieur à la signature mère de 170 marqueurs du panel 50.

Les inventeurs ont ensuite caractérisés différents panels de marqueurs à partir de 5 groupes d'apprentissage / test différents qui sont maintenant décrits dans les paragraphes 8.3 à 8.54.

### 2.3. Identification d'un panel prédictif de 160 marqueurs (PANEL 1)

Les inventeurs ont mis en évidence une combinaison de 160 marqueurs, liée à un algorithme de type KNN, basée sur les séquences SEQ ID Nos : 1; 28; 73; 321; 323; 350; 397; 417; 418; 612; 720; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1800; 1831; 1833; 1834; 1837; 1951; 1952; 1953; 1984; 1995; 2023; 2038; 2117; 2126; 2127; 2128; 2129; 2130; 2133; 2144; 2284; 2305; 2365; 2372; 2378; 2380; 2382; 2399; 2411; 2458; 2459; 2462; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2943; 2944; 3043; 3057; 3092; 3286; 3384; 3387; 3596; 3600; 3690; 3782; 3810; 3811; 3822; 3857; 3904; 3920; 3940; 3958; 4076; 4093; 4094; 4096; 4130; 4295; 4297; 4345; 4351; 4428; 4434; 4442; 4468; 4493; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5067; 5080; 5144; 5198; 5221; 5238; 5380; 5383; 5450; 5532; 5555 (voir tableau 1). Cette combinaison permet de classifier correctement 93,3 % des échantillons.

### 2.4. Identification d'un panel prédictif de 280 marqueurs (PANEL 2)

Les inventeurs ont mis en évidence une combinaison de 280 marqueurs, liée à un algorithme de type KNN, basée sur les séquences SEQ ID Nos : 1; 6; 8; 28; 73; 80; 84; 90; 224; 314; 321; 323; 331; 343; 345; 349; 350; 352; 397; 417; 418; 429; 513; 538; 542; 600; 612; 629; 720; 723; 724; 738; 742; 788; 852; 978; 990; 991; 1107; 1152; 1154; 1169; 1212; 1237; 1239; 1242; 1260; 1295; 1326; 1489; 1490; 1511; 1641; 1683; 1694; 1711; 1714; 1723; 1731; 1732; 1741; 1744; 1747; 1749; 1789; 1800; 1806; 1831; 1833; 1834; 1836; 1837; 1859; 1861; 1950; 1951; 1952; 1953; 1984; 1985; 1994; 1995; 2022; 2023; 2038; 2090; 2091; 2117; 2126; 2127; 2128; 2129; 2130; 2132; 2133; 2143; 2144; 2192; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2562; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3056; 3057; 3061; 3070; 3092; 3173; 3221; 3242; 3265; 3286; 3384; 3387; 3388; 3429; 3487; 3500; 3520; 3574; 3576; 3596; 3600; 3673; 3688; 3690; 3732; 3751; 3767; 3782; 3807; 3809; 3810; 3811; 3819; 3820; 3822; 3857; 3862; 3904; 3920; 3936; 3940; 3950; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4592; 4596; 4603; 4607; 4676; 4706; 4736; 4744; 4804; 4807; 4860; 4885; 4886; 4887; 4888; 4889; 4890; 4891; 4892; 4895; 4897; 4898; 4899; 4918; 4955; 4975; 4976; 4978; 4979; 4982; 5000; 5017; 5031; 5067; 5080; 5109; 5125; 5144; 5147; 5188; 5196; 5198; 5221; 5238; 5247; 5249; 5305; 5333; 5373; 5380; 5383; 5416; 5450; 5501; 5519; 5521; 5529; 5532; 5534; 5555 (voir tableau 1).

Cette combinaison permet de classifier correctement 93,0 % des échantillons.

### 2.5. Identification d'un panel prédictif de 260 marqueurs (PANEL 3)

Les inventeurs ont mis en évidence une combinaison de 260 marqueurs, liée à un algorithme de type KNN, basée sur les séquences SEQ ID Nos : 1; 6; 8; 28; 73; 80; 84; 90; 224; 314; 321; 323; 331; 343; 345; 349; 350; 352; 397; 417; 418; 429; 513; 538; 542; 600; 612; 629; 720; 723; 738; 742; 788; 852; 978; 990; 991; 1152; 1154; 1169; 1212; 1237; 1239; 1242; 1295; 1326; 1489; 1490; 1511; 1641; 1683; 1694; 1711; 1714; 1723; 1731; 1732; 1741; 1744; 1747; 1749; 1789; 1800; 1806; 1831; 1833; 1834; 1836; 1837; 1859; 1861; 1950; 1951; 1952; 1953; 1984; 1985; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2562; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3057; 3061; 3070; 3092; 3173; 3221; 3242; 3265; 3286; 3384; 3387; 3388; 3429; 3500; 3520; 3574; 3596; 3600; 3673; 3690; 3732; 3751; 3767; 3782; 3807; 3809; 3810; 3811; 3819; 3820; 3822; 3857; 3862; 3904; 3920; 3936; 3940; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4596; 4603; 4706; 4736; 4744; 4804; 4807; 4860; 4885; 4887; 4888; 4889; 4890; 4891; 4892; 4897; 4898; 4899; 4918; 4955; 4975; 4976; 4978; 4982; 5017; 5031; 5067; 5080; 5109; 5125; 5144; 5147; 5188; 5196; 5198; 5221; 5238; 5249; 5305; 5373; 5380; 5383; 5416; 5450; 5501; 5519; 5529; 5532; 5534; 5555 (voir tableau 1).

Cette combinaison permet de classifier correctement 93,0 % des échantillons.

### 2.6. Identification d'un panel prédictif de 170 marqueurs (PANEL 4)

Les inventeurs ont mis en évidence une combinaison de 170 marqueurs, liée à un algorithme de type KNN, basée sur les séquences SEQ ID Nos : 1; 28; 73; 321; 323; 350; 397; 417; 418; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1950; 1951; 1952; 1953; 1984; 1994; 1995; 2023; 2038; 2117; 2126; 2127; 2128; 2129; 2130; 2133; 2144; 2284; 2305; 2365; 2372; 2375; 2378; 2380; 2382; 2399; 2406; 2411; 2458; 2459; 2462; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3596; 3600; 3690; 3782; 3807; 3810; 3811; 3822; 3857; 3862; 3904; 3920; 3940; 3958; 4019; 4076; 4093; 4094; 4096; 4130; 4295; 4297; 4345; 4351; 4428; 4434; 4442; 4468; 4493; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5067; 5080; 5144; 5198; 5221; 5238; 5380; 5383; 5450; 5532; 5555 (voir tableau 1).

Cette combinaison permet de classifier correctement 93,0 % des échantillons.

### 2.7. Identification d'un panel prédictif de 200 marqueurs (PANEL 5)

Les inventeurs ont mis en évidence une combinaison de 200 marqueurs, liée à un algorithme de type KNN, basée sur les séquences SEQ ID Nos : 1; 6; 8; 28; 73; 90; 321; 323; 343; 350; 352; 397; 417; 418; 513; 538; 542; 600; 612; 720; 738; 788; 852; 990; 991; 1152; 1154; 1169; 1212; 1237; 1239; 1242; 1326; 1489; 1490; 1511; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3001; 3043; 3057; 3092; 3286; 3384; 3387; 3388; 3429; 3574; 3596; 3600; 3673; 3690; 3767; 3782; 3807; 3810; 3811; 3822; 3857; 3862; 3904; 3920; 3936; 3940; 3958; 3985; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1).

Cette combinaison permet de classifier correctement 92,8 % des échantillons.

### 2.8. Identification d'un panel prédictif de 140 marqueurs (PANEL 6)

Les inventeurs ont mis en évidence une combinaison de 140 marqueurs, liée à un algorithme de type KNN, basée sur les séquences SEQ ID Nos : 1; 28; 73; 321; 323; 350; 417; 418; 720; 852; 990; 1152; 1154; 1169; 1212; 1237; 1239; 1242; 1326; 1489; 1490; 1683; 1694; 1711; 1723; 1731; 1741; 1744; 1749; 1800; 1833; 1834; 1837; 1951; 1952; 1953; 1984; 1995; 2023; 2038; 2117; 2126; 2127; 2128; 2129; 2130; 2133; 2144; 2284; 2305; 2365; 2372; 2378; 2380;.2382; 2399; 2411; 2459; 2462; 2528; 2529; 2532; 2533; 2542; 2543; 2556; 2563; 2564; 2652; 2679; 2703; 2709; 2725; 2727; 2732; 2740; 2887; 2888; 2900; 2941; 2943; 2944; 3043; 3057; 3286; 3387; 3596; 3600; 3690; 3782; 3810; 3811; 3822; 3857; 3904; 3920; 3940; 3958; 4076; 4093; 4094; 4096; 4130; 4295; 4345; 4351; 4428; 4434; 4442; 4468; 4493; 4510; 4546; 4603; 4744; 4804; 4860; 4885; 4888; 4889; 4890; 4891; 4892; 4897; 4898; 4899; 4918; 4955; 4975; 4976; 4978; 4982; 5144; 5198; 5221; 5238; 5380; 5383; 5450; 5532 (voir tableau 1).

Cette combinaison permet de classifier correctement 92,8 % des échantillons.

### 2.9. Identification d'un panel prédictif de 110 marqueurs (PANEL 7)

Les inventeurs ont mis en évidence une combinaison de 110 marqueurs, liée à un algorithme de type KNN, basée sur les séquences SEQ ID Nos : 1; 28; 73; 321; 323; 418; 852; 1152; 1154; 1237; 1242; 1326; 1683; 1694; 1731; 1741; 1744; 1749; 1833; 1834; 1951; 1984; 1995; 2023; 2038; 2126; 2127; 2128; 2129; 2130; 2133; 2144; 2284; 2305; 2365; 2372; 2378; 2380; 2382; 2411; 2459; 2462; 2528; 2529; 2532; 2533; 2542; 2543; 2556; 2563; 2679; 2703; 2725; 2727; 2732; 2740; 2887; 2888; 2900; 2941; 2943; 2944; 3043; 3057; 3286; 3387; 3596; 3600; 3782; 3822; 3857; 3904; 3920; 3940; 3958; 4093; 4094; 4096; 4130; 4295; 4345; 4351; 4428; 4442; 4468; 4493; 4510; 4603; 4744; 4804; 4860; 4885; 4888; 4889; 4890; 4891; 4897; 4898; 4899; 4918; 4975; 4976; 4978; 4982; 5144; 5198; 5221; 5238; 5380; 5383 (voir tableau 1).

Cette combinaison permet de classifier correctement 92,8 % des échantillons.

### 2.10. Identification d'un panel prédictif de 190 marqueurs (PANEL 8)

Les inventeurs ont mis en évidence une combinaison de 190 marqueurs, liée à un algorithme de type KNN, basée sur les séquences SEQ ID Nos : 1; 6; 28; 73; 321; 323; 343; 350; 352; 397; 417; 418; 513; 538; 542; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943;.2944; 3043; 3057; 3092; 3286; 3384; 3387; 3574; 3596; 3600; 3673; 3690; 3767; 3782; 3807; 3810; 3811; 3822; 3857; 3862; 3904; 3920; 3936; 3940; 3958; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1). Cette combinaison permet de classifier correctement 92,8 % des échantillons.

### 2.11. Identification d'un panel prédictif de 240 marqueurs (PANEL 9)

Les inventeurs ont mis en évidence une combinaison de 240 marqueurs, liée à un algorithme de type KNN, basée sur les séquences SEQ ID Nos : 1; 6; 8; 28; 73; 80; 90; 224; 314; 321; 323; 331; 343; 345; 349; 350; 352; 397; 417; 418; 429; 513; 538; 542; 600; 612; 720; 723; 738; 742; 788; 852; 978; 990; 991; 1152; 1154; 1169; 1212; 1237; 1239; 1242; 1295; 1326; 1489; 1490; 1511; 1641; 1683; 1694; 1711; 1723; 1731; 1741; 1744; 1747; 1749; 1789; 1800; 1806; 1831; 1833; 1834; 1837; 1859; 1861; 1950; 1951; 1952; 1953; 1984; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2743; 2812; 2879; 2887; 2888; 2900; 2941; 2942; 2943; 2944; 2975; 3001; 3043; 3057; 3061; 3070; 3092; 3173; 3242; 3286; 3384; 3387; 3388; 3429; 3520; 3574; 3596; 3600; 3673; 3690; 3751; 3767; 3782; 3807; 3809; 3810; 3811; 3820; 3822; 3857; 3862; 3904; 3920; 3936; 3940; 3958; 3985; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4596; 4603; 4706; 4736; 4744; 4804; 4860; 4885; 4887; 4888; 4889; 4890; 4891; 4892; 4897; 4898; 4899; 4918; 4955; 4975; 4976; 4978; 4982; 5017; 5031; 5067; 5080; 5109; 5125; 5144; 5147; 5196; 5198; 5221; 5238; 5373; 5380; 5383; 5416; 5450; 5501; 5529; 5532; 5534; 5555 (voir tableau 1).

Cette combinaison permet de classifier correctement 92,8 % des échantillons.

### 2.12. Identification d'un panel prédictif de 200 marqueurs (PANEL 10)

Les inventeurs ont mis en évidence une combinaison de 200 marqueurs, liée à un algorithme de type KNN, basée sur les séquences SEQ ID Nos : 1; 6; 8; 28; 73; 90; 321; 323; 343; 350; 352; 397; 417; 418; 513; 538; 542; 600; 612; 720; 738; 788; 852; 990; 991; 1152; 1154; 1169; 1212; 1237; 1239; 1242; 1326; 1489; 1490; 1511; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3001; 3043; 3057; 3092; 3286; 3384; 3387; 3388; 3429; 3574; 3596; 3600; 3673; 3690; 3767; 3782; 3807; 3810; 3811; 3822; 3857; 3862; 3904; 3920; 3936; 3940; 3958; 3985; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1).

Cette combinaison permet de classifier correctement 92,5 % des échantillons.

### 2.13. Identification d'un panel prédictif de 190 marqueurs (PANEL 11)

Les inventeurs ont mis en évidence une combinaison de 190 marqueurs, liée à un algorithme de type KNN, basée sur les séquences SEQ ID Nos : 1; 6; 28; 73; 321; 323; 343; 350; 352; 397; 417; 418; 513; 538; 542; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3574; 3596; 3600; 3673; 3690; 3767; 3782; 3807; 3810; 3811; 3822; 3857; 3862; 3904; 3920; 3936; 3940; 3958; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1). Cette combinaison permet de classifier correctement 92,5 % des échantillons.

### 2.14. Identification d'un panel prédictif de 210 marqueurs (PANEL 12)

Les inventeurs ont mis en évidence une combinaison de 210 marqueurs, liée à un algorithme de type KNN, basée sur les séquences SEQ ID Nos : 1; 6; 8; 28; 73; 90; 321; 323; 343; 345; 350; 352; 397; 417; 418; 513; 538; 542; 600; 612; 720; 738; 788; 852; 990; 991; 1152; 1154; 1169; 1212; 1237; 1239; 1242; 1326; 1489; 1490; 1511; 1641; 1683; 1694; 1711; 1723; 1731; 1741; 1744; 1747; 1749; 1789; 1800; 1806; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2743; 2887; 2888; 2900; 2941; 2942; 2943; 2944; 3001; 3043; 3057; 3092; 3173; 3286; 3384; 3387; 3388; 3429; 3574; 3596; 3600; 3673; 3690; 3767; 3782; 3807; 3810; 3811; 3820; 3822; 3857; 3862; 3904; 3920; 3936; 3940; 3958; 3985; 4019; 4076; 4093; 4094; 4096; 4130; 4295; 4297; 4345; 4350; 4351; 4399; 4428; 4434; 4442; 4468; 4493; 4497; 4510; 4515; 4546; 4596; 4603; 4706; 4736; 4744; 4804; 4860; 4885; 4887; 4888; 4889; 4890; 4891; 4892; 4897; 4898; 4899; 4918; 4955; 4975; 4976; 4978; 4982; 5017; 5067; 5080; 5144; 5147; 5198; 5221; 5238; 5373; 5380; 5383; 5416; 5450; 5529; 5532; 5555 (voir tableau 1).

Cette combinaison permet de classifier correctement 92,5 % des échantillons.

### 2.15. Identification d'un panel prédictif de 260 marqueurs (PANEL 13)

Les inventeurs ont mis en évidence une combinaison de 260 marqueurs, liée à un algorithme de type KNN, basée sur les séquences SEQ ID Nos : 1; 6; 8; 28; 73; 80; 84; 90; 224; 314; 321; 323; 331; 343; 345; 349; 350; 352; 397; 417; 418; 429; 513; 538; 542; 600; 612; 629; 720; 723; 738; 742; 788; 852; 978; 990; 991; 1152; 1154; 1169; 1212; 1237; 1239; 1242; 1295; 1326; 1489; 1490; 1511; 1641; 1683; 1694; 1711; 1714; 1723; 1731; 1732; 1741; 1744; 1747; 1749; 1789; 1800; 1806; 1831; 1833; 1834; 1836; 1837; 1859; 1861; 1950; 1951; 1952; 1953; 1984; 1985; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 25.43; 2556; 2562; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3057; 3061; 3070; 3092; 3173; 3221; 3242; 3265; 3286; 3384; 3387; 3388; 3429; 3500; 3520; 3574; 3596; 3600; 3673; 3690; 3732; 3751; 3767; 3782; 3807; 3809; 3810; 3811; 3819; 3820; 3822; 3857; 3862; 3904; 3920; 3936; 3940; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4596; 4603; 4706; 4736; 4744; 4804; 4807; 4860; 4885; 4887; 4888; 4889; 4890; 4891; 4892; 4897; 4898; 4899; 4918; 4955; 4975; 4976; 4978; 4982; 5017; 5031; 5067; 5080; 5109; 5125; 5144; 5147; 5188; 5196; 5198; 5221; 5238; 5249; 5305; 5373; 5380; 5383; 5416; 5450; 5501; 5519; 5529; 5532; 5534; 5555 (voir tableau 1).

Cette combinaison permet de classifier correctement 92,5 % des échantillons.

### 2.16. Identification d'un panel prédictif de 180 marqueurs (PANEL 14)

Les inventeurs ont mis en évidence une combinaison de 180 marqueurs, liée à un algorithme de type KNN, basée sur les séquences SEQ ID Nos : 1; 28; 73; 321; 323; 350; 352; 397; 417; 418; 513; 538; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2133; 2143; 2144; 2284; 2305; 2365; 2372; 2375; 2378; 2380; 2382; 2390; 2399; 2406; 2411; 2458; 2459; 2462; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387;.3574; 3596; 3600; 3673; 3690; 3782; 3807; 3810; 3811; 3822; 3857; 3862; 3904; 3920; 3936; 3940; 3958; 4019; 4076; 4093; 4094; 4096; 4130; 4295; 4297; 4345; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5067; 5080; 5144; 5198; 5221; 5238; 538U; 5383; 5450; 5532; 5555 (voir tableau 1).

Cette combinaison permet de classifier correctement 92,5 % des échantillons.

### 2.17. Identification d'un panel prédictif de 190 marqueurs (PANEL 15)

Les inventeurs ont mis en évidence une combinaison de 190 marqueurs, liée à un algorithme de type KNN, basée sur les séquences SEQ ID Nos : 1; 6; 28; 73; 321; 323; 343; 350; 352; 397; 417; 418; 513; 538; 542; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3574; 3596; 3600; 3673; 3690; 3767; 3782; 3807; 3810; 3811; 3822; 3857; 3862; 3904; 3920; 3936; 3940; 3958; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1). Cette combinaison permet de classifier correctement 92,5 % des échantillons.

### 2.18. Identification d'un panel prédictif de 250 marqueurs (PANEL 16)

Les inventeurs ont mis en évidence une combinaison de 250 marqueurs, liée à un algorithme de type KNN, basée sur les séquences SEQ ID Nos : 1; 6; 8; 28; 73; 80; 90; 224; 314; 321; 323; 331; 343; 345; 349; 350; 352; 397; 417; 418; 429; 513; 538; 542; 600; 612; 629; 720; 723; 738; 742; 788; 852; 978; 990; 991; 1152; 1154; 1169; 1212; 1237; 1239; 1242; 1295; 1326; 1489; 1490; 1511; 1641; 1683; 1694; 1711; 1723; 1731; 1732; 1741; 1744; 1747; 1749; 1789; 1800; 1806; 1831; 1833; 1834; 1837; 1859; 1861; 1950; 1951; 1952; 1953; 1984; 1985; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3057; 3061; 3070; 3092; 3173; 3221; 3242; 3286; 3384; 3387; 3388; 3429; 3520; 3574; 3596; 3600; 3673; 3690; 3751; 3767; 3782; 3807; 3809; 3810; 3811; 3819; 3820; 3822; 3857; 3862; 3904; 3920; 3936; 3940; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4596; 4603; 4706; 4736; 4744; 4804; 4807; 4860; 4885; 4887; 4888; 4889; 4890; 4891; 4892; 4897; 4898; 4899; 4918; 4955; 4975; 4976; 4978; 4982; 5017; 5031; 5067; 5080; 5109; 5125; 5144; 5147; 5188; 5196; 5198; 5221; 5238; 5373; 5380; 5383; 5416; 5450; 5501; 5529; 5532; 5534; 5555 (voir tableau 1).

Cette combinaison permet de classifier correctement 92,5 % des échantillons.

### 2.19. Identification d'un panel prédictif de 250 marqueurs (PANEL 17)

Les inventeurs ont mis en évidence une combinaison de 250 marqueurs, liée à un algorithme de type KNN, basée sur les séquences SEQ ID Nos : 1; 6; 8; 28; 73; 80; 90; 224; 314; 321; 323; 331; 343; 345; 349; 350; 352; 397; 417; 418; 429; 513; 538; 542; 600; 612; 629; 720; 723; 738; 742; 788; 852; 978; 990; 991; 1152; 1154; 1169; 1212; 1237; 1239; 1242; 1295; 1326; 1489; 1490; 1511; 1641; 1683; 1694; 1711; 1723; 1731; 1732; 1741; 1744; 1747; 1749; 1789; 1800; 1806; 1831; 1833; 1834; 1837; 1859; -1861; 1950; 1951;1952;1953;1984;1985;1994;1995;2023;2038;2090;2091;2117;2126;2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3057; 3061; 3070; 3092; 3173; 3221; 3242; 3286; 3384; 3387; 3388; 3429; 3520; 3574; 3596; 3600; 3673; 3690; 3751; 3767; 3782; 3807; 3809; 3810; 3811; 3819; 3820; 3822; 3857; 3862; 3904; 3920; 3936; 3940; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4596; 4603; 4706; 4736; 4744; 4804; 4807; 4860; 4885; 4887; 4888; 4889; 4890; 4891; 4892; 4897; 4898; 4899; 4918; 4955; 4975; 4976; 4978; 4982; 5017; 5031; 5067; 5080; 5109; 5125; 5144; 5147; 5188; 5196; 5198; 5221; 5238; 5373; 5380; 5383; 5416; 5450; 5501; 5529; 5532; 5534; 5555 (voir tableau 1).

Cette combinaison permet de classifier correctement 92,5 % des échantillons.

### 2.20. Identification d'un panel prédictif de 110 marqueurs (PANEL 18)

Les inventeurs ont mis en évidence une combinaison de 110 marqueurs, liée à un algorithme de type KNN, basée sur les séquences SEQ ID Nos : 1; 28; 73; 321; 323; 418; 852; 1152; 1154; 1237; 1242; 1326; 1683; 1694; 1731; 1741; 1744; 1749; 1833; 1834; 1951; 1984; 1995; 2023; 2038; 2126; 2127; 2128; 2129; 2130; 2133; 2144; 2284; 2305; 2365; 2372; 2378; 2380; 2382; 2411; 2459; 2462; 2528; 2529; 2532; 2533; 2542; 2543; 2556; 2563; 2679; 2703; 2725; 2727; 2732; 2740; 2887; 2888; 2900; 2941; 2943; 2944; 3043; 3057; 3286; 3387; 3596; 3600; 3782; 3822; 3857; 3904; 3920; 3940; 3958; 4093; 4094; 4096; 4130; 4295; 4345; 4351; 4428; 4442; 4468; 4493; 4510; 4603; 4744; 4804; 4860; 4885; 4888; 4889; 4890; 4891; 4897; 4898; 4899; 4918; 4975; 4976; 4978; 4982; 5144; 5198; 5221; 5238; 5380; 5383 (voir tableau 1).

Cette combinaison permet de classifier correctement 92,5 % des échantillons.

### 2.21. Identification d'un panel prédictif de 220 marqueurs (PANEL 19)

Les inventeurs ont mis en évidence une combinaison de 220 marqueurs, liée à un algorithme de type KNN, basée sur les séquences SEQ ID Nos : 1; 6; 8; 28; 73; 80; 90; 321; 323; 343; 345; 350; 352; 397; 417; 418; 513; 538; 542; 600; 612; 720; 738; 788; 852; 978; 990; 991; 1152; 1154; 1169; 1212; 1237; 1239; 1242; 1295; 1326; 1489; 1490; 1511; 1641; 1683; 1694; 1711; 1723; 1731; 1741; 1744; 1747; 1749; 1789; 1800; 1806; 1831; 1833; 1834; 1837; 1859; 1950; 1951; 1952; 1953; 1984; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2743; 2812; 2887; 2888; 2900; 2941; 2942; 2943; 2944; 3001; 3043; 3057; 3061; 3092; 3173; 3242; 3286; 3384; 3387; 3388; 3429; 3574; 3596; 3600; 3673; 3690; 3767; 3782; 3807; 3810; 3811; 3820; 3822; 3857; 3862; 3904; 3920; 3936; 3940; 3958; 3985; 4019; 4051; 4076; 4093; 4094; 4096; 4130; 4295; 4297; 4345; 4350; 4351; 4399; 4428; 4434; 4442; 4468; 4493; 4497; 4510; 4515; 4546; 4596; 4603; 4706; 4736; 4744; 4804; 4860; 4885; 4887; 4888; 4889; 4890; 4891; 4892; 4897; 4898; 4899; 4918; 4955; 4975; 4976; 4978; 4982; 5017; 5067; 5080; 5144; 5147; 5198; 5221; 5238; 5373; 5380; 5383; 5416; 5450; 5529; 5532; 5534; 5555 (voir tableau 1).

Cette combinaison permet de classifier correctement 92,5 % des échantillons.

### 2.22. Identification d'un panel prédictif de 270 marqueurs (PANEL 20)

Les inventeurs ont mis en évidence une combinaison de 270 marqueurs, liée à un algorithme de type KNN, basée sur les séquences SEQ ID Nos : 1; 6; 8; 28; 73; 80; 84; 90; 224; 314; 321; 323; 331; 343; 345; 349; 350; 352; 397; 417; 418; 429; 513; 538; 542; 600; 612; 629; 720; 723; 724; 738; 742; 788; 852; 978; 990; 991; 1107; 1152; 1154; 1169; 1212; 1237; 1239; 1242; 1260; 1295; 1326; 1489; 1490; 1511; 1641; 1683; 1694; 1711; 1714; 1723; 1731; 1732; 1741; 1744; 1747; 1749; 1789; 1800; 1806; 1831; 1833; 1834; 1836; 1837; 1859; 1861; 1950; 1951; 1952; 1953; 1984; 1985; 1994; 1995; 2023; 2038; 2090; 2091; 2117; 2126; 2127; 2128; 2129; 2130; 2132; 2133; 2143; 2144; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2562; 2563; 2564; 2575; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3056; 3057; 3061; 3070; 3092; 3173; 3221; 3242; 3265; 3286; 3384; 3387; 3388; 3429; 3500; 3520; 3574; 3576; 3596; 3600; 3673; 3690; 3732; 3751; 3767; 3782; 3807; 3809; 3810; 3811; 3819; 3820; 3822; 3857; 3862; 3904; 3920; 3936; 3940; 3950; 3958; 3985; 4017; 4019; 4051; 4076; 4093; 4094; 4096; 4130; 4295; 4297; 4345; 4350; 4351; 4399; 4428; 4434; 4442; 4468; 4493; 4497; 4510; 4515; 4523; 4546; 4592; 4596; 4603; 4706; 4736; 4744; 4804; 4807; 4860; 4885; 4887; 4888; 4889; 4890; 4891; 4892; 4897; 4898; 4899; 4918; 4955; 4975; 4976; 4978; 4979; 4982; 5017; 5031; 5067; 5080; 5109; 5125; 5144; 5147; 5188; 5196; 5198; 5221; 5238; 5249; 5305; 5333; 5373; 5380; 5383; 5416; 5450; 5501; 5519; 5521; 5529; 5532; 5534; 5555 (voir tableau 1).

Cette combinaison permet de classifier correctement 92,5 % des échantillons.

### 2.23. Identification d'un panel prédictif de 290 marqueurs (PANEL 21)

Les inventeurs ont mis en évidence une combinaison de 290 marqueurs, liée à un algorithme de type KNN, basée sur les séquences SEQ ID Nos : 1; 6; 8; 28; 55; 73; 80; 84; 90; 224; 314; 321; 323; 331; 343; 345; 349; 350; 352; 397; 417; 418; 429; 513; 538; 542; 600; 612; 629; 720; 723; 724; 738; 742; 788; 852; 978; 990; 991; 1107; 1152; 1154; 1169; 1212; 1237; 1239; 1242; 1260; 1295; 1326; 1489; 1490; 1511; 1641; 1683; 1694; 1699; 1711; 1714; 1723; 1731; 1732; 1741; 1744; 1747; 1749; 1789; 1800; 1806; 1831; 1833; 1834; 1836; 1837; 1859; 1861; 1950; 1951; 1952; 1953; 1984; 1985; 1994; 1995; 2022; 2023; 2038; 2090; 2091; 2117; 2126; 2127; 2128; 2129; 2130; 2132; 2133; 2143; 2144; 2192; 2209; 2284; 2305; 2365; 2372; 2374; 2375; 2378; 2380; 2382; 2390; 2399; 2406; 2411; 2458; 2459; 2462; 2469; 2528; 2529; 2532; 2533; 2534; 2538; 2542; 2543; 2556; 2562; 2563; 2564; 2575; 2590; 2652; 2679; 2703; 2708; 2709; 2725; 2727; 2732; 2740; 2743; 2812; 2879; 2887; 2888; 2900; 2941; 2942; 2943; 2944; 2975; 3001; 3018; 3043; 3056; 3057; 3061; 3070; 3092; 3172; 3173; 3221; 3242; 3265; 3286; 3384; 3387; 3388; 3429; 3487; 3500; 3520; 3574; 3576; 3596; 3600; 3673; 3688; 3690; 3732; 3751; 3767; 3782; 3807; 3809; 3810; 3811; 3819; 3820; 3822; 3857; 3862; 3904; 3920; 3936; 3940; 3950; 3958; 3985; 3996; 4017; 4019; 4051; 4076; 4093; 4094; 4096; 4130; 4295; 4297; 4343; 4345; 4350; 4351; 4399; 4428; 4434; 4442; 4468; 4493; 4497; 4498; 4510; 4515; 4523; 4546; 4592; 4596; 4603; 4607; 4676; 4706; 4736; 4744; 4804; 4807; 4860; 4885; 4886; 4887; 4888; 4889; 4890; 4891; 4892; 4895; 4897; 4898; 4899; 4918; 4955; 4975; 4976; 4978; 4979; 4982; 4986; 5000; 5017; 5031; 5067; 5080; 5109; 5125; 5144; 5147; 5188; 5196; 5198; 5221; 5237; 5238; 5239; 5247; 5249; 5305; 5333; 5373; 5380; 5383; 5416; 5450; 5501; 5519; 5521; 5529; 5532; 5534; 5555 (voir tableau 1).

Cette combinaison permet de classifier correctement 92,5 % des échantillons.

### 2.24. Identification d'un panel prédictif de 270 marqueurs (PANEL 22)

Les inventeurs ont mis en évidence une combinaison de 270 marqueurs, liée à un algorithme de type SVM, basée sur les séquences SEQ ID Nos : 26; 51; 52; 58; 63; 75; 98; 101; 103; 105; 114; 117; 120; 163; 166; 171; 172; 277; 278; 300; 325; 338; 354; 397; 398; 401; 403; 407; 419; 421; 424; 482; 511; 512; 518; 527; 531; 539; 569; 570; 571; 591; 596; 597; 598; 614; 619; 629; 658; 662; 668; 689; 690; 691; 694; 719; 721; 722; 789; 842; 872; 949; 958; 1032; 1033; 1159; 1162; 1166; 1169; 1174; 1214; 1218; 1220; 1229; 1237; 1238; 1240; 1242; 1253; 1257; 1258; 1259; 1270; 1307; 1308; 1346; 1348; 1398; 1432; 1440; 1494; 1495; 1522; 1523; 1551; 1561; 1588; 1624; 1632; 1654; 1668; 1674; 1701; 1724; 1744; 1767; 1786; 1809; 1811; 1841; 1853; 1859; 1951; 1986; 2001; 2003; 2007; 2009; 2010; 2011; 2106; 2107; 2110; 2111; 2112; 2114; 2115; 2144; 2148; 2158; 2185; 2249; 2250; 2251; 2252; 2253; 2260; 2263; 2269; 2281; 2307; 2310; 2320; 2333; 2349; 2350; 2400; 2421; 2442; 2462; 2519; 2594; 2599; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3566; 3567; 3575; 3625; 3626; 3672; 3685; 3782; 3814; 3816; 3824; 3828; 3842; 3846; 3847; 3848; 3897; 3912; 3933; 3960; 3987; 3993; 4172; 4182; 4183; 4192; 4233; 4257; 4281; 4370; 4381; 4419; 4425; 4428; 4429; 4468; 4480; 4489; 4502; 4507; 4553; 4574; 4575; 4587; 4622; 4660; 4665; 4711; 4721; 4722; 4736; 4857; 4872; 4900; 4961; 5019; 5027; 5049; 5107; 5141; 5161; 5176; 5183; 5184; 5268; 5272; 5273; 5331; 5334; 5343; 5375; 5391; 5417; 5470; 5529 (voir tableau 1). Cette combinaison permet de classifier correctement 96,2 % des échantillons.

### 2.25. Identification d'un panel prédictif de 250 marqueurs (PANEL 23)

Les inventeurs ont mis en évidence une combinaison de 250 marqueurs, liée à un algorithme de type SVM, basée sur les séquences SEQ ID Nos : 26; 52; 58; 75; 98; 101; 103; 105; 114; 120; 163; 166; 171; 172; 277; 278; 300; 325; 354; 397; 398; 401; 403; 407; 419; 421; 424; 482; 511; 512; 518; 527; 531; 539; 569; 570; 571; 591; 596; 597; 598; 614; 619; 629; 658; 662; 668; 689; 690; 691; 694; 719; 721; 722; 789; 842; 872; 949; 1033; 1159; 1162; 1166; 1169; 1174; 1214; 1218; 1220; 1229; 1237; 1238; 1240; 1242; 1253; 1257; 1258; 1259; 1270; 1307; 1308; 1346; 1348; 1398; 1432; 1440; 1494; 1495; 1522; 1523; 1551; 1561; 1588; 1632; 1654; 1668; 1674; 1701; 1724; 1744; 1786; 1809; 1811; 1841; 1853; 1859; 1951; 1986; 2001; 2007; 2009; 2010; 2011; 2107; 2110; 2111; 2112; 2114; 2115; 2144; 2148; 2158; 2185; 2249; 2250; 2251; 2252; 2253; 2260; 2263; 2269; 2281; 2307; 2310; 2320; 2333; 2349; 2350; 2400; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3912; 3933; 3960; 3987; 3993; 4172; 4182; 4183; 4192; 4233; 4257; 4281; 4370; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5049; 5107; 5141; 5176; 5183; 5184; 5268; 5272; 5273; 5331; 5334; 5343; 5375; 5391; 5417; 5470; 5529 (voir tableau 1).

Cette combinaison permet de classifier correctement 96,0 % des échantillons.

### 2.26. Identification d'un panel prédictif de 260 marqueurs (PANEL 24)

Les inventeurs ont mis en évidence une combinaison de 260 marqueurs, liée à un algorithme de type SVM, basée sur les séquences SEQ ID Nos : 26; 52; 58; 63; 75; 98; 101; 103; 105; 114; 117; 120; 163; 166; 171; 172; 277; 278; 300; 325; 338; 354; 397; 398; 401; 403; 407; 419; 421; 424; 482; 511; 512; 518; 527; 531; 539; 569; 570; 571; 591; 596; 597; 598; 614; 619; 629; 658; 662; 668; 689; 690; 691; 694; 719; 721;'722; 789; $42; 872; 949; 1033; 1159; 1162; 1166; 1169; 1174; 1214; 1218; 1220; 1229; 1237; 1238; 1240; 1242; 1253; 1257; 1258; 1259; 1270; 1307; 1308; 1346; 1348; 1398; 1432; 1440; 1494; 1495; 1522; 1523; 1551; 1561; 1588; 1624; 1632; 1654; 1668; 1674; 1701; 1724; 1744; 1786; 1809; 1811; 1841; 1853; 1859; 1951; 1986; 2001; 2003; 2007; 2009; 2010; 2011; 2107; 2110; 2111; 2112; 2114; 2115; 2144; 2148; 2158; 2185; 2249; 2250; 2251; 2252; 2253; 2260; 2263; 2269; 2281; 2307; 2310; 2320; 2333; 2349; 2350; 2400; 2421; 2442; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3566; 3567; 3575; 3625; 3626; 3672; 3685; 3782; 3814; 3816; 3824; 3828; 3842; 3846; 3847; 3848; 3897; 3912; 3933; 3960; 3987; 3993; 4172; 4182; 4183; 4192; 4233; 4257; 4281; 4370; 4381; 4419; 4425; 4428; 4429; 4468; 4480; 4489; 4502; 4507; 4553; 4574; 4575; 4587; 4622; 4660; 4711; 4722; 4736; 4872; 4900; 4961; 5019; 5027; 5049; 5107; 5141; 5176; 5183; 5184; 5268; 5272; 5273; 5331; 5334; 5343; 5375; 5391; 5417; 5470; 5529 (voir tableau 1).

Cette combinaison permet de classifier correctement 96,0 % des échantillons.

### 2.27. Identification d'un panel prédictif de 230 marqueurs (PANEL 25)

Les inventeurs ont mis en évidence une combinaison de 230 marqueurs, liée à un algorithme de type SVM, basée sur les séquences SEQ ID Nos : 26; 52; 58; 75; 98; 101; 103; 105; 114; 120; 163; 166; 171; 172; 277; 278; 300; 325; 354; 397; 398; 403; 407; 419; . 421; 424; 482; 511; 512; 518; 527; 531; 539; 569; 570; 571; 591; 596; 597; 598; 614; 619; 629; 658; 662; 689; 690; 691; 694; 719; 721; 872; 949; 1033; 1159; 1162; 1166; 1169; 1174; 1214; 1218; 1229; 1237; 1238; 1240; 1242; 1257; 1258; 1259; 1270; 1307; 1308; 1346; 1348; 1398; 1432; 1440; 1494; 1495; 1522; 1523; 1551; 1561; 1588; 1632; 1654; 1668; 1674; 1724; 1744; 1786; 1809; 1841; 1853; 1859; 1951; 1986; 2001; 2007; 2009; 2010; 2011; 2110; 2111; 2115; 2144; 2148; 2158; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3933; 3960; 3987; 3993; 4172; 4182; 4183; 4192; 4233; 4257; 4281; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5375; 5391; 5417; 5529 (voir tableau 1).

Cette combinaison permet de classifier correctement 96,0 % des échantillons.

### 2.28. Identification d'un panel prédictif de 240 marqueurs (PANEL 26)

Les inventeurs ont mis en évidence une combinaison de 240 marqueurs, liée à un algorithme de type SVM, basée sur les séquences SEQ ID Nos : 26; 52; 58; 75; 98; 101; 103; 105; 114; 120; 163; 166; 171; 172; 277; 278; 300; 325; 354; 397; 398; 403; 407; 419; 421; 424; 482; 511; 512; 518; 527; 531; 539; 569; 570; 571; 591; 596; 597; 598; 614; 619; 629; 658; 662; 668; 689; 690; 691; 694; 719; 721; 722; 789; 872; 949; 1033; 1159; 1162; 1166; 1169; 1174; 1214; 1218; 1220; 1229; 1237; 1238; 1240; 1242; 1257; 1258; 1259; 1270; 1307; 1308; 1346; 1348; 1398; 1432; 1440; 1494; 1495; 1522; 1523; 1551; 1561; 1588; 1632; 1654; 1668; 1674; 1724; 1744; 1786; 1809; 1841; 1853; 1859; 1951; 1986; 2001; 2007; 2009; 2010; 2011; 2110; 2111; 2115; 2144; 2148; 2158; 2185; 2249; 2250; 2251; 2252; 2253; 2260; 2263; 2269; 2281; 2307; 2310; 2320; 2333; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3912; 3933; 3960; 3987; 3993; 4172; 4182; 4183; 4192; 4233; 4257; 4281; 4370; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5049; 5107; 5141; 5176; 5183; 5184;.5268; 5272; 5331; 5334; 5343; 5375; 5391; 5417; 5470; 5529 (voir tableau 1).

Cette combinaison permet de classifier correctement 95,8 % des échantillons.

### 2.29. Identification d'un panel prédictif de 280 marqueurs (PANEL 27)

Les inventeurs ont mis en évidence une combinaison de 280 marqueurs, liée à un algorithme de type SVM, basée sur les séquences SEQ ID Nos : 26; 51; 52; 58; 63; 75; 98; 101; 103; 105; 114; 117; 120; 163; 166; 171; 172; 277; 278; 300; 325; 338; 354; 397; 398; 401; 403; 407; 419; 421; 424; 482; 511; 512; 518; 527; 531; 539; 569; 570; 571; 591; 596; 597; 598; 614; 619; 629; 658; 662; 668; 689; 690; 691; 694; 719; 721; 722; 789; 842; 872; 921; 949; 958; 1032; 1033; 1159; 1162; 1166; 1169; 1174; 1185; 1214; 1218; 1220; 1229; 1237; 1238; 1240; 1242; 1253; 1257; 1258; 1259; 1270; 1307; 1308; 1346; 1348; 1398; 1432; 1440; 1494; 1495; 1522; 1523; 1551; 1561; 1588; 1624; 1632; 1654; 1668; 1674; 1701; 1724; 1744; 1767; 1786; 1809; 1811; 1841; 1853; 1859; 1951; 1986; 2001; 2003; 2007; 2009; 2010; 2011; 2106; 2107; 2110; 2111; 2112; 2114; 2115; 2144; 2148; 2158; 2185; 2249; 2250; 2251; 2252; 2253; 2260; 2263; 2269; 2281; 2307; 2310; 2320; 2333; 2349; 2350; 2400; 2421; 2442; 2462; 2519; 2594; 2599; 2604; 2619; 2620; 2621; 2622; 2813; 2835; 2838; 2869; 2873; 2887; 2888; 2889; 2891; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506;.3508; 3520; 3525; 3527; 3531; 3565; 3566; 3567; 3575; 3624; 3625; 3626; 3672; 3685; 3782; 3814; 3816; 3824; 3826; 3828; 3842; 3843; 3846; 3847; 3848; 3897; 3912; 3933; 3960; 3987; 3993; 4065; 4172; 4182; 4183; 4192; 4233; 4257; 4281; 4370; 4381; 4419; 4425; 4428; 4429; 4468; 4480; 4489; 4502; 4507; 4553; 4574; 4575; 4587; 4622; 4660; 4665; 4700; 4711; 4721; 4722; 4736; 4857; 4872; 4900; 4961; 5019; 5027; 5049; 5107; 5123; 5141; 5161; 5176; 5183; 5184; 5268; 5272; 5273; 5331; 5334; 5343; 5375; 5391; 5417; 5470; 5529 (voir tableau 1).

Cette combinaison permet de classifier correctement 95,8 % des échantillons.

### 2.30. Identification d'un panel prédictif de 290 marqueurs (PANEL 28)

Les inventeurs ont mis en évidence une combinaison de 290 marqueurs, liée à un algorithme de type SVM, basée sur les séquences SEQ ID Nos : 26; 51; 52; 58; 63; 75; 98; 101; 103; 105; 114; 117; 120; 163; 166; 171; 172; 277; 278; 300; 325; 338; 354; 397; 398; 401; 403; 407; 419; 421; 424; 480; 482; 511; 512; 518; 527; 531; 539; 569; 570; 571; 591; 596; 597; 598; 614; 619; 629; 658; 662; 668; 689; 690; 691; 693; 694; 719; 721; 722; 789; 842; 871; 872; 921; 949; 958; 1032; 1033; 1159; 1162; 1166; 1169; 1174; 1185; 1214; 1218; 1220; 1229; 1237; 1238; 1240; 1242; 1253; 1257; 1258; 1259; 1270; 1307; 1308; 1346; 1348; 1398; 1432; 1440; 1494; 1495; 1522; 1523; 1551; 1561; 1588; 1624; 1632; 1654; 1668; 1674; 1701; 1724; 1744; 1767; 1786; 1809; 1811; 1841; 1853; 1859; 1951; 1986; 2001; 2003; 2007; 2009; 2010; 2011; 2085; 2106; 2107; 2110; 2111; 2112; 2114; 2115; 2144; 2148; 2158; 2185; 2249; 2250; 2251; 2252; 2253; 2260; 2263; 2269; 2281; 2307; 2310; 2320; 2333; 2349; 2350; 2400; 2421; 2442; 2462; 2519; 2594; 2596; 2599; 2604; 2619; 2620; 2621; 2622; 2679; 2813; 2835; 2838; 2869; 2873; 2887; 2888; 2889; 2891; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3520; 3525; 3527; 3531; 3565; 3566; 3567; 3573; 3574; 3575; 3624; 3625; 3626; 3672; 3685; 3782; 3814; 3816; 3824; 3826; 3828; 3842; 3843; 3846; 3847; 3848; 3897; 3912; 3933; 3960; 3987; 3993; 4065; 4172; 4182; 4183; 4192; 4199; 4233; 4257; 4281; 4370; 4381; 4419; 4425; 4428; 4429; 4468; 4480; 4489; 4502; 4507; 4553; 4574; 4575; 4587; 4622; 4660; 4665; 4700; 4711; 4712; 4721; 4722; 4736; 4857; 4872; 4900; 4961; 5019; 5027; 5049; 5107; 5123; 5141; 5161; 5176; 5183; 5184; 5268; 5272; 5273; 5331; 5334; 5343; 5375; 5391; 5417; 5470; 5529 (voir tableau 1).

Cette combinaison permet de classifier correctement 95,8 % des échantillons.

### 2.31. Identification d'un panel prédictif de 220 marqueurs (PANEL 29)

Les inventeurs ont mis en évidence une combinaison de 220 marqueurs, liée à un algorithme de type SVM, basée sur les séquences SEQ ID Nos : 26; 52; 58; 75; 98; 103; 105; 114; 120; 163; 166; 171; 172; 277; 278; 300; 325; 354; 397; 398; 407; 419; 421; 424; 482; 511; 512; 518; 527; 539; 569; 570; 571; 591; 596; 597; 598; 614; 619; 629; 658; 662; 689; 691; 694; 719; 721; 872; 949; 1033; 1159; 1162; 1166; 1169; 1174; 1214; 1218; 1229; 1237; 1238; 1240; 1242; 1257; 1258; 1259; 1270; 1307; 1308; 1346; 1348; 1398; 1432; 1440; 1494; 1495; 1522; 1523; 1551; 1588; 1632; 1654; 1668; 1674; 1724; 1744; 1786; 1809; 1841; 1853; 1859; 1951; 1986; 2001; 2007; 2009; 2010; 2011; 2110; 2115; 2144; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3933; 3960; 3987; 3993; 4172; 4182; 4192; 4233; 4257; 4281; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5375; 5391; 5417; 5529 (voir tableau 1).

Cette combinaison permet de classifier correctement 95,8 % des échantillons.

### 2.32. Identification d'un panel prédictif de 200 marqueurs (PANEL 30)

Les inventeurs ont mis en évidence une combinaison de 200 marqueurs, liée à un algorithme de type SVM, basée sur les séquences SEQ ID Nos : 26; 52; 58; 75; 103; 105; 120; 163; 166; 171; 172; 277; 278; 300; 325; 354; 397; 398; 419; 421; 424; 482; 512; 518; 527; 539; 569; 570; 571; 591; 596; 597; 598; 614; 619; 629; 658; 662; 689; 694; 719; 721; 872; 949; 1033; 1159; 1162; 1166; 1174; 1214; 1218; 1229; 1237; 1238; 1240; 1242; 1258; 1259; 1270; 1307; 1308; 1346; 1348; 1432; 1440; 1494; 1495; 1522; 1523; 1588; 1632; 1654; 1668; 1674; 1724; 1744; 1786; 1841; 1853; 1859; 1951; 1986; 2001; 2007; 2009; 2010; 2110; 2115; 2144; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3266; 3304; 3340; 3376; 3430; 3508; 3525; 3527; 3531; 3565; 3567; 3575; 3625; 3672; 3685; 3782; 3814; 3824; 3828; 3842; 3846; 3847; 3848; 3897; 3960; 3993; 4172; 4182; 4192; 4233; 4257; 4281; 4381; 4419; 4425; 4428; 4429; 4468; 4489; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5391; 5417; 5529 (voir tableau 1).

Cette combinaison permet de classifier correctement 95,8 % des échantillons.

### 2.33. Identification d'un panel prédictif de 230 marqueurs (PANEL 31)

Les inventeurs ont mis en évidence une combinaison de 230 marqueurs, liée à un algorithme de type SVM, basée sur les séquences SEQ ID Nos : 26; 52; 58; 75; 98; 101; 103; 105; 114; 120; 163; 166; 171; 172; 277; 278; 300; 325; 354; 397; 398; 403; 407; 419; 421; 424; 482; 511; 512; 518; 527; 531; 539; 569; 570; 571; 591; 596; 597; 598; 614; 619; 629; 658; 662; 689; 690; 691; 694; 719; 721; 872; 949; 1033; 1159; 1162; 1166; 1169; 1174; 1214; 1218; 1229; 1237; 1238; 1240; 1242; 1257; 1258; 1259; 1270; 1307; 1308; 1346; 1348; 1398; 1432; 1440; 1494; 1495; 1522; 1523; 1551; 1561; 1588; 1632; 1654; 1668; 1674; 1724; 1744; 1786; 1809; 1841; 1853; 1859; 1951; 1986; 2001; 2007; 2009; 2010; 2011; 2110; 2111; 2115; 2144; 2148; 2158; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3933; 3960; 3987; 3993; 4172; 4182; 4183; 4192; 4233; 4257; 4281; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5375; 5391; 5417; 5529 (voir tableau 1).

Cette combinaison permet de classifier correctement 95,8 % des échantillons.

### 2.34. Identification d'un panel prédictif de 230 marqueurs (PANEL 32)

Les inventeurs ont mis en évidence une combinaison de 230 marqueurs, liée à un algorithme de type SVM, basée sur les séquences SEQ ID Nos : 26; 52; 58; 75; 98; 101; 103; 105; 114; 120; 163; 166; 171; 172; 277; 278; 300; 325; 354; 397; 398; 403; 407; 419; 421; 424; 482; 511; 512; 518; 527; 531; 539; 569; 570; 571; 591; 596; 597; 598; 614; 619; 629; 658; 662; 689; 690; 691; 694; 719; 721; 872; 949; 1033; 1159; 1162; 1166; 1169; 1174; 1214; 1218; 1229; 1237; 1238; 1240; 1242; 1257; 1258; 1259; 1270; 1307; 1308; 1346; 1348; 1398; 1432; 1440; 1494; 1495; 1522; 1523; 1551; 1561; 1588; 1632; 1654; 1668; 1674; 1724; 1744; 1786; 1809; 1841; 1853; 1859; 1951; 1986; 2001; 2007; 2009; 2010; 2011; 2110; 2111; 2115; 2144; 2148; 2158; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3296; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3933; 3960; 3987; 3993; 4172; 4182; 4183; 4192; 4233; 4257; 4281; 4381; 4419; 4425; 4428; 4429; 4468; 4480; 4489; 4502; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5375; 5391; 5417; 5529 (voir tableau 1).

Cette combinaison permet de classifier correctement 95,8 % des échantillons.

### 2.35. Identification d'un panel prédictif de 300 marqueurs (PANEL 33)

Les inventeurs ont mis en évidence une combinaison de 300 marqueurs, liée à un algorithme de type SVM, basée sur les séquences SEQ ID Nos : 26; 51; 52; 58; 63; 75; 98; 101; 103; 105; 114; 117; 120; 163; 166; 171; 172; 277; 278; 300; 325; 338; 354; 397; 398; 401; 403; 407; 419; 421; 424; 480; 482; 511; 512; 518; 527; 531; 539; 569; 570; 571; 591; 596; 597; 598; 614; 619; 629; 658; 662; 668; 689; 690; 691; 693; 694; 719; 721; 722; 789; 842; 871; 872; 921; 949; 958; 1032; 1033; 1159; 1162; 1166; 1169; 1174; 1185; 1214; 1218; 1220; 1229; 1237; 1238; 1240; 1242; 1253; 1257; 1258; 1259; 1270; 1307; 1308; 1346; 1348; 1398; 1421; 1432; 1440; 1494; 1495; 1522; 1523; 1551; 1561; 1588; 1624; 1632; 1654; 1668; 1674; 1701; 1724; 1744; 1767; 1786; 1809; 1811; 1841; 1853; 1859; 1951; 1986; 2001; 2003; 2007; 2009; 2010; 2011; 2085; 2106; 2107; 2110; 2111; 2112; 2114; 2115; 2144; 2148; 2158; 2185; 2249; 2250; 2251; 2252; 2253; 2260; 2263; 2266; 2269; 2281; 2307; 2310; 2320; 2333; 2349; 2350; 2400; 2421; 2442; 2462; 2519; 2594; 2596; 2599; 2604; 2619; 2620; 2621; 2622; 2679; 2703; 2813; 2835; 2838; 2869; 2873; 2887; 2888; 2889; 2891; 2896; 2900; 2979; 3005; 3053; 3088; 3106; 3157; 3160; 3185; 3186; 3190; 3193; 3200; 3213; 3218; 3266; 3276; 3278; 3296; 3304; 3340; 3376; 3430; 3506; 3508; 3520; 3525; 3527; 3531; 3565; 3566; 3567; 3573; 3574; 3575; 3624; 3625; 3626; 3672; 3685; 3782; 3814; 3816; 3824; 3826; 3828; 3842; 3843; 3844; 3845; 3846; 3847; 3848; 3897; 3912; 3933; 3960; 3987; 3993; 4065; 4172; 4182; 4183; 4192; 4199; 4233; 4257; 4281; 4370; 4381; 4419; 4425; 4428; 4429; 4468; 4480; 4489; 4502; 4507; 4553; 4574; 4575; 4587; 4622; 4660; 4665; 4700; 4711; 4712; 4721; 4722; 4736; 4857; 4872; 4900; 4961; 5019; 5027; 5049; 5107; 5123; 5141; 5161; 5162; 5176; 5183; 5184; 5185; 5268; 5272; 5273; 5330; 5331; 5334; 5343; 5375; 5391; 5417; 5470; 5513; 5529 (voir tableau 1).

Cette combinaison permet de classifier correctement 95,8 % des échantillons.

### 2.36. Identification d'un panel prédictif de 160 marqueurs (PANEL 34)

Les inventeurs ont mis en évidence une combinaison de 160 marqueurs, liée à un algorithme de type SVM, basée sur les séquences SEQ ID Nos : 26; 58; 75; 103; 105; 120; 166; 171; 172; 278; 325; 354; 397; 398; 419; 421; 424; 482; 518; 527; 539; 570; 571; 591; 597; 598; 619; 629; 658; 662; 689; 721; 872; 1033; 1159; 1162; 1166; 1174; 1218; 1229; 1237; 1238; 1240; 1242; 1258; 1259; 1270; 1307; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2144; 2185; 2249; 2251; 2263; 2269; 2307; 2310; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3160; 3185; 3186; 3190; 3193; 3200; 3213; 3304; 3376; 3430; 3508; 3527; 3531; 3565; 3567; 3575; 3625; 3672; 3685; 3824; 3842; 3846; 3847; 3848; 3897; 3960; 3993; 4172; 4182; 4192; 4257; 4281; 4419; 4425; 4428; 4429; 4468; 4507; 4553; 4660; 4722; 4872; 4900; 4961; 5019; 5027; 5107; 5141; 5176; 5183; 5184; 5268; 5272; 5334; 5343; 5391; 5417 (voir tableau 1).

Cette combinaison permet de classifier correctement 95,6 % des échantillons.

### 2.37. Identification d'un panel prédictif de 150 marqueurs (PANEL 35)

Les inventeurs ont mis en évidence une combinaison de 150 marqueurs, liée à un algorithme de type SVM, basée sur les séquences SEQ ID Nos : 26; 58; 75; 103; 105; 120; 166; 171; 172; 278; 325; 354; 397; 398; 419; 421; 482; 518; 527; 539; 570; 571; 591; 597; 598; 619; 629; 658; 662; 689; 721; 872; 1033; 1159; 1162; 1174; 1218; 1229; 1237; 1238; 1240; 1258; 1259; 1270; 1307; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2249; 2251; 2263; 2269; 2307; 2310; 2349; 2350; 2421; 2462; 2519; 2594; 2619; 2620; 262i; 2622; 2813; 2835; 2838; 2873; 2887; 2888; 2889; 2896; 2900; 2979; 3005; 3053; 3088; 3160; 3185; 3186; 3190; 3193; 3200; 3304; 3376; 3430; 3508; 3527; 3531; 3565; 3567; 3575; 3672; 3685; 3824; 3842; 3846; 3847; 3848; 3897; 3960; 3993; 4172; 4182; 4192; 4257; 4419; 4425; 4428; 4429; 4468; 4507; ₄553; 4660; 4722; 4872; 4961; 5019; 5027; 5107; 5176; 5183; 5184; 5268; 5272; 5334; 5343; 5391; 5417 (voir tableau 1). Cette combinaison permet de classifier correctement 95,6 % des échantillons.

### 2.38. Identification d'un panel prédictif de 170 marqueurs (PANEL 36)

Les inventeurs ont mis en évidence une combinaison de 170 marqueurs, liée à un algorithme de type SVM, basée sur les séquences SEQ ID Nos : 26; 58; 75; 103; 105; 120; 166; 171; 172; 278; 325; 354; 397; 398; 419; 421; 424; 482; 512; 518; 527; 539; 570; 571; 591; 597; 598; 619; 629; 658; 662; 689; 694; 721; 872; 1033; 1159; 1162; 1166; 1174; 1218; 1229; 1237; 1238; 1240; 1242; 1258; 1259; 1270; 1307; 1308; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2144; 2185; 2249; 2251; 2253; 2263; 2269; 2307; 2310; 2320; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3160; 3185; 3186; 3190; 3193; 3200; 3213; 3266; 3304; 3376; 3430; 3508; 3525; 3527; 3531; 3565; 3567; 3575; 3625; 3672; 3685; 3824; 3828; 3842; 3846; 3847; 3848; 3897; 3960; 3993; 4172; 4182; 4192; 4233; 4257; 4281; 4381; 4419; 4425; 4428; 4429; 4468; 4507; 4553; 4660; 4722; 4872; 4900; 4961; 5019; 5027; 5107; 5141; 5176; 5183; 5184; 5268; 5272; 5334; 5343; 5391; 5417 (voir tableau 1).

Cette combinaison permet de classifier correctement 95,6 % des échantillons.

### 2.39. Identification d'un panel prédictif de 180 marqueurs (PANEL 37)

Les inventeurs ont mis en évidence une combinaison de 180 marqueurs, liée à un algorithme de type SVM, basée sur les séquences SEQ ID Nos : 26; 58; 75; 103; 105; 120; 166; 171; 172; 278; 300; 325; 354; 397; 398; 419; 421; 424; 482; 512; 518; 527; 539; 569; 570; 571; 591; 596; 597; 598; 619; 629; 658; 662; 689; 694; 721; 872; 949; 1033; 1159; 1162; 1166; 1174; 1218; 1229; 1237; 1238; 1240; 1242; 1258; 1259; 1270; 1307; 1308; 1346; 1348; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2144; 2185; 2249; 2251; 2253; 2263; 2269; 2307; 2310; 2320; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3106; 3160; 3185; 3186; 3190; 3193; 3200; 3213; 3266; 3304; 3340; 3376; 3430; 3508; 3525; 3527; 3531; 3565; 3567; 3575; 3625; 3672; 3685; 3814; 3824; 3828; 3842; 3846; 3847; 3848; 3897; 3960; 3993; 4172; 4182; 4192; 4233; 4257; 4281; 4381; 4419; 4425; 4428; 4429; 4468; 4507; 4553; 4622; 4660; 4722; 4736; 4872; 4900; 4961; 5019; 5027; 5107; 5141; 5176; 5183; 5184; 5268; 5272; 5334; 5343; 5391; 5417 (voir tableau 1).

Cette combinaison permet de classifier correctement 95,6 % des échantillons.

### 2.40. Identification d'un panel prédictif de 190 marqueurs (PANEL 38)

Les inventeurs ont mis en évidence une combinaison de 190 marqueurs, liée à un algorithme de type SVM, basée sur les séquences SEQ ID Nos : 26; 52; 58; 75; 103; 105; 120; 163; 166; 171; 172; 277; 278; 300; 325; 354; 397; 398; 419; 421; 424; 482; 512; 518; 527; 539; 569; 570; 571; 591; 596; 597; 598; 614; 619; 629; 658; 662; 689; 694; 719; 721; 872; 949; 1033; 1159; 1162; 1166; 1174; 1218; 1229; 1237; 1238; 1240; 1242; 1258; 1259; 1270; 1307; 1308; 1346; 1348; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2144; 2185; 2249; 2251; 2253; 2263; 2269; 2307; 2310; 2320; 2349; 2350; 2421; 2462; 2519; 2594; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3266; 3304; 3340; 3376; 3430; 3508; 3525; 3527; 3531; 3565; 3567; 3575; 3625; 3672; 3685; 3814; 3824; 3828; 3842; 3846; 3847; 3848; 3897; 3960; 3993; 4172; 4182; 4192; 4233; 4257; 4281; 4381; 4419; 4425; 4428; 4429; 4468; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5391; 5417 (voir tableau 1).

Cette combinaison permet de classifier correctement 95,6 % des échantillons.

### 2.41. Identification d'un panel prédictif de 150 marqueurs (PANEL 39)

Les inventeurs ont mis en évidence une combinaison de 150 marqueurs, liée à un algorithme de type SVM, basée sur les séquences SEQ ID Nos : 26; 58; 75; 103; 105; 120; 166; 171; 172; 278; 325; 354; 397; 398; 419; 421; 482; 518; 527; 539; 570; 571; 591; 597; 598; 619; 629; 658; 662; 689; 721; 872; 1033; 1159; 1162; 1174; 1218; 1229; 1237; 1238; 1240; 1258; 1259; 1270; 1307; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2249; 2251; 2263; 2269; 2307; 2310; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3160; 3185; 3186; 3190; 3193; 3200; 3304; 3376; 3430; 3508; 3527; 3531; 3565; 3567; 3575; 3672; 3685; 3824; 3842; 3846; 3847; 3848; 3897;.3960; 3993; 4172; 4182; 4192; 4257; 4419; 4425; 4428; 4429; 4468; 4507; 4553; 4660; 4722; 4872; 4961; 5019; 5027; 5107; 5176; 5183; 5184; 5268; 5272; 5334; 5343; 5391; 5417 (voir tableau 1). Cette combinaison permet de classifier correctement 95,6 % des échantillons.

### 2.42. Identification d'un panel prédictif de 150 marqueurs (PANEL 40)

Les inventeurs ont mis en évidence une combinaison de 150 marqueurs, liée à un algorithme de type SVM, basée sur les séquences SEQ ID Nos : 26; 58; 75; 103; 105; 120; 166; 171; 172; 278; 325; 354; 397; 398; 419; 421; 482; 518; 527; 539; 570; 571; 591; 597; 598; 619; 629; 658; 662; 689; 721; 872; 1033; 1159; 1162; 1174; 1218; 1229; 1237; 1238; 1240; 1258; 1259; 1270; 1307; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2249; 2251; 2263; 2269; 2307; 2310; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3160; 3185; 3186; 3190; 3193; 3200; 3304; 3376; 3430; 3508; 3527; 3531; 3565; 3567; 3575; 3672; 3685; 3824; 3842; 3846; 3847; 3848; 3897; 3960; 3993; 4172; 4182; 4192; 4257; 4419; 4425; 4428; 4429; 4468; 4507; 4553; 4660; 4722; 4872; 4961; 5019; 5027; 5107; 5176; 5183; 5184; 5268; 5272; 5334; 5343; 5391; 5417 (voir tableau 1). Cette combinaison permet de classifier correctement 95,6 % des échantillons.

### 2.43. Identification d'un panel prédictif de 210 marqueurs (PANEL 41)

Les inventeurs ont mis en évidence une combinaison de 210 marqueurs, liée à un algorithme de type SVM, basée sur les séquences SEQ ID Nos : 26; 52; 58; 75; 103; 105; 114; 120; 163; 166; 171; 172; 277; 278; 300; 325; 354; 397; 398; 407; 419; 421; 424; 482; 512; 518; 527; 539; 569; 570; 571; 591; 596; 597; 598; 614; 619; 629; 658; 662; 689; 694; 719; 721; 872; 949; 1033; 1159; 1162; 1166; 1169; 1174; 1214; 1218; 1229; 1237; 1238; 1240; 1242; 1258; 1259; 1270; 1307; 1308; 1346; 1348; 1432; 1440; 1494; 1495; 1522; 1523; 1551; 1588; 1632; 1654; 1668; 1674; 1724; 1744; 1786; 1841; 1853; 1859; 1951; 1986; 2001; 2007; 2009; 2010; 2110; 2115; 2144; 2185; 2249; 2250; 2251; 2253; 2260; 2263; 2269; 2281; 2307; 2310; 2320; 2349; 2350; 2421; 2462; 2519; 2594; 2604; 2619; 2620; 2621; 2622; 2813; 2835; 2838; 2869; 2873; 2887; 2888; 2889; 2896; 2900; 2979; 3005; 3053; 3088; 3106; 3157; 3160; 3185; 3186; 3190; 3193; 3200; 3213; 3218; 3266; 3278; 3304; 3340; 3376; 3430; 3506; 3508; 3525; 3527; 3531; 3565; 3567; 3575; 3625; 3626; 3672; 3685; 3782; 3814; 3824; 3828; 3842; 3846; 3847; 3848; 3897; 3960; 3993; 4172; 4182; 4192; 4233; 4257; 4281; 4381; 4419; 4425; 4428; 4429; 4468; 4480; 4489; 4507; 4553; 4574; 4587; 4622; 4660; 4722; 4736; 4872; 4900; 4961; 5019; 5027; 5107; 5141; 5176; 5183; 5184; 5268; 5272; 5331; 5334; 5343; 5375; 5391; 5417; 5529 (voir tableau 1).

Cette combinaison permet de classifier correctement 95,6 % des échantillons.

### 2.44. Identification d'un panel prédictif de 170 marqueurs (PANEL 42)

Les inventeurs ont mis en évidence une combinaison de 170 marqueurs, liée à un algorithme de type SVM, basée sur les séquences SEQ ID Nos : 26; 58; 75; 103; 105; 120; 166; 171; 172; 278; 325; 354; 397; 398; 419; 421; 424; 482; 512; 518; 527; 539; 570; 571; 591; 597; 598; 619; 629; 658; 662; 689; 694; 721; 872; 1033; 1159; 1162; 1166; 1174; 1218; 1229; 1237; 1238; 1240; 1242; 1258; 1259; 1270; 1307; 1308; 1346; 1432; 1440; 1495; 1522; 1523; 1588; 1632; 1654; 1668; 1674; 1744; 1786; 1841; 1853; 1859; 1951; 1986; 2001; 2007; 2009; 2010; 2110; 2115; 2144; 2185; 2249; 2251; 2253; 2263; 2269; 2307; 2310; 2320; 2349; 2350; 2421; 2462; 2519; 2594; 2619; 2620; 2621; 2622; 2813; 2835; 2838; 2873; 2887; 2888; 2889; 2896; 2900; 2979; 3005; 3053; 3088; 3160; 3185; 3186; 3190; 3193; 3200; 3213; 3266; 3304; 3376; 3430; 3508; 3525; 3527; 3531; 3565; 3567; 3575; 3625; 3672; 3685; 3824; 3828; 3842; 3846; 3847; 3848; 3897; 3960; 3993; 4172; 4182; 4192; 4233; 4257; 4281; 4381; 4419; 4425; 4428; 4429; 4468; 4507; 4553; 4660; 4722; 4872; 4900; 4961; 5019; 5027; 5107; 5141; 5176; 5183; 5184; 5268; 5272; 5334; 5343; 5391; 5417 (voir tableau 1).

Cette combinaison permet de classifier correctement 95,4 % des échantillons.

### 2.45. Identification d'un panel prédictif de 60 marqueurs (PANEL 43)

Les inventeurs ont mis en évidence une combinaison de 60 marqueurs, liée à un algorithme de type SVM, basée sur les séquences SEQ ID Nos : 26; 58; 105; 120; 166; 325; 354; 397; 398; 570; 571; 598; 619; 629; 721; 1033; 1218; 1229; 1237; 1238; 1258; 1259; 1270; 1346; 1432; 1440; 1495; 1588; 1632; 1668; 1853; 1859; 2009; 2269; 2594; 2620; 2622; 2838; 2887; 3200; 3304; 3430; 3508; 3527; 3575; 3960; 3993; 4192; 4419; 4428; 4429; 4660; 4722; 4961; 5107; 5176; 5183; 5184; 5391; 5417 (voir tableau 1).

Cette combinaison permet de classifier correctement 95,2 % des échantillons.

### 2.46. Identification d'un panel prédictif de 190 marqueurs (PANEL 44)

Les inventeurs ont mis en évidence une combinaison de 190 marqueurs, liée à un algorithme de type SVM, basée sur les séquences SEQ ID Nos : 1; 6; 28; 73; 321; 323; 343; 350; 352; 397; 417; 418; 513; 538; 542; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2132; 2133; 2143; 2144; 2209; 2284; 2305; 2365; 2372; 2375; 2378; 2380; 2382; 2390; 2399; 2406; 2411; 2458; 2459; 2462; 2469; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3574; 3596; 3600; 3673; 3690; 3767; 3782; 3807; 3810; 3811; 3822; 3857; 3862; 3904; 3920; 3936; 3940; 3958; 4019; 4076; 4093; 4094; 4096; 4130; 4295; 4297; 4345; 4350; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5017; 5067; 5080; 5144; 5198; 5221; 5238; 5380; 5383; 5450; 5529; 5532; 5555 (voir tableau 1). Cette combinaison permet de classifier correctement 92,8 % des échantillons.

### 2.47. Identification d'un panel prédictif de 170 marqueurs (PANEL 45)

Les inventeurs ont mis en évidence une combinaison de 170 marqueurs, liée à un algorithme de type SVM, basée sur les séquences SEQ ID Nos : 1; 28; 73; 321; 323; 350; 397; 417; 418; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1950; 1951; 1952; 1953; 1984; 1994; 1995; 2023; 2038; 2117; 2126; 2127; 2128; 2129; 2130; 2133; 2144; 2284; 2305; 2365; 2372; 2375; 2378; 2380; 2382; 2399; 2406; 2411; 2458; 2459; 2462; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3596; 3600; 3690; 3782; 3807; 3810; 3811; 3822; 3857; 3862; 3904; 3920; 3940; 3958; 4019; 4076; 4093; 4094; 4096; 4130; 4295; 4297; 4345; 4351; 4428; 4434; 4442; 4468; 4493; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5067; 5080; 5144; 5198; 5221; 5238; 5380; 5383; 5450; 5532; 5555 (voir tableau 1).

Cette combinaison permet de classifier correctement 92,5 % des échantillons.

### 2.48. Identification d'un panel prédictif de 180 marqueurs (PANEL 46)

Les inventeurs ont mis en évidence une combinaison de 180 marqueurs, liée à un algorithme de type SVM, basée sur les séquences SEQ ID Nos : 1; 28; 73; 321; 323; 350; 352; 397; 417; 418; 513; 538; 612; 720; 788; 852; 990; 991; 1152; 1154; 1169; 1212; 1237; 1239; 1242; 1326; 1489; 1490; 1641; 1683; 1694; 1711; 1723; 1731; 1741; 1744; 1747; 1749; 1789; 1800; 1831; 1833; 1834; 1837; 1859; 1950; 1951; 1952; 1953; 1984; 1994; 1995; 2023; 2038; 2117; 2126; 2127; 2128; 2129; 2130; 2133; 2143; 2144; 2284; 2305; 2365; 2372; 2375; 2378; 2380; 2382; 2390; 2399; 2406; 2411; 2458; 2459; 2462; 2528; 2529; 2532; 2533; 2534; 2538; 2542; 2543; 2556; 2563; 2564; 2652; 2679; 2703; 2708; 2709; 2725; 2727; 2732; 2740; 2887; 2888; 2900; 2941; 2942; 2943; 2944; 3043; 3057; 3092; 3286; 3384; 3387; 3574; 3596; 3600; 3673; 3690; 3782; 3807; 3810; 3811; 3822; 3857; 3862; 3904; 3920; 3936; 3940; 3958; 4019; 4076; 4093; 4094; 4096; 4130; 4295; 4297; 4345; 4351; 4428; 4434; 4442; 4468; 4493; 4497; 4510; 4515; 4546; 4596; 4603; 4706; 4736; 4744; 4804; 4860; 4885; 4888; 4889; 4890; 4891; 4892; 4897; 4898; 4899; 4918; 4955; 4975; 4976; 4978; 4982; 5067; 5080; 5144; 5198; 5221; 5238; 5380; 5383; 5450; 5532; 5555 (voir tableau 1).

Cette combinaison permet de classifier correctement 92,5 % des échantillons.

### 2.49. Identification d'un panel prédictif de 35 marqueurs (PANEL 55)

Les inventeurs ont mis en évidence une combinaison de 35 marqueurs, liée à un algorithme de type LDA, basée sur les séquences SEQ ID Nos : 60; 1154; 1169; 1242; 1641; 1699; 1744; 1834; 1837; 2023; 2078; 2126; 2127; 2128; 2130; 2142; 2145; 2328; 2375; 2411; 2458; 2529; 2533; 2543; 2725; 2727; 2943; 2944; 3044; 3057; 3822; 4351; 4523; 5233; 5238 (voir tableau 1).

Les performances obtenues sont : une sensiblité de 100% et une spécificité de 80%.

### 2.50. Identification d'un panel prédictif de 35 marqueurs (PANEL 56)

Les inventeurs ont mis en évidence une combinaison de 35 marqueurs, liée à un algorithme de type LDA, basée sur les séquences SEQ ID Nos : 60; 1152; 1154; 1169; 1242; 1744; 1834; 2023; 2038; 2078; 2126; 2127; 2128; 2142; 2145; 2300; 2328; 2380; 2411; 2458; 2528; 2529; 2533; 2543; 2725; 2727; 2943; 2944; 3044; 3057; 3904; 4351; 4975; 5233; 5238 (voir tableau 1).

Les performances obtenues sont : une sensiblité de 100% et une spécificité de 80%.

### 2.51. Identification d'un panel prédictif de 16 marqueurs (PANEL 57)

Les inventeurs ont mis en évidence une combinaison de 16 marqueurs, liée à un algorithme de type LDA, basée sur les séquences SEQ ID Nos : 60; 1169; 1744; 1834; 2078; 2127; 2145; 2529; 2533; 2543; 2725; 2727; 2943; 2944; 3057; 5238 (voir tableau 1).

Les performances obtenues sont : une sensiblité de 100% et une spécificité de 80%.

### 2.52. Identification d'un panel prédictif de 13 marqueurs (PANEL 58)

Les inventeurs ont mis en évidence une combinaison de 13 marqueurs, liée à un algorithme de type LDA, basée sur les séquences SEQ ID Nos : 1169; 1834; 2038; 2078; 2127; 2145; 2533; 2543; 2725; 2727; 2943; 2944; 5238 (voir tableau 1).

Les performances obtenues sont : une sensiblité de 100% et une spécificité de 80%.

### 2.53. Identification d'un panel prédictif de 28 marqueurs (PANEL 59)

Les inventeurs ont mis en évidence une combinaison de 28 marqueurs, liée à un algorithme de type LDA, basée sur les séquences SEQ ID Nos : 26; 58; 527; 529; 629; 1169; 1259; 1270; 1588; 1632; 1668; 1841; 1854; 1912; 2622; 2623; 3187; 3527; 3625; 3672; 3994; 4428; 4429; 4961; 4988; 5087; 5107; 5113 (voir tableau 1).

Les performances obtenues sont : une sensiblité de 100% et une spécificité de 85%.

### 2.54. Identification d'un panel prédictif de 14 marqueurs (PANEL 60)

Les inventeurs ont mis en évidence une combinaison de 14 marqueurs, liée à un algorithme de type LDA, basée sur les séquences SEQ ID Nos : 58; 529; 1169; 1259; 1270; 1588; 1632; 1668; 1841; 1854; 3672; 4429; 5087; 5107 (voir tableau 1).

Les performances obtenues sont : une sensiblité de 100% et une spécificité de 80%.

Ces dernières signatures confirment que l'analyse de l'expression de 13, 14 ou 16 marqueurs peut être un bon outil pour discriminer les patients atteints de maladie d'Alzheimer. L'utilisation de panel de gènes restreint peut être particulièrement adaptée pour obtenir un outil de diagnostique et de pronostique. En effet, l'analyse de l'expression d'une dizaine de marqueurs ne nécessite pas la fabrication de puce à ADN de haute densité, et peut être mise en oeuvre directement par d'autres techniques de détection d'acides nucléiques, telles la PCR, ou par une puce de basse densité, ce qui peut présenter un atout économique important et une mise en oeuvre simplifiée.

### Exemple 3. Mise en évidence de profils d'expression pour le diagnostic de la maladie d'Alzheimer à partir d'échantillon sanguins par mesure relative de paires de sondes

Une autre approche possible de recherche de biomarqueurs d'expression consiste à prendre en compte, non pas l'expression absolue d'un ou de plusieurs probe sets mais l'expression relative de différents probe sets intra- ou inter-gènes. Un gène produit plusieurs transcrits qui participent à la complexité de son expression, de son transcriptome. Les probe sets présents sur le microarray GWSA couvrent le transcriptome de chaque gène à des positions stratégiques (spécifiques de variants d'épissage et de la structure exon-intron du gène). Tout gene pourrait ainsi donner lieu à un probe set qui pourrait être up-régulé dans un groupe de patients AD, alors qu'un second probe set dérivé du même gène pourrait lui être down-régulé. L'expression relative de ces deux probe sets pourrait représenter alors un pouvoir diagnostic statistiquement plus significatif que l'expression individuelle de chaque probe set. Une analyse simplifiée de l'expression relative consiste à identifier les paires de probe sets pour lesquels l'expression relative est inversée entre le groupe de patients AD et le groupe contrôle. Ainsi, un score compris entre +1 et -1 peut être associé à toute paire de probe sets (x,y) basé sur la fréquence de patients AD et de contrôles pour lesquels x > y. Par exemple, si x > y chez 100% des patients AD et 0% des individus contrôles, alors le score est de 1 - 0 = 1. Si x > y chez 80% des patients AD et 20% des patients contrôlés, alors le score sera de 0,8 - 0,2 = 0,6.

Cette analyse a été effectuée à partir de tous les genes presents sur la puce GWSA, en comparant tous les probe sets au sein d'un même gène (filtrés au dessus du seuil d'expression de 3,8 (log2)), ceci à partir du jeu de 150 échantillons (80 AD et 70 contrôles). Cette analyse a été effectuée dans un premier temps à partir des valeurs d'expression normalisées. Plus de 90% des paires ont un score dont la valeur absolue est inférieure à 0,2, indiquant une répartition équilibrée et équivalente de paires (x,y) pour lesquelles x > ydans le groupe AD et le groupe Contrôle. Le score obtenu le plus important est de 0,75 et concerne la paire SEQ ID NO : 2677 et 2678, issue du gène ATPase, Na+/K+ transporting, alpha 1 (ATP1A1, Gene ID : 476). 2007 paires de probe sets présentent un score dont la valeur absolue est supérieure ou égal à 0.5. Il s'agit du groupe TSP N (scores en colomne I du tableau 2). De tels scores sont significatifs comme le démontre l'analyse suivante éffectuée sur les trois gènes : Presenilin 1 (PSEN1), CD45 (PTPRC) et Sortilin-1 (SORL1). Les meilleurs scores obtenus pour ces trois gènes sont respectivement : 0,55 ; 0,57 et 0,69. Cette analyse a été répétée sur 1000 cas en créant pour chaque cas deux groupes au hasard de 80 et 70 individus parmi les 150 individus. Les meilleurs scores obtenus « au hasard » sont alors de 0,40 ; 0,39 et 0,45, tous trois inférieurs aux scores de 0,55 ; 0,57 et 0,69 obtenus à partir du groupe AD et contrôle. Ces résultats démontrent que les meilleurs scores associés aux paires de probe sets de ces trois gènes sont statistiquement significatifs des deux groupes comprés. La définition d'un seuil de score à 0,5 est donc significative.

Ce groupe TSP N est représenté par les paires de probe sets suivantes (une paire étant représentée selon x _ y) :
SEQ ID NO: 1 _ 3; 1 _ 5; 1 _ 10; 1 _ 11; 1 _ 12; 1 _ 13; 1 _ 14; 2 _ 3; 3 _ 4; 4 _ 10; 7 _ 9; 8 _ 9; 28 _ 29; 29 _ 31; 33 _ 34; 33 _ 35; 33 _ 36; 33 _ 37; 44 _ 49; 53 _ 54; 56 _ 57; 68 _ 72; 69 _ 71; 69 _ 73; 69 _ 76; 69 _ 77; 69 _ 78; 69 _ 81; 69 _ 82; 69 _ 83; 70 _ 83; 74 _ 79; 74 _ 85; 92 _ 93; 95 _ 96; 95 _ 97; 106 _ 107; 111 _ 112; 122 _ 124; 123 _ 124; 129 _ 130; 133 _ 134; 135 _ 139; 135 _ 142; 135 _ 144; 135 _ 145; 135 _ 146; 136 _ 140; 136 _ 141; 136 _ 148; 136 _ 149; 151 _ 152; 154 _ 155; 168 _ 169; 168 _ 170; 176 _ 177; 182 _ 183; 188 _ 189; 192 _ 193; 194 _ 201; 194 _ 207; 195 _ 199; 195 _ 203; 195 _ 210; 196 _ 212; 197 _ 210; 198 _ 202; 200 _ 205; 202 _ 204; 204 _ 211; 205 _ 208; 205 _ 209; 206 _ 212; 217_ 222; 218_ 220; 219 _ 220; 220 _ 221; 223 _ 224; 224 _ 225; 224 _ 227; 224 _ 228; 224 _ 229; 224 _ 230; 224 _ 232; 224 _ 233; 224 _ 235; 224 _ 236; 224 _ 237; 224 _ 238; 224 _ 239; 224 _ 240; 224 _ 242; 224 _ 243; 224 _ 244; 224 _ 246; 224 _ 247; 224 _ 248; 224 _ 249; 224 _ 250; 224 _ 251; 224 _ 252; 224 _ 253; 224 _ 254; 224 _ 255; 224 _ 257; 224 _ 258; 224 _ 259; 231 _ 224; 264 _ 268; 265 _ 272; 266 _ 269; 267 _ 269; 269 _ 273; 270 _ 272; 270 _ 274; 270 _ 275; 270 _ 276; 280 _ 281; 282 _ 283; 286 _ 288; 287 _ 288; 289 _ 290; 291 _ 292; 296 _ 297; 298 _ 299; 302 _ 303; 303 _ 304; 307 _ 309; 314 _ 315; 316 _ 317; 318 _ 319; 320 _ 323; 326 _ 327; 328 _ 331; 329 _ 332; 330 _ 331; 331 _ 333; 331 _ 334; 331 _ 335; 331 _ 336; 344 _ 346; 358 _ 361; 363 _ 364; 367 _ 368; 367 _ 369; 373 _ 375; 380 _ 381; 385_ 386; 386 _ 391; 386 _ 392;387 _ 389; 388 _ 390; 393 _ 395; 411 _ 412; 414 _ 418; 415 _ 418; 429 _ 430; 433 _ 434; 434 _ 435; 434 _ 438; 436 _ 437; 439 _ 440; 463 _ 464; 477_ 478; 477 _ 479; 481 _ 483; 484 _ 485; 486 _ 487; 486 _ 488; 486_ 489; 486 _ 490; 486 _ 494; 486 _ 495; 486 _ 496; 486 _ 497; 486 _ 499; 491_ 493; 504 _ 505; 507 _ 508; 533 _ 534; 544 _ 548; 545 _ 547; 546 _ 552; 547 _ 550; 547 _ 553; 547 _ 554; 549 _ 551; 549 _ 555; 557 _ 558; 559 _ 561; 560 _ 566; 562 _ 564; 573 _ 574; 575 _ 578; 576 _ 578; 577 _ 578; 579 _ 580; 584 _ 585; 586 _ 587; 588 _ 589; 610 _ 611; 621 _ 629; 622 _ 629; 623 _ 629; 624 _ 629; 625 _ 629; 626 _ 629; 627 _ 629; 628 _ 629; 629 _ 630; 629 _ 632; 639 _ 640; 642 _ 643; 644 _ 645; 649 _ 651; 652 _ 653; 670 _ 672; 671 _ 672; 674 _ 675; 680 _ 681; 682 _ 683; 684 _ 685; 700 _ 702; 701 _ 702; 703 _ 704; 714 _ 715; 717 _ 718; 727 _ 728; 733 _ 735; 734 _ 736; 740 _ 743; 741 _ 743; 742 _ 746; 742 _ 748; 743 _ 745; 750 _ 753; 755 _ 756; 761 _ 763; 761 _ 764; 765 _ 766; 765 _ 767; 771 _ 772; 771 _ 773; 772 _ 774; 773 _ 774; 779 _ 780; 791 _ 795; 791 _ 799; 791 _ 801; 791 _ 803; 791 _ 814; 792 _ 811; 793 _ 807; 793 _ 811; 794 _ 811; 796 _ 824; 797 _ 809; 797 _ 811; 804 _ 811; 805 _ 811; 806 _ 825; 808 _ 816; 808 _ 823; 809 _ 813; 810 _ 816; 810 _ 823; 811 _ 813; 811 _ 815; 811 _ 818; 811 _ 819; 811 _ 820; 811 _ 821; 811 _ 822; 812 _ 821; 827 _ 829; 827 _ 831; 828 _ 830; 832 _ 835; 833 _ 837;834 _ 836; 835 _ 838; 835 _ 841; 836 _ 841; 838 _ 840; 847 _ 849; 848 _ 851; 849 _ 850; 852 _ 854; 852 _ 855; 856 _ 863; 856 _ 866; 858 _ 860; 862 _ 865; 869 _ 870; 873 _ 882; 874 _ 878; 874 _ 882; 875 _ 882; 876 _ 882; 877 _ 882; 879 _ 882; 882 _ 885; 883 _ 884; 895 _ 899; 895 _ 900; 897 _ 898; 898 _ 904; 899 _ 901; 907 _ 909; 908 _ 911;919 _ 920; 929 _ 930; 929 _ 931; 937 _ 938; 941 _ 942;943 _ 945; 943 _ 946; 943 _ 947; 944 _ 947; 955 _ 959; 960 _ 961; 961 _ 962; 961 _ 963; 961_ 964; 961 _ 965; 961 _ 966; 961 _ 968; 961 _ 969; 970 _ 971; 970 _ 972; 974 _ 975; 993 _ 1011; 994 _ 996; 994 _ 1013; 995 _ 997; 995 _ 1001; 995 _ 1002; 995 _ 1003; 996 _ 999; 998 _ 1004; 998 _ 1009; 998 _ 1012; 1000 _ 1009; 1005 _ 1011; 1006 _ 1011; 1007 _ 1011; 1008 _ 1011; 1009 _ 1010; 1018 _ 1021; 1022 _ 1025; 1023 _ 1024; 1024 _ 1026; 1027 _ 1028; 1034 _ 1037; 1035 _ 1036; 1038 _ 1039; 1047 _ 1048; 1053 _ 1055; 1056 _ 1059; 1056 _ 1063; 1057 _ 1060; 1058 _ 1062; 1059 _ 1061; 1064 _ 1072; 1065 _ 1067; 1066 _1067; 1067 _ 1068; 1067 _ 1069; 1067 _ 1071; 1068 _ 1072; 1073 _ 1074; 1081 _ 1083; 1081 _ 1084; 1081 _ 1085; 1082 _ 1086; 1087 _ 1088; 1088 _ 1089; 1088 _ 1090; 1088 _ 1091; 1088 _ 1092; 1093 _ 1094; 1097 _ 1099; 1098 _ 1101; 1098_1105; 1102 _ 1117; 1103 _ 1110; 1104_1114; 1107_1111; 1110 _ 1118; 1110 _ 1119; 1123 _ 1133; 1124 _ 1133; 1125 _ 1126; 1125 _ 1127; 1125 _ 1128; 1125 _ 1129; 1125 _ 1130; 1125 _ 1131; 1125 _ 1132; 1134 _ 1139; 1135 _ 1138; 1136 _ 1137; 1141 _1144;1174_ 1176;1180 _1183;1181_ 1182;1189 _1190;1189_ 1191;1193_ 1194; 1193 _ 1195; 1196 _ 1197; 1196 _ 1198; 1201 _ 1202; 1208 _ 1209; 1208 _ 1210; 1208 _ 1211; 1212 - 1213; 1219 _ 1239; 1221 _ 1237; 1222 _ 1239; 1223 _ 1242; 1224 _ 1239; 1226 _ 1244; 1226 _ 1261; 1229 _ 1231; 1229 _ 1233; 1229 _ 1247; 1231 _ 1237; 1232 _ 1239; 1233 _ 1260; 1234 _ 1242; 1234 _ 1258; 1234 _ 1259; 1236 _ 1242; 1237 _ 1243; 1237 _ 1245; 1237 _ 1249; 1237 _ 1251; 1237 _ 1263; 1237 _ 1265; 1237 _ 1267; 1238 _ 1245; 1238 _ 1249; 1238 _ 1251; 1239 _ 1244; 1239 _ 1262; 1239 _ 1266; 1239 _ 1269; 1241 _ 1256; 1242 _ 1248; 1242 _ 1250; 1242 _ 1252; 1242 _ 1255; 1245 _ 1254; 1247 _ 1253; 1247 _ 1254; 1248 _ 1258; 1248 _ 1259; 1249 _ 1254; 1271 _ 1273; 1272 _ 1274; 1275 _ 1276; 1279 _ 1280; 1281 _ 1285; 1282 _ 1284; 1283 _ 1284; 1289 _ 1292; 1290 _1291; 1291 _ 1293; 1295 _ 1296; 1295 _ 1297; 1299 _ 1306; 1300 _ 1303; 1301 _ 1302; 1310 _ 1311; 1310 _ 1319; 1310 _ 1320; 1310 _ 1325; 1312 _ 1321; 1312 _ 1324; 1313 _ 1326; 1314 _ 1324; 1315 _ 1328; 1317 _ 1326; 1318 _ 1319; 1323 _ 1329; 1323 _ 1330; 1326 _ 1331; 1334 _ 1336; 1335 _ 1336; 1339 _ 1340; 1341 _ 1342; 1344 _ 1345; 1350 _ 1352; 1351 _ 1353; 1359 _ 1360; 1363 _ 1364; 1367 _ 1382; 1367 _ 1383; 1368 _ 1372; 1370 _ 1385; 1371 _ 1374; 1373 _ 1389; 1375 _ 1383; 1376 _ 1388; 1380 _ 1383; 1382 _ 1392; 1383 _ 1392; 1384 _ 1390; 1384 _ 1393; 1386 _ 1387; 1386 _ 1391; 1399 _ 1402; 1400 _ 1402; 1402 _ 1403; 1402 _ 1405; 1402 _ 1407; 1402 _ 1409; 1408 _ 1412; 1418 _ 1419; 1423 _ 1427; 1424 _ 1427; 1429 _ 1430; 1429 _ 1431; 1433 _ 1435; 1434 _ 1435; 1435 _ 1438; 1436 _ 1437; 1441 _ 1442; 1443 _ 1444; 1444 _ 1446; 1444 _ 1447; 1448 _ 1450; 1448 _ 1452; 1448 _ 1453; 1448 _ 1456; 1448 _ 1457; 1448 _ 1458; 1448 _ 1459; 1448 _ 1460; 1448 _ 1461; 1448 _ 1462; 1448 _ 1463; 1448 _ 1465; 1451 _ 1471; 1453 _ 1470; 1454 _ 1467; 1466 _ 1469;1475 _1476;1476 _1481;1476 _ 1482; 1477 _ 1478; 1478 _ 1479; 1478 _ 1483; 1485 _ 1489; 1486 _ 1490; 1488 _ 1490; 1488 _ 1499; 1490 _ 1492;1490 _ 1497;1490 _ 1500;1501 _ 1502; 1501 _ 1503; 1501 _ 1504; 1507 _ 1508; 1511 _ 1514; 1511 _ 1519; 1536 _ 1539; 1537 _ 1539; 1538 _ 1539; 1539 _ 1540; 1539 _ 1542; 1539 _ 1544; 1541 _ 1543; 1543 _ 1545; 1546 _ 1548; 1546 _ 1550; 1547 _ 1549; 1553 _ 1554; 1553 _ 1555; 1556 _ 1558; 1557 _ 1558; 1564 _ 1565; 1567 _ 1572; 1568 _ 1569; 1570 _ 1571; 1572 _ 1573; 1572 _ 1574; 1586 _ 1587; 1593 _ 1595; 1594 _ 1596; 1602 _ 1601; 1603 _ 1605; 1604 _ 1606; 1607 _ 1608; 1610 _ 1615; 1611 _ 1614; 1612 _ 1615; 1613 _ 1614; 1620 _ 1625; 1625 _ 1626; 1628 _ 1631; 1637 _ 1638; 1641 _ 1642; 1641 _ 1644; 1641 _ 1645; 1641 _ 1646; 1655 _ 1657; 1658 _ 1659; 1658 _ 1660; 1658 _ 1661; 1658 _ 1662; 1658 _ 1663; 1658 _ 1664; 1658 _ 1665; 1658 _ 1666; 1676 _ 1677; 1678 _ 1679; 1680 _ 1681; 1682 _ 1685; 1683 _ 1684; 1683 _ 1686; 1687 _ 1688; 1694 _ 1698; 1703 _ 1704; 1704 _ 1707; 1706 _ 1708; 1711 _ 1712; 1715 _ 1716; 1726 _ 1727; 1727 _ 1728; 1732 _ 1738; 1733 _ 1745; 1735 _ 1751; 1736 _ 1745; 1738 _ 1745; 1739 _ 1745; 1745 _ 1746; 1745 _ 1750; 1749 _ 1752; 1754 _ 1755; 1757 _ 1758; 1760 _ 1761; 1768 _ 1769; 1777 _ 1781; 1778 _ 1781; 1781 _ 1783; 1795 _ 1797; 1796 _ 1797; 1803 _ 1804; 1817 _ 1818; 1817 _ 1819; 1820 _ 1821; 1835 _ 1837; 1842 _ 1843; 1848 _ 1850; 1848 _ 1852; 1864 _ 1866; 1865 _ 1866; 1866 _ 1867; 1866 _ 1868; 1866 _ 1869; 1866 _ 1871; 1866 _ 1872; 1866 _ 1873; 1866 _ 1874; 1866 _ 1875; 1866 _ 1877; 1866 _ 1878; 1866 _ 1879; 1866 _ 1880; 1866 _ 1881; 1866 _ 1882; 1866 _ 1883; 1866 _ 1884; 1866 _ 1885; 1866 _ 1886; 1866 _ 1887; 1866 _ 1888; 1866 _ 1889; 1866 _ 1891; 1866 _ 1892; 1866 _ 1893; 1866 _ 1894; 1866 _ 1895; 1866 _ 1896; 1866 _ 1897; 1866 _ 1898; 1866 _ 1899; 1866 _ 1900; 1866 _ 1901; 1866 _ 1902; 1866 _ 1903; 1866 _ 1904; 1866 _ 1905; 1866 _ 1906; 1866 _ 1907; 1866 _ 1908; 1866 _ 1909; 1870 _ 1866; 1921 _ 1922; 1921 _ 1923; 1921 _ 1924; 1922 _ 1927; 1922 _ 1928; 1922 _ 1929; 1922 _ 1930; 1925 _ 1931; 1925 ₋ 1932; 1936 _ 1937; 1939 _ 1941; 1939 _ 1943; 1939 _ 1944; 1939 _ 1945; 1949 _ 1954; 1957 _ 1958; 1962 _ 1963; 1965 _ 1967; 1974 _ 1975; 1988 _ 1992; 1991 _ 1993; 1997 _ 1998; 2004 _ 2006; 2013 _ 2016; 2014 _ 2016; 2015 _ 2016; 2018 _ 2019; 2026 _ 2027; 2028 _ 2029; 2029 _ 2031; 2029 _ 2034; 2030 _ 2033; 2035 _ 2036; 2043 _ 2044; 2046 _ 2048; 2051 _ 2058; 2051 _ 2059; 2052 _ 2057; 2053 _ 2059; 2055 _ 2059; 2056 _ 2059; 2065 _ 2067; 2065 _ 2079; 2066 _ 2069; 2067 _ 2076; 2068 _ 2074; 2070 _ 2078; 2071 _ 2078; 2071 _ 2082; 2072 _ 2073; 2072 _ 2081; 2073 _ 2075; 2075 _ 2081; 2076 _ 2080; 2077 _ 2083; 2077 _ 2084; 2089 _ 2090; 2101 ₋ 2102; 2102 _ 2105; 2103 _ 2105; 2126 _ 2131; 2138 _ 2146; 2149 _ 2152; 2150 _ 2155; 2151 _ 2154; 2151 _ 2156; 2162 _ 2163; 2169 _ 2170; 2169 _ 2171; 2169 _ 2173; 2169 _ 2174; 2178 _ 2179; 2178 _ 2181; 2183 _ 2184; 2189 _ 2191; 2191 _ 2193; 2191 _ 2194; 2191 _ 2198; 2192 _ 2197; 2192 _ 2199; 2194 _ 2196; 2196 _ 2198; 2205 _ 2206; 2208 _ 2213; 2209 _ 2210; 2211 _ 2212; 2219 _ 2226; 2220 _ 2223; 2222 _ 2226; 2223 _ 2231; 2224 _ 2226; 2225 _ 2226; 2226 _ 2227; 2226 _ 2229; 2226 _ 2230; 2226 _ 2232; 2226 _ 2233; 2226 _ 2234; 2236 _ 2237; 2236 _ 2238; 2239 _ 2240; 2244 _ 2245; 2248 _ 2262; 2251 _ 2262; 2253 _ 2262; 2259 _ 2261; 2262 _ 2265; 2273 _ 2276; 2274 _ 2276; 2275 _ 2276; 2276 _ 2277; 2282 _ 2283; 2285 _ 2297; 2286 _ 2287; 2286 _ 2292; 2286 _ 2297; 2288 _ 2299; 2289 _ 2299; 2291 _ 2299; 2293 _ 2299; 2294 _ 2298; 2295 _ 2296; 2302 _ 2303; 2334 _ 2338; 2338 _ 2339; 2342 _ 2343; 2351 _ 2355; 2351 _ 2357; 2353 _ 2356; 2363 _ 2364; 2366 _ 2385; 2370 _ 2386; 2375 _ 2388; 2403 _ 2404; 2422 _ 2424; 2424 _ 2427; 2424 _ 2428; 2425 _ 2427; 2426 _ 2428; 2429 _ 2430; 2432 _ 2433; 2432 _ 2434; 2448 _ 2449; 2452 _ 2453; 2455 _ 2458; 2458 _ 2466; 2459 _ 2463; 2462 _ 2463; 2464 _ 2467; 2464 _ 2468; 2472 _ 2473; 2477 _ 2478; 2477 _ 2479; 2480 _ 2481; 2482 _ 2483; 2484 _ 2485; 2487 _ 2488; 2491 _ 2492; 2495 _ 2500; 2496 _ 2505; 2496 _ 2507; 2497 _ 2499; 2497 _ 2504; 2497 _ 2505; 2497 _ 2506; 2497 _ 2508; 2497 _ 2510; 2497 _ 2512; 2497 _ 2516; 2498 _ 2504; 2498 _ 2505; 2498 _ 2509; 2498 _ 2510; 2498 _ 2511; 2498 _ 2513; 2498 _ 2514; 2500 _ 2508; 2501 _ 2511; 2502 _ 2509; 2502 _ 2511; 2502 _ 2513; 2503 _ 2506; 2503 _ 2508; 2522 _ 2523; 2528 _ 2535; 2529 _ 2537; 2532 _ 2535; 2540 _ 2563; 2541 _ 2570; 2541 _ 2572; 2542 _ 2547; 2542 _ 2550; 2542 _ 2567; 2542 _ 2568; 2543 _ 2567; 2544 _ 2572; 2549 _ 2544; 2549 _ 2545; 2549 _ 2561; 2549 _ 2576; 2551 _ 2575; 2552 _ 2575; 2553 _ 2557; 2554 _ 2558; 2561 _ 2572; 2561 _ 2573; 2562 _ 2571; 2563 _ 2565; 2563 _ 2566; 2563 _ 2569; 2564 _ 2567; 2564 _ 2568; 2572 _ 2574; 2580 _ 2581; 2608 _ 2609; 2614 _ 2615; 2624 _ 2631; 2625 _ 2626; 2626 _ 2629; 2627 _ 2630; 2637 _ 2639; 2640 _ 2642; 2641 _ 2642; 2644 _ 2645; 2646 _ 2647; 2649 _ 2650; 2652 _ 2653; 2652 _ 2656; 2652 _ 2657; 2654 _ 2655; 2661 _ 2670; 2662 _ 2664; 2662 _ 2671; 2663 _ 2669; 2664 _ 2673; 2665 _ 2667; 2666 _ 2669; 2668 _ 2670; 2668 _ 2671; 2670 _ 2673; 2671 _ 2672; 2677 _ 2678; 2677 _ 2682; 2677 _ 2688; 2677 _ 2689; 2677 _ 2694; 2678 _ 2686; 2678 _ 2690; 2682 _ 2685; 2682 _ 2686; 2682 _ 2690; 2683 _ 2692; 2686 _ 2688; 2686 _ 2689; 2687 _ 2688; 2688 _ 2690; 2689 _ 2690; 2693 _ 2694; 2704 _ 2705; 2706 _ 2708; 2707 _ 2711; 2708 _ 2712; 2713 _ 2714; 2716 _ 2718; 2716 _ 2726; 2718 _ 2722; 2719 _ 2727; 2720 _ 2723; 2722 ₋ 2723; 2730 _ 2745; 2731 _ 2732; 2732 _ 2733; 2732 _ 2734; 2732 _ 2736; 2732 _ 2737; 2732 _ 2738; 2734 _ 2744; 2746 _ 2748; 2748 _ 2750; 2748 _ 2757; 2748 _ 2758; 2748 _ 2759; 2749 _ 2751; 2751 _ 2756; 2752 _ 2755; 2753 _ 2754; 2760 _ 2761; 2767 _ 2768;2773 _ 2774; 2777 _ 2779; 2778 _ 2779; 2778 _ 2780; 2778 _ 2781; 2784 _ 2786; 2785 _ 2786; 2792 _ 2794; 2797 _ 2810; 2798 _ 2799; 2800 _ 2807; 2800 _ 2808; 2801 _ 2810; 2802 _ 2806; 2803 _ 2805; 2812 _ 2814; 2812 _ 2815; 2819 _ 2827; 2820 _ 2822; 2821 _ 2822; 2822 _ 2829; 2826 _ 2832; 2830 _ 2832; 2833 _ 2834; 2839 _ 2840; 2840 _ 2842; 2845 _ 2847; 2848 _ 2849; 2854 _ 2857;2854 _ 2864; 2854 _ 2867; 2855 _ 2858; 2855 _ 2859; 2855 _ 2868; 2856 _ 2861; 2860 _ 2861; 2861 _ 2865; 2861 _ 2866; 2875 _ 2876; 2875 _ 2877; 2875 _ 2881; 2875 _ 2884; 2878 _ 2880; 2878 _ 2883; 2878 _ 2885; 2880 _ 2882; 2911 _ 2912; 2918 _ 2923; 2926 _ 2927; 2928 _ 2929; 2948 _ 2949; 2952 _ 2953; 2952 _ 2954; 2955 _ 2959; 2956 _ 2961; 2962 _ 2965; 2963 _ 2964; 2967 _ 2973; 2968 _ 2971; 2969 _ 2972; 2970 _ 2972; 2975 _ 2977; 2980 _ 2981; 2983 _ 2984; 2986 _ 2987; 2988 _ 2989; 2988 _ 2990; 2988 _ 2992; 3001 _ 3003; 3001 _ 3004; 3008 _ 3009; 3010 _ 3011; 3011 _ 3013; 3012 _ 3013; 3015 _ 3023; 3016 _ 3027; 3017 _ 3018; 3017 _ 3023; 3017 _ 3025; 3017 _ 3026; 3017 _ 3029; 3018 _ 3030; 3018 _ 3032; 3020 _ 3023; 3023 _ 3022; 3023 _ 3030; 3023 _ 3031; 3023 _ 3032; 3027 _ 3034; 3047 _ 3049; 3051 _ 3052; 3054 _ 3056; 3057 _ 3058; 3062 _ 3063; 3064 _ 3065; 3071 _ 3073; 3072 _ 3073; 3077 _ 3078; 3077 _ 3084; 3078 _ 3080; 3078 _ 3082; 3079 _ 3084; 3090 _ 3092; 3091 _ 3092; 3092 _ 3093; 3092 _ 3094; 3092 _ 3095; 3092 _ 3099; 3100 _ 3101; 3111 _ 3112; 3111 _ 3114; 3111 _ 3115; 3116 _ 3117; 3119 _ 3137; 3120 _ 3122; 3121 _ 3123; 3121 _ 3144; 3122 _ 3125; 3122 _ 3131; 3122 _ 3133; 3122 _ 3138; 3122 _ 3139; 3122 _ 3146; 3122 _ 3147; 3123 _ 3135; 3123 _ 3140; 3126 _ 3128; 3126 _ 3130; 3127 _ 3144; 3129 _ 3145; 3132 _ 3149; 3136 _ 3144; 3140 _ 3144; 3141 _ 3146; 3141 _ 3147; 3151 _ 3152; 3154 _ 3155; 3162 _ 3163; 3168 _ 3169; 3168 _ 3170; 3176 _ 3181; 3177 _ 3182; 3178 _ 3180; 3180 _ 3183; 3197 _ 3198; 3198 _ 3199; 3201 _ 3202; 3203 _ 3204; 3227 _ 3230; 3228 _ 3231; 3236 _ 3238; 3236 _ 3239; 3236 _ 3240; 3237 _ 3238; 3242 _ 3248; 3243 _ 3244; 3243 _ 3245; 3243 _ 3246; 3243 _ 3247; 3247 _ 3249; 3251 _ 3254; 3251 _ 3256; 3258 _ 3259; 3261 _ 3263; 3262 _ 3265; 3269 _ 3271; 3274 _ 3275; 3283 _ 3284; 3287 _ 3293; 3288 _ 3289;3289 _ 3292; 3290 _ 3293; 3290 _ 3294; 3299 _ 3300; 3316 _ 3318; 3317 _ 3321; 3318 _ 3319; 3328 _ 3329; 3331 _ 3332; 3333 _ 3334; 3336 _ 3337; 3339 _ 3344; 3343 _ 3348; 3351 _ 3352; 3351 _ 3353; 3354 _ 3357; 3357 _ 3364; 3359 _ 3363; 3367 _ 3368; 3368 _ 3369; 3370 _ 3371; 3382 _ 3383; 3385 _ 3396; 3387 _ 3395; 3387 _ 3396; 3388 _ 3398; 3388 _ 3399; 3388 _ 3401; 3390 _ 3391; 3390 _ 3392; 3390 _ 3400; 3391 _ 3397; 3393 _ 3396; 3394 _ 3404; 3395 _ 3404; 3395 _ 3405; 3396 _ 3403; 3396 _ 3404; 3396 _ 3405; 3397 _ 3398; 3397 _ 3399; 3406 _ 3407; 3407 _ 3408; 3414 _ 3415; 3415 _ 3416; 3418 _ 3419; 3421 _ 3422; 3423 _ 3424; 3431 _ 3436; 3432 _ 3437; 3433 _ 3441; 3434 _ 3439; 3435 _ 3436; 3438 _ 3440; 3438 _ 3441; 3444 _ 3463; 3444 _ 3471; 3445 _ 3446; 3447 _ 3454; 3447 _.3460; 3449 _ 3473; 3451 _ 3464; 3451 _ 3467; 3451 _ 3468; 3453 _ 3467; 3454 _ 3464; 3454 _ 3470; 3455 _ 3465; 3456 _ 3465; 3456 _ 3467; 3475 _ 3477; 3475 _ 3479; 3476 _ 3477; 3477 _ 3482; 3478 _ 3481; 3483 _ 3484; 3485 _ 3486; 3487 _ 3488; 3492 _ 3493; 3493 _ 3495; 3494 _ 3496; 3498 _ 3499; 3501 _ 3503; 3501 _ 3504; 3510 _ 3511; 3511 _ 3513; 3512 _ 3513; 3513 _ 3514; 3528 _ 3529; 3543 _ 3544; 3544 _ 3545; 3546 _ 3549; 3547 _ 3548; 3554 _ 3555; 3556 _ 3557; 3560 _ 3561; 3570 _ 3571; 3584 _ 3585; 3589 _ 3591; 3591 _ 3592; 3591 _ 3593; 3591 _ 3594; 3603 _ 3604; 3605 _ 3606; 3610 _ 3611; 3610 _ 3612; 3610 _ 3614; 3610 _ 3616; 3610 _ 3618; 3610 _ 3619; 3610 _ 3621; 3611 _ 3622; 3612 _ 3613; 3613 _ 3618; 3613 _ 3620; 3617 _ 3618; 3630 _ 3635; 3631 _ 3633; 3631 _ 3643; 3632 _ 3634; 3632 _ 3637; 3632 _ 3641; 3638 _ 3642; 3644 _ 3645; 3647 _ 3648; 3651 _ 3653; 3652 _ 3653; 3653 _ 3655; 3653 _ 3656; 3653 _ 3658; 3653 _ 3659; 3653 _ 3662; 3653 _ 3664; 3653 _ 3668; 3675 _ 3676; 3677 _ 3680; 3678 _ 3695; 3679 _ 3690; 3681 _ 3682; 3682 _ 3687; 3682 _ 3693; 3682 _ 3694; 3682 _ 3697; 3683 _ 3689; 3683 _ 3691; 3683 _ 3696; 3684 _ 3693; 3700 _ 3701; 3703 _ 3704; 3710 _ 3720; 3711 _ 3719; 3712 _ 3715; 3714 _ 3718; 3722 _ 3725; 3723 _ 3724; 3724 _ 3726; 3724 _ 3727; 3724 _ 3728; 3724 _ 3729; 3730 _ 3731; 3735 _ 3736; 3737 _ 3741; 3738 _ 3741; 3739 _ 3741; 3740 _ 3741; 3740 _ 3742; 3744 _ 3750; 3746 _ 3747; 3754 _ 3756; 3755 _ 3757; 3760 _ 3763; 3764 _ 3765; 3766 _ 3767; 3767 _ 3768; 3767 _ 3770; 3780 _ 3781; 3784 _ 3785; 3787 _ 3788; 3791 _ 3792; 3794 _ 3795; 3797 _ 3798; 3800 _ 3803; 3801 _ 3802; 3817 _ 3818; 3831 _ 3832; 3833 _ 3834; 3834 _ 3836; 3834 _ 3839; 3834 _ 3840; 3835 _ 3838; 3837 _ 3839; 3850 _ 3857; 3852 _ 3859; 3854 _ 3861; 3857 _ 3858; 3857 _ 3860; 3865 _ 3866; 3868 _ 3869; 3873 _ 3875; 3874 _ 3877; 3874 _ 3878; 3874 _ 3879; 3874 _ 3880; 3874 _ 3881; 3874 _ 3882; 3874 _ 3883; 3885 _ 3886; 3885 _ 3888; 3891 _ 3892; 3893 _ 3894; 3899 _ 3900; 3904 _ 3905; 3908 _ 3909; 3909 _ 3910; 3909 _ 3911; 3914 _ 3919; 3916 _ 3917; 3920 _ 3923; 3924 _ 3928; 3925 _ 3926; 3925 _ 3927; 3945 _ 3947; 3946 _ 3948; 3946 _ 3949; 3958 _ 3959; 3970 _ 3971; 3980 _ 3981; 3981 _ 3982; 3981 _ 3983; 3985 _ 3986; 3988 _ 3989; 3997 _ 3998; 4000 _ 4002; 4016 _ 4017; 4017 _ 4020; 4018 _ 4021; 4022 _ 4027; 4023 _ 4037; 4024 _ 4027; 4025 _ 4029; 4025 _ 4030; 4027 _ 4034; 4027 _ 4035; 4027 _ 4036; 4027 _ 4038; 4042 _ 4044; 4043 _ 4044; 4044 _ 4047; 4046 _ 4048; 4049 _ 4050; 4050 _ 4052; 4055 _ 4060; 4056 _ 4057; 4056 _ 4059; 4058 _ 4059; 4061 _ 4062; 4066 _ 4091; 4067 _ 4076; 4067 _ 4096; 4068 _ 4093; 4069 _ 4096; 4071 _ 4076; 4072 _ 4096; 4073 _ 4093; 4074 _ 4096; 4075 _ 4093; 4075 _ 4096; 4076 _ 4080; 4076 _ 4081; 4076 _ 4082; 4076 _ 4083; 4076 _ 4103; 4076 _ 4106; 4077 _ 4093;4077 _ 4096; 4079 _ 4093; 4079 _ 4094; 4079 _ 4096; 4080 _ 4091; 4084 _ 4096; 4085 _ 4096; 4086 _ 4096; 4087 _ 4096; 4088 _ 4096; 4089 _ 4096; 4090 _ 4096; 4093 _ 4098; 4093 _ 4105; 4093 _ 4107; 4093 _ 4108; 4093 _ 4109; 4096 _ 4099; 4096 _ 4100; 4096 _ 4102; 4096 _ 4103; 4096 _ 4104; 4096 _ 4105; 4096 _ 4107; 4096 _ 4108; 4096 _ 4109; 4113 _ 4114; 4116 _ 4117; 4125 _ 4127; 4126 _ 4128; 4127 _ 4129; 4130 _ 4131; 4133 _ 4136; 4134 _ 4140; 4134 _ 4145; 4134 _ 4146; 4135 _ 4140; 4135 _ 4142; 4135 _ 4152; 4136 _ 4139; 4137 _ 4141; 4137 _ 4151; 4140 _ 4148; 4140 _ 4153; 4142 _ 4150; 4143 _ 4149; 4147 _ 4149; 4150 _ 4152; 4155 _ 4160; 4156 _ 4157; 4156 _ 4158; 4156 _ 4159; 4161 _ 4168; 4165 _ 4167; 4166 _ 4169; 4173 _ 4179; 4174 _ 4180; 4175 _ 4176; 4176 _ 4181; 4177 _ 4180; 4186 _ 4189; 4187 _ 4188; 4190 _ 4191; 4195 _ 4197; 4196 _ 4197; 4210 _ 4211; 4211 _ 4214; 4212 _ 4213; 4217 _ 4218; 4224 _ 4225; 4225 _ 4226; 4236 _ 4248; 4237 _ 4239; 4238 _ 4249; 4240 _ 4246; 4241 _ 4247; 4241 _ 4248; 4241 _ 4249; 4242 _ 4248; 4243 _ 4248; 4245 _ 4250; 4258 _ 4259; 4261 _ 4264; 4262 _ 4261; 4267 _ 4268; 4270 _ 4273; 4271 _ 4277; 4272 _ 4275; 4275 _ 4279; 4276 _ 4279; 4283 _ 4291; 4284 _ 4285; 4287 _ 4290; 4288 _ 4290; 4294 _ 4295; 4297 _ 4302; 4297 _ 4303; 4297 _ 4304; 4297 _ 4305; 4309 _ 4313; 4310 _ 4312; 4311 _ 4315; 4312 _ 4313; 4312 _ 4314; 4315 _ 4316; 4317 _ 4322; 4318 _ 4324; 4319 _ 4323; 4321 _ 4325; 4324 _ 4325; 4326 _ 4329; 4328 _ 4333; 4328 _ 4334; 4332 _ 4335; 4353 _ 4354; 4355 _ 4356; 4361 _ 4363; 4366 _ 4367; 4366 _ 4368; 4372 _ 4373; 4374 _ 4377; 4382 _ 4383; 4383 _ 4384; 4387 _ 4390; 4387 _ 4391; 4387 _ 4392; 4387 _ 4393; 4389 _ 4392; 4392 _ 4394; 4392 _ 4395; 4396 _ 4398; 4397 _ 4398; 4401 _ 4408; 4402 _ 4403; 4402 _ 4405; 4404 _ 4407; 4404 _ 4413; 4406 _ 4408; 4408 _ 4409; 4408 _ 4410; 4408 _ 4412; 4416 _ 4417; 4417 _ 4418; 4420 _ 4421; 4422 _ 4423; 4422 _ 4424; 4436 _ 4437; 4439 _ 4442; 4440 _ 4442; 4441 _ 4442; 4442 _ 4444; 4442 _ 4445; 4442 _ 4446; 4442 _ 4447; 4442 _ 4448; 4442 _ 4449; 4442 _ 4450; 4442 _ 4451; 4442 _ 4453; 4442 _ 4454; 4442 _ 4455; 4442 _ 4456; 4442 _ 4457; 4442 _ 4458; 4442 _ 4459; 4442 _ 4460; 4442 _ 4461; 4442 _ 4463; 4464 _ 4465;4464._ 4466; 4465 _ 4467; 4473 _ 4474; 4476 _ 4477; 4484 _ 4487; 4493 _ 4496; 4494 _ 4496; 4498 _ 4500; 4503 _ 4504; 4510 _ 4513; 4511 _ 4527; 4512 _ 4527; 4514 _ 4519; 4517 _ 4528; 4518 _ 4526; 4519 _ 4528; 4520 _ 4527; 4522 _ 4531; 4523 _ 4527; 4523 _ 4531; 4524 _ 4527; 4533 _ 4534; 4535 _ 4537; 4538 _ 4539; 4538 _ 4540; 4538 _ 4542; 4538 _ 4544; 4538 _ 4545; 4546 _ 4557; 4561 _ 4562; 4563 _ 4564; 4563 _ 4565; 4563 _ 4566; 4563 _ 4567; 4563 _ 4568; 4563 _ 4570; 4563 _ 4571; 4563 _ 4572; 4563 _ 4573; 4569 _ 4571; 4576 _ 4577; 4578 _ 4579; 4579 _ 4580; 4585 _ 4586; 4589 _ 4590; 4598 _ 4601; 4602 _ 4607; 4603 _ 4604; 4603 _ 4605; 4603 _ 4606; 4604 _ 4607; 4607 _ 4608; 4614 _ 4615; 4625 _ 4655; 4626 _ 4629; 4627 _ 4649; 4628 _ 4639; 4629 _ 4631; 4629 _ 4634; 4629 _ 4635; 4629 _ 4638; 4629 _ 4639; 4629 _ 4640; 4629 _ 4643; 4629 _ 4644; 4629 _ 4645; 4629 _ 4648; 4629 _ 4650; 4630 _ 4654; 4631 _ 4651; 4631 _ 4654; 4631 _ 4655; 4632 _ 4652; 4635 _ 4651; 4635 _ 4654; 4635 _ 4655; 4636 _ 4654; 4636 _ 4655; 4637 _ 4654; 4637 _ 4655; 4639 _ 4651; 4640 _ 4651; 4640 _ 4654; 4640 _ 4655; 4642 _ 4654; 4645 _ 4651; 4645 _ 4655; 4646 _ 4654; 4647 _ 4654; 4650 _ 4654; 4657 _ 4667; 4668 _ 4670; 4673 _ 4674; 4680 _ 4683; 4684 _ 4687; 4689 _ 4690; 4689 _ 4696; 4691 _ 4696; 4695 _ 4697; 4699 _ 4701; 4702 _ 4703; 4704 _ 4710; 4705 _ 4710; 4706 _ 4707; 4707 _ 4708; 4719 _ 4720; 4723 _ 4733; 4724 _ 4733; 4724 _ 4735; 4724 _ 4736; 4725 _ 4733; 4726 _ 4733; 4726 _ 4736; 4727 _ 4733; 4728 _ 4733; 4728 _ 4735; 4729 _ 4731; 4730 _ 4733; 4740 _ 4749; 4740 _ 4754; 4741 _ 4752; 4741 _ 4765; 4741 _ 4769; 4742 _ 4744; 4742 _ 4765; 4742 _ 4768; 4742 _ 4771; 4744 _ 4746; 4744 _ 4747; 4744 _ 4755; 4744 _ 4757; 4744 _ 4758; 4744 _ 4762; 4744 _ 4766; 4744 _ 4772; 4744 _ 4773; 4745 _ 4764; 4746 _ 4771; 4748 _ 4765; 4749 _ 4756; 4749 _ 4759; 4749 _ 4761; 4754 _ 4759; 4754 _ 4763; 4760 _ 4767; 4775 _ 4776; 4777 _ 4778; 4785 _ 4786; 4786 _ 4787; 4786 _ 4788; 4793 _ 4795; 4793 _ 4796; 4794 _ 4795; 4800 _ 4801; 4802 _ 4803; 4814 _ 4815; 4817 _ 4818; 4819 _ 4821; 4820 _ 4822; 4823 _ 4825; 4823 _ 4829; 4834 _ 4850; 4835 _ 4839; 4836 _ 4843; 4836 _ 4846; 4836 _ 4849; 4837 _ 4844; 4838 _ 4845; 4840 _ 4846; 4841 _ 4843; 4841 _ 4846; 4841 _ 4847; 4841 _ 4849; 4842 _ 4846; 4852 _ 4854; 4855 _ 4860; 4858 _ 4860; 4864 _ 4866; 4864 _ 4871; 4877 _ 4879; 4878 _ 4881; 4880 _ 4881; 4888 _ 4894; 4889 _ 4893; 4889 _ 4894; 4890 _ 4894; 4902 _ 4903; 4905 _ 4906; 4911 _ 4913; 4912 _ 4913; 4930 _ 4931; 4939 _ 4942; 4940 _ 4941; 4945 _ 4955; 4947 _ 4951; 4950 _ 4951; 4950 _ 4956; 4971 _ 4972; 4983 _ 4984; 4989 _ 4994; 4989 _ 5000; 4990 _ 4994; 4990 _ 5000; 4990 _ 5001; 4991 _ 4994; 4991 _ 5000; 4992 _ 5006; 4992 _ 5008; 4993 _ 5001; 4995 _ 5003; 4995 _ 5007; 4995 _ 5010; 4996 _ 5009; 4997 _ 5002; 5000 _ 5002; 5000 _ 5005; 5001 _ 5008; 5013 _ 5014; 5033 _ 5034; 5035 _ 5036; 5054 _ 5055; 5054 _ 5056; 5054 _ 5058; 5062 _ 5063; 5064 _ 5067; 5067 _ 5068; 5070 _ 5080; 5071 _ 5080; 5072 _ 5080; 5074 _ 5079; 5075 _ 5080; 5077 _ 5080; 5080 _ 5081; 5080 _ 5082; 5080 _ 5083; 5085 _ 5089; 5086 _ 5087; 5087 _ 5088; 5091 _ 5096; 5091 _ 5097; 5092 _ 5095; 5093 _ 5094; 5102 _ 5103; 5105 _ 5106; 5117 _ 5118; 5120 _ 5121; 5124 _ 5126; 5127 _ 5128; 5131 _ 5132; 5142 _ 5143; 5148 _ 5151; 5149 _ 5150; 5152 _ 5153; 5168 _ 5169; 5171 _ 5172; 5178 _ 5179; 5192 _ 5197; 5193 _ 5197; 5194 _ 5197; 5207 _ 5208; 5213 _ 5221; 5215 _ 5221; 5216 _ 5221; 5217 _ 5221; 5218 _ 5229; 5220 _ 5221; 5221 _ 5222; 5221 _ 5223; 5221 _ 5224; 5221 _ 5225; 5221 _ 5226; 5221 _ 5230; 5221 _ 5232; 5227 _ 5229; 5228 _ 5234; 5235 _ 5239; 5236 _ 5239; 5239 _ 5243; 5240 _ 5247; 5242 _ 5247; 5250 _ 5251; 5253 _ 5255; 5254 _ 5256; 5258 _ 5261; 5259 _ 5260; 5262 _ 5264; 5263 _ 5264; 5266 _ 5267; 5276 _ 5285; 5277 _ 5285; 5279 _ 5285; 5280 _ 5285; 5281 _ 5285; 5283 _ 5285; 5284 _ 5287; 5285 _ 5286; 5285 _ 5287; 5291 _ 5292; 5293 _ 5294; 5293 _ 5295; 5296 _ 5297; 5296 _ 5298; 5296 _ 5299; 5296 _ 5302; 5300 _ 5301; 5308 _ 5311; 5309 _ 5311; 5309 _ 5312; 5310 _ 5311; 5314 _ 5324; 5315 _ 5316; 5315 _ 5323; 5318 _ 5320; 5319 _ 5322; 5320 _ 5321; 5320 _ 5323; 5326 _ 5327; 5339 _ 5340; 5341 _ 5344; 5342 _ 5348; 5345 _ 5347; 5346 _ 5349; 5351 _ 5352; 5351 _ 5354; 5359 _ 5360; 5364 _ 5369; 5366 _ 5367; 5367 _ 5368; 5377 _ 5379; 5378 _ 5379; 5379 _ 5382; 5380 _ 5381; 5385 _ 5386; 5385 _ 5388; 5386 _ 5387; 5389 _ 5390; 5392 _ 5394; 5392 _ 5395; 5397 _ 5398; 5398 _ 5399; 5398 _ 5400; 5398 _ 5401; 5398 _ 5402; 5398 _ 5404; 5398 _ 5405; 5398 _ 5406; 5423 _ 5424; 5430 _ 5432; 5431 _ 5433; 5437 _ 5438; 5442 _ 5444; 5445 _ 5447; 5446 _ 5449; 5448 _ 5449; 5451 _ 5452; 5451 _ 5454; 5451 _ 5455; 5451 _ 5457; 5452 _ 5459; 5452 _ 5460; 5453 _ 5458; 5454 _ 5460; 5461 _ 5464; 5462 _ 5464; 5463 _ 5464; 5471 _ 5473; 5472 _ 5474; 5476 _ 5477; 5478 _ 5480; 5478 _ 5482; 5479 _ 5487; 5481 _ 5485; 5481 _ 5486; 5481 _ 5487; 5481 _ 5488; 5483 _ 5484; 5484 _ 5489; 5491 _ 5492; 5496 _ 5497;5501 _ 5502; 5501 _ 5503;5501 _ 5506;5501 _ 5509; 5504 _ 5508; 5505 _ 5507; 5507 _ 5509; 5508 _ 5510; 5511 _ 5512; 5515 _ 5519; 5516 _ 5519; 5517 _ 5519; 5526 _ 5527;5532 _ 5536; 5541 _ 5542; 5543 _ 5545; 5550 _ 5551; 5551 _ 5552; 5554 _ 5556; 5555 _ 5560; 5557 _ 5558; 5558 _ 5559;5558 _ 5563;5567 _ 5568;5570 _ 5573;5571_ 5570;5572 _ 5574

Les inventeurs ont ensute démontré qu'il est possible de définir des fonctions de classification à partir de telles paires de probe sets significatives, comme illustré dans l'exemple ci-dessous ou une fonction de classification est construite à partir de 51 paires de probe sets identifiées pour les trois gènes PSEN1, PTPRC et SORL1.

Un score de 0 ou de +1 peut être associé à chaque paire de probe sets pour chaque patient. Un score global par patient est alors obtenu en additionant les scores individuels. Par individu, le score minimal est donc de 0 et le score maximal de 51 (pour ces 51 paires). La régle suivante peut alors être appliquée. Un individu sera classé en AD si son score est supérieur à 51/2 = 25,5 et un individu sera classé en contrôle si son score est inférieur à 51/2 = 25,5. Figure 3 indique les scores obtenus pour les 80 AD et les 70 contrôles. 83% des patients AD sont bien classés et 93% des contrôles sont bien classés.

Des fonctions de classification performantes peuvent donc être caractérisées à partir de ces paires de probe sets significatives.

L'avantage d'une analyse dépendante de l'expression relative de probe sets est que ce type d'analyse doit être non ou peu sensible au procédé de normalisation. Il est ainsi possible d'effectuer le même type d'analyse à partir des valeurs brutes d'expression non normalisée. Cette analyse a permis de définir le groupe TSP NN représenté par les 1865 séquences de paires suivantes aux scores supérieurs à 0,5 (colonne H du tableau 2):
SEQIDNos: 1_3;1_9; 1_10; 1_11; 1_12; 1_13; 1_14;3_4;4_10;7_9;8_9; 23 _ 24; 27 _ 30; 32 _ 33; 33 _ 34; 33 _ 35; 33 _ 37; 44 _ 49; 56 _ 57; 64 _ 65; 68 _ 72; 69 _ 73; 69 _ 77; 69 _ 78; 69 _ 81; 69 _ 82; 69 _ 83; 70 _ 83; 92 _ 93; 95 _ 96; 95 _ 97; 106 _ 107; 111 _112; 122 _ 124; 123 _ 124; 129 _ 130; 131 _ 132; 135 _ 139; 135 _ 142; 135 _ 144; 135 _ 145; 135 _ 147; 136 _ 137; 136 _ 138; 136 _ 140; 136 _ 141; 136 _ 148; 151 _ 152;154 _155;158 _159;160 _161;161_162;168 _169;168 _170;176 _177;185 _ 186;188 _189;194 _ 198;195 _203;195 _210;197 _210;200_205;204 _211;205 _ 208; 206 _ 212;214 _ 215; 220 _ 221; 223 _ 224;224 _ 226;224 _ 227;224 _ 228; 224 _ 230;224 _ 232;224 _ 233; 224 _ 234;224 _ 235; 224 _ 236; 224 _ 237;224 _ 238; 224 _ 239;224 _ 240;224 _ 241; 224 _ 242;224 _ 243; 224 _ 245;224 _ 246;224 _ 247; 224 _ 248; 224 _ 249; 224 _ 250; 224 _ 251; 224 _ 252; 224 _ 253; 224 _ 254; 224 _ 255; 224 _ 256;224 _ 257;224 _ 258;224 _ 259;231 _ 224; 266 _ 269;267 _ 269;269 _ 273;270 _ 272;270 _ 275;279 _ 281;282 _ 283;287 _ 288;289 _ 290; 296 _ 297; 302 _ 303; 303 _ 305; 314 _ 315;318 _ 319; 323 _ 324;326 _ 327; 328 _ 331;329 _ 332;330 _ 331; 331 _ 333; 331 _ 334;331 _ 335;331 _ 336;344 _ 346; 358 _ 361; 359 _ 360; 380 _ 381; 383 _ 384;385 _ 386; 386 _ 391; 387 _ 389; 393 _ 395; 411 _ 412;415 _ 418; 428 _ 429; 429 _ 430; 429 _ 431; 433 _ 434; 434 _ 438;447 _ 453;460 _ 461; 463 _ 464;477 _ 478; 477 _ 479; 481 _ 483;484 _ 485; 486 _ 488; 486 _ 489; 486 _ 490; 486 _ 492; 486 _ 494; 486 _ 495; 486 _ 496; 486 _ 497;486 _ 498; 486 _ 500; 491 _ 493; 525 _ 526; 533 _ 534; 545 _ 547; 546 _ 552;547 _ 553; 547 _ 554; 549 _ 551; 549 _ 552;562 _ 563; 562 _ 564;575 _ 578;576 _ 578; 577 _ 578; 579 _ 580;584 _ 585; 586 _ 587; 588 _ 589; 604 _ 605;610 _ 611; 615 _ 616; 621 _ 629;622 _ 629; 623 _ 629;624 _ 629; 627 _ 629; 628 _ 629; 629 _ 630; 629 _ 631; 629 _ 632; 629 _ 633; 639 _ 640;642 _ 643;644 _ 645; 670 _ 672;674 _ 675;678 _ 679; 680 _ 681; 697 _ 698; 699 _ 702;701 _ 702;717 _ 718; 727 _ 728; 729 _ 730; 733 _ 735; 742 _ 744; 742 _ 746;742 _ 747; 742 _ 748; 742 _ 749; 750 _ 753; 755 _ 756; 761 _ 763; 765 _ 767; 773 _ 774; 779 _ 781; 791 _ 795; 791 _ 798; 791 _ 799; 791 _ 800; 791 _ 803; 791 _ 814; 792 _ 811; 793 _ 811;794 _ 811; 796 _ 800; 796 _ 817; 796 _ 824; 797 _ 809; 797 _ 811;802 _ 808; 804 _ 811; 805 _ 811; 806 _ 825; 808 _ 816;808 _ 823; 809 _ 813; 809 _ 820; 810 _ 823; 811 _ 813; 811 _ 815;811_ 818;811 _ 819;811 _ 820; 811 _ 821; 811 _ 822; 812 _ 821; 827 _ 829; 828 _ 830; 832 _ 835; 834 _ 836; 835 _ 837; 835 _ 838; 835 _ 841; 836 _ 841; 838 _ 840; 843 _ 844; 848 _ 851; 849 _ 850; 852 _ 853; 852 _ 854; 856 _ 863; 859 _ 865; 861 _ 864; 862 _ 865; 869 _ 870; 873 _ 882; 874 _ 882; 875 _ 882; 876 _ 882; 877 _ 882; 879 _ 882; 880 _ 884; 881 _ 884; 882 _ 885; 886 _ 889; 887 _ 890;888 _ 890; 895 _ 899; 895 _ 900; 897 _ 898; 899 _ 901; 899 _ 903; 900 _ 902; 906 _ 910; 908 _ 911; 917 _ 918; 937 _ 938; 943 _ 945; 943 _ 946; 943 _ 947; 944 _ 947; 953 _ 954; 960 _ 961; 961 _ 962; 961 _ 964; 961 _ 965; 961 _ 967; 961 _ 969; 970 _ 971; 974 _ 975; 993 _ 1011; 994 _ 1013; 995 _ 1001; 995 _ 1002; 995 _ 1003; 996 _ 999; 997 _ 1000; 998 _ 1009; 1000 _ 1009; 1005 _ 1011; 1006 _ 1011; 1007 _ 1011; 1008 _ 1011; 1009 _ 1010; 1018 _ 1021; 1022 _ 1025; 1035 _ 1036; 1038 _ 1039; 1047 _ 1048; 1051 _ 1052; 1054 _ 1055; 1056 _ 1063; 1057 _ 1060; 1059 _ 1061; 1065 _ 1067; 1066 _ 1067; 1067 _ 1068; 1067 _ 1069; 1067 _ 1071; 1073 _ 1074; 1081 _ 1083; 1081 _ 1084; 1087 _ 1088; 1088 _ 1089; 1088 _ 1090; 1088 _ 1092; 1097 _ 1099; 1098 _ 1101; 1098 _ 1105; 1098 _ 1106; 1098 _ 1110; 1102 _ 1117; 1103 _ 1110; 1107 _ 1111; 1107 _ 1120; 1110 _ 1118; 1110 _1119; 1123 _ 1133; 1124 _ 1133; 1125 _ 1126; 1125 _ 1129; 1125 _ 1130; 1125 _ 1131; 1125 _ 1132; 1135 _ 1138; 1141 _ 1144; 1143 _ 1144; 1174 _ 1177; 1180 _ 1183; 1181 _ 1182; 1182 _ 1184; 1188 _ 1192; 1189 _ 1190; 1193 _ 1194; 1196 _ 1197; 1196 _ 1198; 1199 _ 1200; 1204 _ 1205; 1208 _ 1209; 1208 _ 1210; 1212 _ 1213; 1217 _ 1237; 1219 _ 1239; 1221 _ 1237; 1222 _ 1239; 1223 _1242; 1224 _ 1239; 1226 _ 1244; 1226 _ 1246; 1226 _ 1261; 1227 _ 1245; 1228 _ 1233; 1229 _ 1233; 1229 _ 1235; 1229 _ 1247;1231 _1237;1233 _ 1260; 1234 _ 1242;1234 _ 1259; 1236 _ 1242; 1237 _ 1243; 1237 _ 1245; 1237 _ 1249; 1237 _ 1251; 1237 _ 1263; 1237 _ 1264; 1237 _ 1265; 1237 _1267;1238 _1245;1238 _1249;1239 _ 1262; 1239 _ 1266; 1239 _ 1269; 1242 _ 1248; 1242 _ 1250; 1242 _ 1252; 1242 _ 1255; 1245 _ 1254; 1247 _ 1253; 1247 _ 1254; 1248 _1258;1248 _1259;1249 _1254;1275 _1276;1281 _ 1285;1282 _1284;1283 _ 1284; 1295 _ 1296; 1295 _ 1297; 1295 _ 1298; 1299 _ 1305; 1299 _ 1306; 1300 _ 1303; 1301 _ 1302; 1310 _ 1311; 1310 _ 1316; 1310 _ 1319; 1310 _ 1320; 1310 _ 1325; 1312 _ 1324; 1312 _ 1327; 1313 _ 1326; 1315 _ 1328; 1317 _ 1326; 1318 _ 1319; 1323 _ 1329; 1323 _ 1330; 1326 _ 1331; 1334 _ 1337; 1335 _ 1336; 1338 _ 1343; 1350 _ 1352; 1351 _ 1353; 1359 _ 1360; 1368 _ 1372; 1369 _ 1383; 1371 _ 1374; 1376 _ 1388; 1377 _ 1379; 1377 _ 1381; 1378 _ 1383;1381 _ 1391; 1383 _ 1392;1384 _ 1393; 1400 _ 1402; 1402 _ 1403; 1402 _ 1405; 1402 _ 1407; 1402 _ 1409; 1402 _ 1410; 1402 _ 1411; 1403 _ 1404; 1406 _ 1408; 1408 _ 1412; 1418 _ 1419; 1423 _ 1427; 1424 _ 1427; 1425 _ 1427; 1426 _ 1427; 1429 _ 1430; 1429 _ 1431; 1433 _ 1435; 1434 _ 1435; 1436 _ 1437; 1443 _ 1444; 1444 _ 1446; 1448 _ 1450; 1448 _ 1452; 1448 _ 1453; 1448 _ 1456; 1448 _ 1457; 1448 _ 1458; 1448 _ 1459; 1448 _ 1460; 1448 _ 1464; 1448 _ 1465; 1448 _ 1468; 1449 _ 1455; 1451 _ 1471; 1466 _ 1469; 1475 _ 1476; 1476 _ 1480; 1476 _ 1481; 1476 _ 1482; 1477 _ 1478; 1478 _ 1483; 1485 _ 1489; 1486 _ 1490; 1488 _ 1490; 1488 _ 1499; 1490 _ 1492; 1490 _ 1497; 1490 _ 1500; 1501 _ 1503; 1501 _ 1504; 1506 _ 1505; 1511 _ 1514; 1536 _ 1539; 1537 _ 1539; 1538 _ 1539; 1539 _ 1544; 1541 _ 1543; 1543 _ 1545; 1546 _ 1548; 1546 _ 1550; 1547 _ 1549; 1553 _ 1554; 1553 _ 1555; 1556 _ 1558; 1557 _ 1558; 1564 _ 1565; 1567 _ 1572; 1568 _ 1569; 1570 _ 1571; 1570 _ 1574; 1572 _ 1573; 1572 _ 1574; 1586 _1587;1594 _ 1596;1602 _ 1601; 1603 _ 1605; 1604 _ 1606; 1610 _ 1615; 1611 _ 1614; 1612 _ 1615; 1613 _ 1614; 1620 _ 1625; 1625 _ 1626; 1636 _ 1637; 1641 _ 1642; 1641 _1643;1641 _1644;1641 _1645;1641_1646;1647 _ 1648; 1655 _ 1657; 1656 _ 1657; 1658 _ 1659; 1658 _ 1660; 1658 _ 1661; 1658 _ 1662; 1658 _ 1664; 1658 _ 1665; 1658 _1666;1680 _1681;1682 _1685;1683 _ 1684; 1683 _ 1686; 1690 _ 1693; 1703 _ 1704; 1704 _ 1707; 1705 _ 1707; 1706 _ 1708; 1707 _ 1708; 1711 _ 1712; 1715 _ 1716; 1719 _ 1720; 1726 _ 1727; 1727 _ 1728; 1732 _ 1738; 1733 _ 1745; 1736 _ 1745; 1737 _ 1741; 1738 _ 1745; 1739 _ 1745; 1745 _ 1750; 1749 _ 1752; 1754 _ 1755; 1754 _ 1759; 1757 _1758;1760 _1761;1768 _1769;1778 _1781;1780 _1782;1781 _ 1783;1799 _ 1800; 1803 _ 1804; 1817 _ 1818; 1817 _ 1819; 1822 _ 1823; 1835 _ 1837; 1842 _ 1843; 1848 _ 1850; 1863 _ 1866; 1865 _ 1866; 1866 _ 1867; 1866 _ 1868; 1866 _ 1869; 1866 _ 1872; 1866 _ 1873; 1866 _ 1874; 1866 _ 1875; 1866 _ 1876; 1866 _ 1877; 1866 _ 1878; 1866 _ 1879; 1866 _ 1880; 1866 _ 1881; 1866 _ 1882; 1866 _ 1883; 1866 _ 1886; 1866 _ 1887; 1866 _ 1888; 1866 _ 1889; 1866 _ 1890; 1866 _ 1891; 1866 _ 1893; 1866 _ 1895; 1866 _ 1897; 1866 _ 1900; 1866 _ 1902; 1866 _ 1903; 1866 _ 1904; 1866 _ 1906; 1866 _ 1907; 1866 _ 1908; 1866 _ 1909; 1870 _ 1866; 1921 _ 1922; 1921 _ 1923; 1922 _ 1927; 1922 _ 1928; 1922 _ 1929; 1925 _ 1932; 1925 _ 1933; 1926 _ 1934; 1936 _ 1937; 1939 _ 1940; 1939 _ 1941; 1939 _ 1943; 1939 _ 1944; 1939 _ 1945; 1949 _ 1954; 1949 _ 1955; 1957 _ 1958; 1957 _ 1959; 1957 _ 1960; 1965 _ 1967; 1968 _ 1970; 1968 _ 1973; 1974 _ 1975; 1979 _ 1980; 1981 _ 1982; 1988 _ 1992; 1989 _ 1990; 1991 _ 1993; 1997 _ 1998; 2004 _ 2005; 2013 _ 2016; 2014 _ 2016; 2018 _ 2019; 2026 _ 2027; 2028 _ 2029; 2029 _ 2031; 2029 _ 2034; 2030 _ 2032; 2030 _ 2033; 2035 _ 2036; 2035 _ 2037; 2039 _ 2040; 2046 _ 2048; 2051 _ 2059; 2052 _ 2057; 2054 _ 2058; 2055 _ 2059; 2056 _ 2059;2065 _ 2067; 2065 _ 2079; 2066 _ 2069; 2067 _ 2076; 2068 _ 2074; 2068 _ 2077; 2070 _ 2078; 2071 _ 2082; 2072 _ 2073; 2072 _ 2081; 2073 _ 2075; 2075 _ 2081; 2076 _ 2080; 2077 _ 2083; 2077 _ 2084; 2089 _ 2090; 2091 _ 2092; 2094 _ 2095; 2100 _ 2104; 2101 _ 2102; 2102 _ 2105; 2103 _ 2105; 2138 _ 2146; 2149 _ 2152; 2149 _ 2153; 2150 _ 2155; 2151 _ 2155; 2151 _ 2156; 2162 _ 2163; 2169 _ 2171; 2169 _ 2172; 2175 _ 2176; 2178 _ 2179; 2178 _ 2180; 2178 _ 2181; 2183 _ 2184; 2189 _ 2191; 2191 _ 2193; 2191 _ 2198; 2191 _ 2200; 2192 _ 2199; 2196 _ 2198; 2201 _ 2202; 2205 _ 2206; 2209 _ 2210; 2220 _ 2223; 2221 _ 2226;2222 _ 2226; 2224 _ 2226; 2226 _ 2227; 2226 _ 2228; 2226 _ 2230; 2226 _ 2233; 2226 _ 2234; 2236 _ 2237; 2236 _ 2238; 2239 _ 2240; 2244 _ 2245; 2248 _ 2262; 2251 _ 2262; 2253 _ 2262; 2262 _ 2265; 2273 _ 2276; 2274 _ 2276; 2275 _ 2276;2282 _ 2283; 2286 _ 2287; 2286 _ 2292; 2286 _ 2297; 2288 _ 2299; 2289 _ 2299; 2290 _ 2299; 2291 _ 2299; 2291 _ 2301; 2294 _ 2298; 2295 _ 2296; 2302 _ 2304; 2308 _ 2309; 2335 _ 2337; 2351 _ 2355; 2351 _ 2357; 2353 _ 2356; 2354 _ 2359; 2363 _ 2364; 2372 _ 2387; 2375 _ 2388; 2403 _ 2404; 2419 _ 2420; 2422 _ 2424; 2423 _ 2424; 2424 _ 2427; 2424 _ 2428; 2425 _ 2427; 2429 _ 2430; 2431 _ 2432; 2432 _ 2433; 2432 _ 2434; 2437 _ 2439; 2438 _ 2440; 2455 _ 2457; 2455 _ 2458; 2458 _ 2466; 2459 _ 2463; 2462 _ 2463; 2464 _ 2467; 2470 _ 2471; 2472 _ 2473; 2472 _ 2474; 2475 _ 2476; 2477 _ 2478; 2477 _ 2479; 2480 _ 2481; 2482 _ 2483; 2487 _ 2488; 2491 _ 2492; 2496 _ 2505; 2496 _ 2507; 2496 _ 2509; 2497 _ 2499; 2497 _ 2504; 2497 _ 2505; 2497 _ 2506; 2497 _ 2508; 2497 _ 2510; 2497 _ 2512;2497 _ 2516;2498 _ 2504; 2498 _ 2505; 2498 _ 2509; 2498 _ 2510; 2498 _ 2511; 2498 _ 2513; 249.8 _ 2514; 2498 _ 2515; 2501 _ 2511; 2502 _ 2509; 2502 _ 2511; 2502 _ 2515; 2503 _ 2508; 2525 _ 2526; 2528 _ 2535; 2529 _ 2537; 2532 _ 2535; 2541 _ 2570; 2541 _ 2572;2542 _ 2547;2542 _ 2550; 2542 _ 2567; 2542 _ 2568; 2543 _ 2567; 2544 _ 2572; 2549 _ 2544; 2549 _ 2545; 2549 _ 2561; 2549 _ 2576; 2551 _ 2575; 2552 _ 2575; 2553 _ 2557; 2554 _ 2558; 2561 _ 2572; 2561 _ 2573; 2562 _ 2571; 2563 _ 2566; 2563 _ 2569; 2564 _ 2567; 2564 _ 2568;2572 _ 2574;2597 _ 2598; 2608 _ 2609; 2609 _ 2611; 2610 _ 2611; 2614 _ 2615; 2624 _ 2631; 2625 _ 2626; 2626 _ 2628; 2626 _ 2629; 2627 _ 2630; 2634 _ 2635; 2637 _ 2639;2640 _ 2642; 2641 _ 2642; 2642 _ 2643; 2644 _ 2645; 2651 _ 2655; 2652 _ 2653; 2652 _ 2656; 2652 _ 2657; 2654 _ 2655; 2661 _ 2670; 2661 _ 2671; 2662 _ 2664; 2662 _ 2671; 2663 _ 2669; 2664 _ 2673;2665 _ 2667; 2666 _ 2669; 2670 _ 2673; 2674 _ 2675; 2677 _ 2678; 2677 _ 2688; 2677 _ 2689; 2677 _ 2694; 2678 _ 2686; 2678 _ 2690; 2681 _ 2694; 2682 _ 2685;2682 _ 2686;2682 _ 2687;2682 _ 2690; 2683 _ 2692; 2686 _ 2688; 2686 _ 2689; 2687 _ 2688; 2688 _ 2690; 2689 _ 2690; 2693 _2694;2704 _2705;2706 _2708;2707 _2711;2715 _ 2721; 2715 _ 2722; 2716 _ 2718; 2716 _ 2726; 2717 _ 2726; 2718 _ 2722; 2719 _ 2725; 2719 _ 2727; 2722 _ 2723; 2730 _ 2745; 2731 _ 2732;2732 _ 2733; 2732 _ 2734;2732 _ 2735;2732 _ 2736;2732 _ 2737; 2732 _ 2738; 2732 _ 2739; 2746 _ 2748; 2747 _ 2748; 2748 _ 2750; 2748 _ 2757; 2748 _ 2758; 2748 _ 2759; 2749 _ 2751; 2751 _ 2756; 2753 _ 2754; 2760 _ 2761; 2764 _ 2766; 2777 _ 2779; 2778 _ 2780; 2784 _ 2786; 2785 _ 2786; 2792 _ 2794; 2797 _ 2800; 2797 _ 2810; 2798 _ 2799; 2800 _ 2807; 2800 _ 2808; 2801 _ 2810;2802 _ 2806;2808 _ 2810;2812 _ 2814; 2812 _ 2815; 2819 _ 2827; 2820 _ 2822; 2821 _ 2822; 2822 _ 2829; 2826 _ 2832; 2830 _ 2832; 2836 _ 2837; 2839 _ 2840; 2840 _ 2841; 2840 _ 2842; 2846 _ 2847; 2848 _ 2849; 2854 _ 2857; 2854 _ 2864; 2854 _ 2867; 2855 _ 2858; 2855 _ 2859; 2855 _ 2861; 2856 _ 2861; 2861 _ 2865; 2875 _ 2881; 2875 _ 2884;2878 _ 2880; 2878 _ 2883; 2878 _ 2885; 2880 _ 2882; 2911 _ 2912; 2926 _ 2927; 2928 _ 2929; 2930 _ 2931; 2948 _ 2949; 2952 _ 2953; 2952 _ 2954; 2955 _ 2959; 2955 _ 2960; 2956 _ 2961;2957 _ 2958; 2962 _ 2965; 2963 _ 2966; 2968 _ 2971; 2969 _ 2972; 2970 _ 2972; 2974 _ 2975; 2975 _ 2977; 2995 _ 2996; 2998 _ 2999; 3001 _ 3002; 3001 _ 3003; 3006 _ 3007; 3015 _ 3023; 3016 _ 3023; 3017 _ 3018; 3017 _ 3023; 3017 _ 3026; 3018 _ 3030; 3018 _ 3032; 3019 _ 3024; 3020 _ 3023; 3021 _ 3023; 3023 _ 3022; 3023 _ 3030; 3023 _ 3032; 3023 _ 3033; 3023 _ 3034; 3027 _ 3032; 3038 _ 3039; 3039 _ 3040; 3046 _ 3048; 3047 _ 3049; 3054 _ 3056; 3057 _ 3058; 3059 _ 3060; 3062 _ 3063; 3069 _ 3070; 3077 _ 3078; 3077 _ 3083; 3077 _ 3084; 3078 _ 3080; 3078 _ 3081; 3078 _ 3082; 3079 _ 3084; 3090 _ 3092; 3091 _ 3092; 3092 _ 3094; 3092 _ 3095; 3092 _ 3096; 3092 _ 3097; 3092 _ 3098; 3100 _ 3101; 3111 _3112; 3111 3113; 3111 _3114; 3111 _ 3115; 3116 _ 3117; 3120 _ 3122; 3120 _ 3142; 3121 _ 3123;3122 _ 3125;3122 _ 3131; 3122 _ 3133;3122 _ 3138;3122 _ 3139; 3122 _ 3146; 3122 _ 3147; 3123 _ 3134; 3123 _ 3135; 3123 _ 3140; 3124 _ 3143; 3124 _ 3144; 3126 _ 3130; 3127 _ 3144; 3129 _ 3145; 3132 _ 3149; 3136 _ 3144; 3136 _ 3145; 3139 _ 3142; 3141 _3147; 3144 _ 3148; 3150 _ 3151; 3154 _ 3155; 3174 _ 3176; 3176 _ 3181; 3177 _ 3182; 3178 _ 3180; 3179 _ 3184; 3180 _ 3183; 3198 _ 3199; 3201 _ 3202; 3209 _ 3210; 3212 _ 3214; 3227 _ 3230; 3228 _ 3231; 3229 _ 3232; 3236 _ 3238; 3236 _ 3239; 3236 _ 3240; 3236 _ 3241; 3251 _ 3256; 3253 _ 3255; 3254 _ 3255; 3257 _ 3258; 3261 _ 3263; 3264 _ 3265; 3267 _ 3271; 3269 _ 3271; 3274 _ 3275; 3287 _ 3293; 3288 _ 3289; 3290 _ 3293; 3290 _ 3294; 3290 _ 3295; 3291 _ 3293; 3316 _ 3318; 3317 _ 3321; 3318 _ 3319; 3339 _ 3344; 3342 _ 3347; 3343 _ 3348; 3351 _ 3352; 3351 _ 3353; 3354 _ 3357; 3355 _ 3363; 3357 _ 3364; 3359 _ 3363; 3367 _ 3368; 3368 _ 3369; 3382 _ 3383; 3385 _ 3396; 3386 _ 3402; 3387 _ 3395; 3387 _ 3396; 3388 _ 3398; 3388 _ 3401; 3390 _ 3391; 3390 _ 3392; 3390 _ 3400; 3391 _ 3397;3395 _ 3405; 3396 _ 3403; 3396 _ 3404; 3396 _ 3405; 3397 _ 3399; 3406 _ 3407; 3414 _ 3415; 3418 _ 3419; 3423 _ 3424; 3425 _ 3426; 3431 _ 3436; 3433 _ 3441; 3434 _ 3439; 3435 _ 3436; 3438 _ 3440; 3444 _ 3448; 3444 _ 3465; 3444 _ 3471; 3444 _ 3472; 3445 _ 3446; 3447 _ 3454; 3447 _ 3459; 3447 _ 3460; 3449 _ 3450; 3451 _ 3472; 3454 _ 3469; 3454 _ 3470; 3456 _ 3464; 3456 _ 3465; 3457 _ 3468; 3458 _ 3466; 3458 _ 3467; 3475 _ 3477; 3475 _ 3479; 3476 _ 3477; 3478 _ 3481; 3483 _ 3484; 3492 _ 3493; 3493 _ 3495; 3494 _ 3496; 3495 _ 3497; 3498 _ 3499; 3501 _ 3504; 3511 _ 3513; 3512 _ 3513; 3513 _ 3514; 3543 _ 3544; 3544 _ 3545; 3546 _ 3549; 3550 _ 3551; 3556 _ 3557; 3568 _ 3569; 3570 _ 3571; 3584 _ 3585; 3591 _ 3592; 3591 _ 3593; 3591 _3594; 3605 _ 3606; 3610 _ 3611; 3610 _ 3612; 3610 _ 3614; 3610 _ 3618; 3610 _ 3619; 3610 _ 3621; 3611 _ 3622; 3613 _ 3618; 3613 _ 3619; 3613 _ 3620; 3615 _ 3618; 3630 _ 3635; 3632 _ 3634; 3632 _ 3637; 3632 _ 3641; 3633 _ 3640; 3634 _ 3636; 3638 _ 3642; 3647 _ 3648; 3651 _ 3653; 3653 _ 3654; 3653 _ 3655; 3653 _ 3656; 3653 _ 3660; 3653 _ 3661; 3653 _ 3662; 3653 _ 3663; 3653 _ 3665; 3653 _ 3666; 3653 _ 3667; 3653 _ 3669; 3677 _ 3680; 3678 _ 3695; 3679 _ 3690; 3681 _ 3682; 3682 _ 3687; 3682 _ 3693; 3682 _ 3694; 3682 _ 3697; 3683 _ 3689; 3683 _ 3696; 3684 _ 3693; 3701 _ 3702; 3710 _ 3720; 3711 _ 3719; 3716 _ 3717; 3721 _ 3724; 3723 _ 3724; 3724 _ 3726; 3724 _ 3727; 3724 _ 3728; 3724 _ 3729; 3730 _ 3731; 3735 _ 3736; 3737 _ 3741; 3738 _ 3741; 3739 _ 3741; 3740 _ 3741; 3744 _ 3745; 3744 _ 3749; 3744 _ 3750; 3746 _ 3747; 3754 _ 3756; 3755 _ 3757; 3760 _ 3763; 3764 _ 3765; 3766 _ 3767; 3767 _ 3768; 3767 _ 3769; 3767 _ 3770; 3774 _ 3775; 3779 _ 3780; 3780 _ 3781; 3784 _ 3785; 3787 _ 3788; 3800 _ 3803; 3801 _ 3802; 3817 _ 3818; 3825 _ 3827; 3831 _ 3832; 3833 _ 3834; 3834 _ 3839; 3834 _ 3840; 3837 _ 3839; 3850 _ 3857; 3852 _ 3857; 3852 _ 3859; 3857 _ 3858; 3857 _ 3860; 3865 _ 3866; 3868 _ 3869; 3873 _ 3875; 3874 _ 3876; 3874 _ 3877; 3874 _ 3881; 3874 _ 3883; 3885 _ 3887; 3891 _ 3892; 3893 _ 3894; 3901 _ 3902; 3903 _ 3904; 3904 _ 3905; 3906 _ 3907; 3908 _ 3909; 3909 _ 3910; 3909 _ 3911; 3918 _ 3922; 3920 _ 3923; 3924 _ 3928; 3925 _ 3926; 3925 _ 3927; 3941 _ 3942; 3945 _ 3947; 3946 _ 3948; 3946 _ 3949; 3980 _ 3981; 3980 _ 3984; 3985 _ 3986; 3988 _ 3989; 3997 _ 3998; 3999 _ 4003; 4000 _ 4002; 4023 _ 4037; 4024 _ 4027; 4025 _ 4029; 4026 _ 4027; 4027 _ 4033; 4027 _ 4035; 4027 _ 4036; 4028 _ 4032; 4031 _ 4036; 4041 _ 4045; 4042 _ 4044; 4043 _ 4044; 4044 _ 4047; 4046 _ 4048; 4049 _ 4050; 4050 _ 4052; 4055 _ 4060; 4056 _ 4059; 4057 _ 4058; 4061 _ 4062; 4067 _ 4076; 4067 _ 4096; 4068 _ 4093; 4069 _ 4093; 4069 _ 4096; 4071 _ 4076; 4073 _ 4093; 4075 _ 4093; 4075 _ 4096; 4076 _ 4101; 4077 _ 4093; 4077 _ 4096; 4079 _ 4093; 4079 _ 4094; 4079 _ 4096; 4085 _ 4096; 4086 _ 4096; 4087 _ 4096; 4088 _ 4096; 4089 _ 4096; 4090 _ 4096; 4093 _ 4098; 4093 _ 4105; 4093 _ 4107; 4093 _ 4108; 4093 _ 4109; 4093 _ 4110; 4096 _ 4099; 4096 _ 4100; 4096 _ 4103; 4096 _ 4104; 4096 _ 4105; 4096 _ 4107; 4096 _ 4108; 4096 _ 4109; 4113 _ 4114; 4116 _ 4117; 4118 _ 4120; 4122 _ 4123; 4125 _ 4127; 4127 _ 4129; 4130 _ 4131; 4132 _ 4136; 4133 _ 4136; 4134 _ 4140; 4135 _ 4138; 4135 _ 4140; 4135 _ 4142; 4135 _ 4152; 4136 _ 4139; 4136 _ 4144; 4137 _ 4141; 4137 _ 4151; 4140 _ 4148; 4140 _ 4153; 4142 _ 4150; 4143 _ 4149; 4147 _ 4149; 4150 _ 4152; 4155 _ 4160; 4156 _ 4157; 4156 _ 4158; 4156 _ 4159; 4162 _ 4163; 4165 _ 4167; 4166 _ 4169; 4175 _ 4176; 4176 _ 4181; 4177 _ 4180; 4178 _ 4180; 4190 _ 4191; 4195 _ 4197; 4196 _ 4197; 4203 _ 4204; 4205 _ 4208; 4210 _ 4211; 4211 _ 4214; 4215 _ 4216; 4217 _ 4218; 4224 _ 4225; 4236 _ 4248; 4237 _ 4239; 4238 _ 4249; 4240 _ 4246; 4241 _ 4249; 4243 _ 4248; 4244 _ 4246; 4251 _ 4252; 4253 _ 4254; 4258 _ 4259; 4261 _ 4263; 4262 _ 4261; 4270 _ 4273; 4271 _ 4277; 4272 _ 4275; 4274 _ 4278; 4283 _ 4291;4284 _ 4285; 4286 _ 4289; 4287 _ 4290; 4288 _ 4290; 4294 _ 4295; 4297 _ 4303; 4297 _ 4305; 4301 _ 4304; 4309 _ 4313; 4310 _ 4312; 4311 _ 4315; 4312 _ 4313; 4312 _ 4314; 4315 _ 4316; 4317 _ 4322; 4318 _ 4324; 4319 _ 4320; 4319 _ 4323; 4321 _ 4325; 4324 _ 4325; 4328 _ 4333; 4328 _ 4334; 4332 _ 4335; 4338 _ 4339; 4355 _ 4356; 4360 _ 4362; 4361 _ 4363; 4366 _ 4369; 4385 _ 4392; 4387 _ 4390; 4387 _ 4391; 4387 _ 4392; 4387 _ 4393; 4396 _ 4398; 4404 _ 4407; 4404 _ 4413; 4406 _ 4408; 4408 _ 4409; 4408 _ 4411; 4408 _ 4412; 4414 _ 4415; 4416 _ 4417; 4417 _ 4418; 4420 _ 4421; 4422 _ 4424; 4436 _ 4437; 4440 _ 4442; 4441 _ 4442; 4442 _ 4443; 4442 _ 4444; 4442 _ 4446; 4442 _ 4449; 4442 _ 4450; 4442 _ 4452; 4442 _ 4453; 4442 _ 4454; 4442 _ 4455; 4442 _ 4456; 4442 _ 4457; 4442 _ 4458; 4442 _ 4459; 4442 _ 4462; 4464 _ 4465; 4464 _ 4466; 4465 _ 4467; 4473 _ 4474; 4476 _ 4477; 4477 _ 4478; 4484 _ 4487; 4485 _ 4486; 4490 _ 4491; 4493 _ 4496; 4494 _ 4496; 4498 _ 4500; 4503 _ 4504; 4510 _ 4513; 4510 _ 4530; 4511 _ 4527; 4512 _ 4527; 4514 _ 4519; 4514 _ 4521; 4516 _ 4526; 4516 _ 4529; 4520 _ 4527; 4520 _ 4531; 4523 _ 4527; 4524 _ 4527; 4533 _ 4534; 4535 _ 4536; 4535 _ 4537; 4538 _ 4539; 4538 _ 4540; 4538 _ 4543; 4538 _ 4545; 4539 _ 4541; 4550 _ 4557; 4561 _ 4562; 4563 _ 4564; 4563 _ 4565; 4563 _ 4566; 4563 _ 4567; 4563 _ 4568; 4563 _ 4570; 4563 _ 4571; 4563 _ 4572; 4563 _ 4573; 4569 _ 4571; 4576 _ 4577; 4579 _ 4580; 4585 _ 4586; 4588 _ 4589; 4589 _ 4590; 4597 _ 4600; 4598 _ 4599; 4603 _ 4604; 4603 _ 4605; 4603 _ 4606; 4604 _ 4607; 4606 _ 4607; 4607 _ 4608; 4614 _ 4615; 4614 _ 4616; 4625 _ 4629; 4625 _ 4651; 4625 _ 4655; 4626 _ 4629; 4627 _ 4649; 4629 _ 4631; 4629 _ 4634; 4629 _ 4635; 4629 _ 4638; 4629 _ 4639; 4629 _ 4640; 4629 _ 4643; 4629 _ 4644; 4629 _ 4645; 4629 _ 4648; 4629 _ 4650; 4629 _ 4653; 4630 _ 4654; 4631 _ 4651; 4631 _ 4654; 4631 _ 4655; 4632 _ 4652; 4633 _ 4652; 4635 _ 4651; 4635 _ 4654; 4635 _ 4655; 4636 _ 4654; 4636 _ 4655; 4637 _ 4651; 4637 _ 4654; 4637 _ 4655; 4639 _ 4651; 4640 _ 4651; 4640 _ 4654; 4640 _ 4655; 4641 _ 4655; 4642 _ 4654; 4645 _ 4651; 4645 _ 4655; 4647 _ 4654; 4648 _ 4654; 4657 _ 4667; 4668 _ 4670; 4669 _ 4670; 4673 _ 4674; 4681 _ 4682; 4684 _ 4685; 4684 _ 4686; 4684 _ 4687; 4689 _ 4690; 4695 _ 4697; 4702 _ 4703; 4704 _ 4710; 4705 _ 4710; 4706 _ 4707; 4707 _ 4708; 4709 _ 4710; 4719 _ 4720; 4723 _ 4733; 4724 _ 4733; 4724 _ 4735; 4724 _ 4736; 4726 _ 4733; 4726 _ 4736; 4728 _ 4733; 4729 _ 4731; 4739 _ 4763; 4740 _ 4749; 4740 _ 4754; 4741 _ 4743; 4741 _ 4753; 4741 _ 4765; 4741 _ 4769; 4742 _ 4744; 4742 _ 4765; 4742 _ 4768; 4742 _ 4770; 4744 _ 4746; 4744 _ 4747; 4744 _ 4755; 4744 _ 4757; 4744 _ 4758; 4744 _ 4762; 4744 _ 4766; 4744 _ 4772; 4745 _ 4764; 4746 _ 4771; 4749 _ 4761; 4750 _ 4768; 4751 _ 4763; 4754 _ 4759; 4760 _ 4765; 4760 _ 4767; 4768 _ 4774; 4771 _ 4772; 4775 _ 4776; 4784 _ 4786; 4785 _ 4786; 4786 _ 4787; 4786 _ 4788; 4793 _ 4795; 4793 _ 4796; 4794 _ 4795; 4814 _ 4815; 4817 _ 4818; 4819 _ 4821; 4820 _ 4822; 4830 _ 4832; 4834 _ 4850; 4836 _ 4843; 4836 _ 4848; 4838 _ 4845; 4840 _ 4846; 4841 _ 4843; 4841 _ 4846; 4841 _ 4847; 4841 _ 4849; 4842 _ 4846; 4852 _ 4853; 4852 _ 4854; 4855 _ 4860; 4858 _ 4860; 4863 _ 4870; 4864 _ 4866; 4864 _ 4871; 4877 _ 4879; 4878 _ 4881; 4880 _ 4881; 4881 _ 4882; 4888 _ 4894; 4889 _ 4893; 4889 _ 4894; 4890 _ 4894; 4902 _ 4903; 4902 _ 4904; 4911 _ 4913; 4914 _ 4915; 4916 _ 4919; 4930 _ 4931; 4936 _ 4937; 4939 _ 4942; 4940 _ 4941; 4947 _ 4951; 4950 _ 4951; 4971 _ 4972; 4983 _ 4984; 4989 _ 4994; 4989 _ 5000; 4990 _ 5000; 4990 _ 5001; 4991 _ 5000; 4992 _ 5006; 4992 _ 5008; 4993 _ 5001; 4995 _ 5003; 4995 _ 5007; 4995 _ 5010; 4996 _ 5009; 5000 _ 5002; 5000 _ 5005; 5001 _ 5008; 5013 _ 5014; 5031 _ 5032; 5033 _ 5034; 5040 _ 5042; 5054 _ 5055; 5054 _ 5056; 5054 _ 5058; 5064 _ 5067; 5067 _ 5068; 5070 _ 5080; 5071 _ 5080; 5072 _ 5080; 5073 _ 5080; 5074 _ 5079; 5075 _ 5080; 5076 _ 5078; 5077 _ 5080; 5080 _ 5081; 5080 _ 5082; 5080 _ 5083; 5086 _ 5087; 5087 _ 5088; 5091 _ 5096; 5092 _ 5095; 5093 _ 5094; 5102 _ 5103; 5105 _ 5106; 5114 _ 5115; 5127 _ 5128; 5129 _ 5130; 5149 _ 5150; 5152 _ 5153; 5176 _ 5177; 5178 _ 5179; 5192 _ 5197; 5193 _ 5197; 5194 _ 5197; 5214 _ 5221; 5215 _ 5221; 5216 _ 5221; 5217 _ 5221; 5219 _ 5221; 5220 _ 5221; 5221 _ 5222; 5221 _ 5223; 5221 _ 5224; 5221 _ 5225; 5221 _ 5230; 5221 _ 5232; 5227 _ 5229; 5235 _ 5239; 5236 _ 5237; 5236 _ 5239; 5239 _ 5243; 5240 _ 5247; 5242 _ 5247; 5246 _ 5248; 5250 _ 5251; 5252 _ 5254; 5258 _ 5261; 5259 _ 5260; 5263 _ 5264; 5275 _ 5278; 5276 _ 5285; 5277 _ 5285; 5279 _ 5282; 5279 _ 5285; 5280 _ 5285; 5281 _ 5285; 5283 _ 5285; 5284 _ 5287; 5285 _ 5286; 5285 _ 5287; 5291 _ 5292; 5293 _ 5294; 5293 _ 5295; 5296 _ 5297; 5296 _ 5302; 5300 _ 5301; 5303 _ 5304; 5306 _ 5307; 5308 _ 5311; 5309 _ 5311; 5309 _ 5312; 5310 _ 5311; 5311 _ 5313; 5315 _ 5316; 5319 _ 5322; 5319 _ 5323; 5320 _ 5321; 5320 _ 5323; 5339 _ 5340; 5345 _ 5347; 5346 _ 5349; 5351 _ 5352; 5351 _ 5354; 5356 _ 5360; 5357 _ 5358; 5359 _ 5360; 5361 _ 5362; 5364 _ 5369; 5366 _ 5367; 5367 _ 5368; 5377 _ 5379; 5378 _ 5379; 5379 _ 5382; 5385 _ 5386; 5385 _ 5388; 5386 _ 5387; 5389 _ 5390; 5392 _ 5393; 5392 _ 5394; 5392 _ 5395; 5397 _ 5398; 5398 _ 5399; 5398 _ 5400; 5398 _ 5403; 5418 _ 5420; 5422 _ 5424; 5423 _ 5424; 5424 _ 5426; 5424 _ 5427; 5431 _ 5433; 5445 _ 5447; 5446 _ 5449; 5448 _ 5449; 5451 _ 5452; 5451 _ 5454; 5451 _ 5455; 5451 _ 5457; 5452 _ 5459; 5453 _ 5458; 5454 _ 5456; 5461 _ 5464; 5462 _ 5464; 5463 _ 5464; 5471 _ 5473; 5471 _ 5475; 5476 _ 5477; 5478 _ 5480; 5478 _ 5482; 5479 _ 5487; 5481 _ 5486; 5481 _ 5487; 5481 _ 5488; 5484 _ 5489; 5490 _ 5493; 5496 _ 5497; 5501 _ 5502; 5501 _ 5503; 5501 _ 5506; 5504 _ 5508; 5508 _ 5510; 5514 _ 5519; 5515 _ 5519; 5516 _ 5519; 5517 _ 5519; 5543 _ 5545; 5544 _ 5546; 5547 _ 5548; 5550 _ 5551; 5551 _ 5552; 5554 _ 5556; 5555 _ 5560; 5557 _ 5558; 5558 _ 5559; 5558 _ 5563; 5565 _ 5566; 5567 _ 5568; 5570 _ 5573; 5571 _ 5570; 5572 _ 5574
   78% des paires de ce groupe sont présentes dans le groupe TSP N établi à partir des valeurs normalisées.

Finalement une telle analyse est également possible en comparant des probe sets provenant de gènes différents. Nous avons effectué cette analyse à partir des 1300 probe sets les plus significatifs issus de l'analyse ANOVA. Un seuil fixé à 0,6 permet de caractériser 406 paires formant le groupe TSP 1300 SS (colonne G du tableau 2), associées aux séquences des paires :
SEQ ID NO : 118 _ 5254; 153 _ 2416; 153 _ 2732; 153 _ 4779; 184 _ 4493; 293 _ 1731; 301 _ 2727; 306 _ 455; 306 _ 614; 306 _ 770; 306 _ 1422; 306 _ 1585; 306 _ 1694; 306 _ 1971; 306 _ 2343; 306 _ 2732; 306 _ 2913; 306 _ 3000; 306 _ 3957; 306 _ 4076; 306 _ 4091; 306 _ 4096; 306 _ 4097; 306 _ 4194; 306 _ 4550; 306 _ 4620; 306 _ 4779; 306 _ 5201; 306 _ 5206; 306 _ 5254; 306 _ 5419; 354 _ 5555; 374 _ 1767; 382 _ 1641; 382 _ 2017; 382 _ 2188; 382 _ 3043; 382 _ 3533; 382 _ 3576; 382 _ 3724; 382 _ 3855; 382 _ 4093; 382 _ 4094; 382 _ 4552; 382 _ 4905; 382 _ 5067; 382 _ 5198; 382 _ 5257; 382 _ 5386; 397 _ 2023; 397 _ 3685; 422 _ 3043; 427 _ 5467; 432 _ 4093;439 _1767;444 _ 3590; 444 _ 5555; 448 _ 3590; 448 _ 5555; 449 _ 4963; 455 _ 3211; 473 _ 2725; 473 _ 2727; 473 _ 5238; 506 _ 1641; 506 _ 4093; 510 _ 3043; 510 _ 4905; 510 _ 5257; 530 _ 1767; 594 _ 3043; 598 _ 2023; 629 _ 673; 648 _ 1749; 648 _ 4493; 650 _ 5196; 664 _ 759; 665 _ 4493; 708 _ 1242; 712 _ 1641; 712 _ 4096; 716 _ 1239; 7511_ 1834; 751 - 2128; 751 _ 2144; 751 _ 2543; 751 _ 2725; 751 _ 2727; 751 _ 5238; 752 _ 2727; 757 _ 3250; 758 _ 2900; 758 _ 3935; 758 _ 5555; 842 _ 3552; 846 _ 1641; 846 _ 4096; 892 _ 1161; 892 _ 5257; 893 _ 2944; 921_ 3211; 973 _ 1834; 973 _ 2543; 973 _ 2727; 981 _ 1641; 981_ 4093; 992 _ 1767; 1122 _ 4093; 1142 _ 1834; 1142 _ 2543; 1142 _ 2727; 1159 _ 2943; 1170 _ 2900; 1170 _ 3540; 1170 _ 5555; 1174 _ 1270; 1174 _ 3211; 1185 _ 2023; 1185 _ 3685; 1216 _ 5221; 1237 _ 2900; 1237 _ 4220; 1237 _ 5555; 1239 _ 1767; 1239 _ 5090; 1239 _ 5549; 1240 _ 2943; 1242 1860; 1242 - 2941; 1242 _ 2944; 1242 _ 3312; 1242 _ 3537; 1242 _ 3540; 1242 _ 4716; 1258 _ 2941; 1258 _ 2944; 1259 _ 2941; 1259 _ 2944; 1287 _ 1767; 1288 _ 1971; 1422 1766; 1422 3211; 1489 _ 2900; 1489 _ 2941; 1489 _ 3540; 1489 _ 5555; 1534 _ 3043; 1564 _ 2462; 1591 _ 2725; 1591 _ 2727; 1629 _ 4493; 1633 2401; 1641 -_2157; 1641 -_4875; 1641 - 4963; 1641 -_5173; 1654 - 3211; 1675 _ 4811; 1694 _ 1766; 1694 _ 4383; 1694 _ 4875; 1731 _ 2900; 1731 _ 5555; 1731 _ 5561; 1744 _ 2900; 1744 _ 2941; 1744 _ 3312; 1744 _ 3540; 1744 _ 3590; 1744 _ 4714; 1744_ 5555; 1747 _ 2900; 17447 _ 2941; 17447 _ 3312; 1747 _ 3540; 1747 _ 4220; 1747 _ 4714; 1747 _ 5555; 1749 _ 3372; 1749 _ 5090; 1749 _ 5332; 1749 _ 5555; 1766 _ 2732; 1766 _ 4096; 1767 _ 2226; 1767 _ 4469; 1767 _ 4736; 1767 _ 4925; 1767 _ 4955; 1767 _ 5196; 1767 _ 5198; 1834 _ 2073; 1834 _ 3068; 1834 _ 4560; 1834 _ 5205; 1846 _ 2941; 1849_ 3312; 1853 _ 2941; 1853 _ 2944; 1853 _ 3540; 1859 _ 2023; 1859 _ 3685; 1860 _ 1951; 1860 _ 2406; 1860 _ 2695; 1860 _ 3222; 1911 _ 2732; 1911 _ 4076; 1911 _ 4096; 1911 _ 4779; 1911 _ 5206; 1911 _ 5419; 1920 _ 3043; 1920 _ 4905; 1920 _ 5198; 1951 _ 2943; 1951 _ 2944; 1972 _ 2049; 1972 _ 3189; 1972 _ 3211; 1987 _ 3043; 2023 _ 2889; 2023 _ 3506; 2023 _ 3979; 2023 _ 4425; 2023 _ 4872; 2049 _ 2732; 2049 _ 4091; 2049 _ 5206; 2073 _ 2128; 2073 _ 2141; 2073 _ 2143; 2073 _ 2144; 2073 _ 2312; 2073 _ 2325; 2073 _ 2332; 2073 _ 2543; 2073 _ 2679; 2073 _ 2725; 2073 _ 2727; 2073 _ 5238; 2115 _ 2943; 2128 _ 2330; 2128 _ 4587; 2139 _ 2941; 2139 _ 2944; 2143 _ 2943; 2157 _ 4093; 2157 _ 4094; 2158 _ 2329 ; 2186 _ 4905; 2226 _ 3272; 2226 _ 3306; 2250 _ 3590; 2250 _ 5555; 2250 _ 5561; 2255 _ 3590; 2257 _ 3590; 2261 _ 3590; 2263 _ 4714; 2310 _ 2941; 2312 _ 3538; 2313 _ 2943; 2313 _ 3358; 2324 _ 2943; 2324 _ 2944; 2326 _ 2943; 2326 _ 2944; 2329 _ 4388; 2330 _ 2725; 2330 _ 2727; 2330 _ 5238; 2332 _ 3538; 2384 _ 2943; 2384 _ 2944; 2398 _ 5555; 2401 _ 3782; 2406 _ 2943; 2406 _ 2944; 2411_ 2900; 2411 _ 2941; 2411 _ 3312; 2411 _ 3540; 2411 _ 4220; 2411 _ 4714; 2411 _ 5555; 2412 _ 2941; 2412 _ 3537; 2412 _ 3540; 2412 _ 4220; 2416 _ 3211; 2462 _ 3067; 2543 _ 4560; 2590 _ 2943; 2590 _ 2944; 2590 _ 3538; 2593 _ 2941; 2595 _ 3043; 2595 _ 4905; 2605 _ 3043; 2607 _ 2725; 2607 _ 2727; 2678 _ 3590; 2679 _ 2943; 2679 _ 3538; 2692 _ 2900; 2692 _ 2941; 2692 _ 3540; 2695 _ 2941; 2695 _ 2944; 2695 _ 3312; 2714 _ 4383; 2714 _ 4875; 2725 _ 2943; 2725 _ 3208; 2725 _ 3538; 2725 _ 4587; 2725 _ 4715; 2727 _ 3538; 2727 _ 3816; 2727 _ 4560; 2727 _ 4587; 2732 _ 3211; 2732 _ 4508; 2769 _ 4096; 2782 _ 4096; 2787 _ 3043; 2787 _ 4094; 2787 _ 4905; 2787 _ 5257; 2870 _ 3043; 2891_ 2943; 2900 _ 4495; 2900 _ 4592; 2900 _ 5051; 2901 _ 3043; 2941 _ 3220; 2941 _ 3531; 2941 _ 4592; 2941 _ 4660; 2941 _ 5355; 2941 _ 5529; 2943 _ 3222; 2943 _ 4235; 2943 _ 5050; 2943 _ 5238; 2944 _ 3222; 2944 _ 3320; 2944 _ 3531; 2944 _ 4502; 2944 _ 5355; 2944 _ 5529; 2983 _ 4963; 3043 _ 3327; 3043 _ 3674; 3043 _ 4231; 3043 _ 4959; 3043 _ 5328; 3189 _ 5254; 3211 _4076; 3211 _4091; 3211 _4097; 3211 _4751; 3211_ 4779; 3211 _ 5206; 3211 _ 5419; 3312 _ 4592; 3317 _ 5555; 3372 _ 4493; 3372 _ 4736; 3417 _ 4096; 3538 _ 3575; 3538 _ 5238; 3540 _ 4592; 3575 _ 4587; 3674 _ 4905; 4093 _ 4875; 4093 _ 4963; 4093 _ 5173; 4093 _ 5435; 4094 _ 4963; 4094 _ 5328; 4096 _ 4383; 4096 _ 4717; 4096 _ 4875; 4096 _ 5173; 4096 _ 5274; 4096 _ 5436; 4185 _ 4493; 4231 _ 4905; 4256 _ 5238; 4376 _ 5221; 4398 _ 4963; 4470 _ 5555; 4493 _ 4901; 4493 _ 5090; 4493 _ 5555; 4495 _ 5555; 4508 _4779; 4587 _ 5238; 4592 _ 4714; 4736 _ 5090; 4736 _ 5549; 4865 _ 5555; 4905 _ 4959; 4905 _ 5328

### Exemple 4. Identification de nouvelles isoformes de PTPRC, SORL1 et PSEN1

L'analyse des expressions relatives des probe sets associés aux gènes PTPRC, SORL1 et PSEN1 (cf paragraphe 9) a identifié des sondes de jonction intron-exon ou exon-intron révélatrices de la présence potentielle de nouvelles isoformes. Ces isoformes sont décrites ci-dessous.

PTPRC : La séquence SEQ ID NO : 3679 correspond à la jonction entre l'intron 5 et l'exon 6 du gène PTPRC. Les valeurs d'expression associées à cette sonde suggèrent qu'un événement d'épissage existe à l'extrémité 5' de l'exon 6. Afin de caractériser cet événement, des essais de RT-PCR ont été réalisés sur des pools de patients AD ou de contrôles, à l'aide d'une amorce située dans la terminaison 3' de l'intron 5 associée à une amorce correspondant aux exons 2 ou 4. Des amplicons sont visualisés sur electrophorèse (figure 4A). Le séquençage de ces amplicons révèle l'utilisation d'un nouveau site alternatif accepteur consensuel situé à 37 nucléotides en amont du site habituel de l'exon 6. L'ARN messager incluant cet événement pourrait encoder une protéine de 208 acides aminés (les 144 premiers correspondant à la partie N-terminale de CD45 (PTPRC). Les autres amplicons correspondent au même événement d'épissage associé à des délétions de l'exon 4 ou de l'exon 4 et de l'exon 5. Les RT-PCR suggérent que ce nouvel événement d'épissage est spécifiquement down-régulé dans la population AD. La séquence SEQ ID NO : 3698 correspond à l'extension de 37 nucléotides spécifiques de cet événement. La séquence SEQ ID NO : 3699 correspond à la nouvelle jonction entre l'exon 5 et le nouvel exon 6 rallongé en 5'.

SORL1 : La séquence SEQ ID NO : 4027 correspond à la jonction entre l'exon 41 et l'intron 41 du gène SORL1. Les valeurs d'expression associées à cette sonde suggèrent qu'un événement d'épissage existe à l'extrémité 3' de l'exon 41. Afin de caractériser cet événement, des essais de RT-PCR ont été réalisés sur des pools de patients AD ou de contrôles, à l'aide d'une amorce située dans la terminaison 5' de l'intron 41 associée à une amorce correspondant à 1 exon 42. Des amplicons sont visualisés sur electrophorèse (figure 4B). Le séquençage de ces amplicons révèle l'utilisation d'un nouveau site alternatif donneur consensuel situé à 28 nucléotides en aval du site habituel de l'exon 41. Un codon STOP est situé dans cette extension suggérant qu'un ARN messager associé coderait pour une protéine tronquée de 1870 acides aminés ne présentant plus le domaine transmembranaire et la queue cytoplasmique. La séquence SEQ ID NO : 4039 correspond à l'extension de 28 nucléotide spécifique de cet événement. La séquence SEQ ID NO : 4040 correspond à la nouvelle jonction entre le nouvel exon 41 rallongé en 3' et l'exon 42.

PSEN1 : Des essais de RT-PCR ont été réalisés sur des pools de patients AD ou de contrôles, à l'aide d'une amorce située dans un exon alternatif connu situé entre l'exon 3 et 4 de PSEN1 (représenté par l'EST AK122722.1), associée à une amorce correspondant à l'exon 4. Des amplicons sont visualisés sur electrophorèse (figure C). Le séquençage de ces amplicons révèle l'utilisation d'un nouveau site alternatif donneur situé dans l'exon alternatif 935 nucléotides en amont du site habituel cet exon alternatif (position 72704987-88 du chromosome 14). Les RT-PCR suggérent que ce nouvel événement d'épissage est spécifiquement exprimé dans la population AD. La séquence SEQ ID NO: 3515 correspond à l'exon alternatif tronqué. La séquence SEQ ID NO : 3516 correspond à la nouvelle jonction entre le nouvel exon alternatif tronqué et l'exon 4.

### Exemple 5. Discrimination entre patients AD, MCI et Contrôles

Des sous groupes de sondes ont été sélectionnés parmi les séquences SEQ ID NO : 1-5578, qui permettent de discriminer entre des patients atteints de AD et des sujets contrôles, ou entre des patients MCI et des sujets contrôles ou entre des sujets MCI et des patients AD.

La possibilité de distinguer les patients MCI et AD est particulièrement importante. En effet, 30-40% des sujets présentant des troubles de type MCI vont évoluer vers un AD. La possibilité d'identifier ces sujets permet, dès le stade MCI, de mettre en place les protocoles thérapeutiques efficaces pour AD, sans devoir attendre l'évolution de la maladie.

L'ARN de 9 patients atteints de MCI, 11 patients atteints de AD et 11 sujets Contrôles a été extrait à partir de sang veineux, reverse-transcrit et marqué à la biotine, puis hybridé sur des lames GWSA. Les lames ont ensuite été lavées, révélées puis scannées (scanner Affymetrix). Les fichiers .CEL ont ensuite été importés dans le logiciel d'analyse statistique Partek. L'ensemble de ces étapes a été réalisé comme décrit précédemment (voir pages 28 à 32).

Après normalisation des données, 5562 groupes de sondes parmi les 5578 ont été filtrées de la liste des 1 455 615 groupes de sondes composant le fichier. Les graphes correspondants sont donnés sur la figure 5.

Un test ANOVA 1-way permet ensuite d'identifier les groupes de sondes statistiquement significatifs des conditions AD vs MCI, AD vs C et C vs MCI. Trois fichiers ont ainsi été générés à partir des résultats du test ANOVA, comprenant les 84, 366 et 969 premiers ensembles de groupes de sondes statistiquement significatifs des conditions ADvsMCI, ADvsC et MCIvsC respectivement. Des groupes de sondes spécifiques des conditions ADvsMCI, ADvsC et MCIvsC ont ensuite été identifiés, ces données ont permis de générer le diagramme de Venn. Les groupes de sondes spécifiques de chacune de ces conditions sont particulièrement pertinents pour discriminer ces différents status cliniques des patients.

### 5.1. Sous-ensembles de groupes de sondes spécifiques de AD vs MCI.

| p-value CutOff | Test Anova | Probe sets spécifiques Ad vs MCI |
|---|---|---|
| 0.01 | 84 | 27 |
| 0.001 | 10 | 6 |

Les noms des groupes de sondes des sous ensembles 6PS et 27PS sont donnés dans le tableau ci-dessous.

| **Ensemble 1 : 6 PS ADvsMCI** | **SEQ ID** | **Ensemble 2 : 27 PS ADvsMCI** | **SEQ ID** |
|---|---|---|---|
| 399 .009.1-B | 2363 | 1019.005.1-T | 53 |
| 399.010.1-B | 2364 | 151888.002.1-B | 613 |
| 9467.009.1-E | 5307 | 1729.004.4-T | 697 |
| 9730.027.1-T | 5426 | 1803.025.1-B | 709 |
| 6934.032.1-B | 4218 | 23161.034.1-T | 1200 |
| 8603.000.5-X | 5063 | 23178.025.1-B | 1205 |
| | | 23312.005.1-F | 1339 |
| | | 284701.000.14-X | 1846 |
| | | 3486.024.1-C | 2157 |
| | | 3837.009.1-T | 2289 |
| | | **399.009.1-B** | 2363 |
| | | 4054.000.26-Z | 2429 |
| | | 442065.001.1-T | 2600 |
| | | 4670.020.1-B | 2639 |
| | | 4678.001.3-B | 2642 |
| | | 4820.026.1-B | 2712 |
| | | 56261.011.1-B | 3503 |
| | | 57680.013.1-B | 3636 |
| | | 57680.028.1-B | 3641 |
| | | 7919.040.1-B | 4529 |
| | | 80024.005.1-T | 4671 |
| | | 8556.016.1-T | 5055 |
| | | **8603.000.5-X** | 5063 |
| | | 9282.000.20-X | 5219 |
| | | **9467.009.1-E** | 5307 |
| | | 9611.052.1-F | 5354 |
| | | **9730.027.1-T** | 5426 |

L'ensemble 1 comprend ainsi:
- de 1 à 3 sondes, chevauchantes ou non, comprenant chacune tout ou une partie de la séquence SEQ ID NO : 2363 ou de sa séquence complémentaire ;
- de 1 à 3 sondes, chevauchantes ou non, comprenant chacune tout ou une partie de la séquence SEQ ID NO : 2364 ou de sa séquence complémentaire ;
- de 1 à 3 sondes, chevauchantes ou non, comprenant chacune tout ou une partie de la séquence SEQ ID NO : 5307 ou de sa séquence complémentaire ;
- de 1 à 3 sondes, chevauchantes ou non, comprenant chacune tout ou une partie de la séquence SEQ ID NO : 5426 ou de sa séquence complémentaire ;
- de 1 à 3 sondes, chevauchantes ou non, comprenant chacune tout ou une partie de la séquence SEQ ID NO : 4218 ou de sa séquence complémentaire ; et
- de 1 à 3 sondes, chevauchantes ou non, comprenant chacune tout ou une partie de la séquence SEQ ID NO : 5063 ou de sa séquence complémentaire.

De manière similaire, l'ensemble 2 comprend de 1-3 sondes comprenant tout ou partie de chaque séquence identifiée ci-dessus ou de son brin complémentaire.

Dans un exemple spécifique, l'ensemble 1 et l'ensemble 2 comprennent, respectivement, les sondes identifiées dans le tableau 3.

Le ratio des signaux d'intensité après déduction du bruit de fond entre les groupes de patients pour chaque groupe de sondes est donné ci-dessous.

| **Ensemble 1** | **Facteur de variation (AD vs. MCI)** |
|---|---|
| 9730.027.1-T | 1,65331 |
| 399.009.1-B | -1,92241 |
| 399.010.1-B | -1,95279 |
| 9467.009.1-E | 1,46337 |
| 8603.000.5-X | 1,76215 |
| 6934.032.1-B | 2,01881 |

| **Ensemble 2** | **Facteur de variation (AD vs. MCI)** |
|---|---|
| 9730.027.1-T | 1,65331 |
| 399.009.1-B | -1,92241 |
| 9467.009.1-E | 1,46337 |
| 8603.000.5-X | 1,76215 |
| 56261.011.1-B | -1,47064 |
| 442065.001.1-T | 1,31817 |
| 961 1.052.1-F | 1,55709 |
| 80024.005.1-T | -1,3923 |
| 23161.034.1-T | -1,73865 |
| 57680.013.1-B | 1,36043 |
| 8556.016.1-T | -1,414 |
| 4054.000.26-Z | 1,36387 |
| 23312.005.1-F | 1,4399 |
| 1019.005.1-T | -1,39817 |
| 151888.002.1-B | -1,8663 |
| 284701.000.14-X | -1,27164 |
| 1803.025.1-B | -1,50387 |
| 7919.040.1-B | -1,32556 |
| 4820.026.1-B | -1,44063 |
| 4678.001.3-B | -2,01683 |
| 23178.025.1-B | -2,34804 |
| 57680.028.1-B | 1,3464 |
| 4670.020.1-B | 1,76039 |
| 1729.004.4-T | 1,56877 |
| 3486.024.1-C | -1,30762 |
| 9282.000.20-X | 1,27867 |
| 3837.009.1-T | 1,2558 |

### 5.2. Sous-ensembles de groupes de sondes spécifiques de AD vs Contrôles.

| p-value CutOff | Test Anova | Probe sets spécifiques AD vs C |
|---|---|---|
| 0.01 | 366 | 75 |
| 0.001 | 54 | 18 |

Les noms des sondes du sous ensemble 18PS sont donnés dans le tableau ci-dessous.

| **Ensemble 3 : 18 PS ADvsC** | **SEQ ID** |
|---|---|
| 55652.007.1-D | 3325 |
| 140564.002.2-B | 568 |
| 10250.000.13-X | 67 |
| 55833.001.2-B | 3408 |
| 402483.005.1-B | 2406 |
| 55700.021.1-T | 3337 |
| 55636.003.1-T | 3323 |
| 2690.003.1-B | 1724 |
| 143.000.1-X | 573 |
| 54882.014.1-B | 3180 |
| 6813.005.1-T | 4178 |
| 23067.007.1-T | 1096 |
| 5527.004.1-F | 3288 |
| 9883.024.1-T | 5523 |
| 441124.002.1-T | 2590 |
| 79623.000.12-Y | 4558 |
| 5930.016.1-F | 3757 |
| 808.038.1-C | 4735 |

L'ensemble 3 comprend ainsi:
- de 1 à 3 sondes, chevauchantes ou non, comprenant chacune tout ou une partie de la séquence SEQ ID NO : 3325 ou de sa séquence complémentaire ;
- de 1 à 3 sondes, chevauchantes ou non, comprenant chacune tout ou une partie de la séquence SEQ ID NO : 568 ou de sa séquence complémentaire ;
- de 1 à 3 sondes, chevauchantes ou non, comprenant chacune tout ou une partie de la séquence SEQ ID NO : 67 ou de sa séquence complémentaire ;
- de 1 à 3 sondes, chevauchantes ou non, comprenant chacune tout ou une partie de la séquence SEQ ID NO : 3408 ou de sa séquence complémentaire ;
- de 1 à 3 sondes, chevauchantes ou non, comprenant chacune tout ou une partie de la séquence SEQ ID NO : 2406 ou de sa séquence complémentaire ;
- de 1 à 3 sondes, chevauchantes ou non, comprenant chacune tout ou une partie de la séquence SEQ ID NO : 3337 ou de sa séquence complémentaire ;
- de 1 à 3 sondes, chevauchantes ou non, comprenant chacune tout ou une partie de la séquence SEQ ID NO : 3323 ou de sa séquence complémentaire ;
- de 1 à 3 sondes, chevauchantes ou non, comprenant chacune tout ou une partie de la séquence SEQ ID NO : 1724 ou de sa séquence complémentaire ;
- de 1 à 3 sondes, chevauchantes ou non, comprenant chacune tout ou une partie de la séquence SEQ ID NO : 573 ou de sa séquence complémentaire ;
- de 1 à 3 sondes, chevauchantes ou non, comprenant chacune tout ou une partie de la séquence SEQ ID NO : 3180 ou de sa séquence complémentaire ;
- de 1 à 3 sondes, chevauchantes ou non, comprenant chacune tout ou une partie de la séquence SEQ ID NO : 4178 ou de sa séquence complémentaire ;
- de 1 à 3 sondes, chevauchantes ou non, comprenant chacune tout ou une partie de la séquence SEQ ID NO : 1096 ou de sa séquence complémentaire ;
- de 1 à 3 sondes, chevauchantes ou non, comprenant chacune tout ou une partie de la séquence SEQ ID NO : 3288 ou de sa séquence complémentaire ;
- de 1 à 3 sondes, chevauchantes ou non, comprenant chacune tout ou une partie de la séquence SEQ ID NO : 5523 ou de sa séquence complémentaire ;
- de 1 à 3 sondes, chevauchantes ou non, comprenant chacune tout ou une partie de la séquence SEQ ID NO : 2590 ou de sa séquence complémentaire ;
- de 1 à 3 sondes, chevauchantes ou non, comprenant chacune tout ou une partie de la séquence SEQ ID NO : 4558 ou de sa séquence complémentaire ;
- de 1 à 3 sondes, chevauchantes ou non, comprenant chacune tout ou une partie de la séquence SEQ ID NO : 3757 ou de sa séquence complémentaire ; et
- de 1 à 3 sondes, chevauchantes ou non, comprenant chacune tout ou une partie de la séquence SEQ ID NO : 4735 ou de sa séquence complémentaire.

Dans un exemple spécifique, l'ensemble 3 comprend les groupes de sondes identifiées dans le tableau 3.

Le ratio des signaux d'intensité après déduction du bruit de fond entre les groupes de patients pour chaque groupe de sondes est donné ci-dessous :

| **Ensemble 3** | **Facteur de variation (AD vs. C)** |
|---|---|
| 55652.007.1-D | -1,17521 |
| 143.000.1-X | 2,06971 |
| 55833.001.2-B | -1,75477 |
| 6813.005.1-T | -1,46021 |
| 23067.007.1-T | -1,92904 |
| 441124.002.1-T | -1,28295 |
| 402483.005.1-B | -1,2913 |
| 9883.024.1-T | -1,48769 |
| 55700.021.1-T | -1,51693 |
| 10250.000.13-X | -1,93485 |
| 79623.000.12-Y | -1,49027 |
| 140564.002.2-B | -1,7224 |
| 5930.016.1-F | 1,53811 |
| 55636.003.1-T | -1,54333 |
| 5527.004.1-F | 1,47689 |
| 54882.014.1-B | -1,33383 |
| 808.038.1-C | -1,62719 |
| 2690.003.1-B | -1,0767 |

### 5.3. Sous-ensembles de groupes de sondes spécifiques de MCI vs Contrôles.

| p-value CutOff | Test Anova | Probe sets spécifiques MCI vs C |
|---|---|---|
| 0.01 | 969 | 635 |
| 0.001 | 413 | 373 |
| 0.0001 | 149 | |
| 0.00001 | 33 | |

Les noms des groupes de sondes du sous ensemble 33PS sont donnés dans le tableau ci-dessous.

| **Ensemble 4 : 33PS** | **SEQ ID** |
|---|---|
| 1163.000.7-X | 42 |
| 10163.008.1-T | 45 |
| 10250.022.1-T | 79 |
| 10847.027.1-B | 275 |
| 11100.032.1-D | 349 |
| 2035.016.1-B | 830 |
| 2039.032.1-F | 841 |
| 2186.001.2-F | 894 |
| 2186.009.1-T | 895 |
| 2186.036.1-F | 902 |
| 23039.000.5-X | 1043 |
| 23327.025.4-B | 1356 |
| 23476.017.1-T | 1445 |
| 26057.008.1-B | 1668 |
| 27102.014.1-T | 1782 |
| 27350.004.1-B | 1800 |
| 283846.001.1-B | 1833 |
| 440345.006.14-B | 2542 |
| 4784.014.1-T | 2698 |
| 54856.007.1-F | 3156 |
| 54856.029.1-T | 3158 |
| 54856.033.1-F | 3160 |
| 55249.001.3-T | 3278 |
| 57610.000.6-X | 3606 |
| 6229.003.1-C | 3826 |
| 65125.000.20-X | 3967 |
| 65125.010.1-F | 3972 |
| 65125.025.1-T | 3976 |
| 81608.009.1-F | 4810 |
| 8289.000.2-X | 4864 |
| 8289.004.1-T | 4865 |
| 8289.008.1-B | 4867 |
| 8289.009.1-F | 4869 |

L'ensemble 4 comprend ainsi, pour chaque séquence identifiée, de 1 à 3 sondes, chevauchantes ou non, comprenant chacune tout ou partie de la séquence ou de sa séquence complémentaire.

Les études de répartition sont présentées sur la figure 6. Elles montrent notamment que l'ensemble 1 composé de 6 groupes de sondes (6PS) tel que défini ci-dessus permet de discriminer les patients MCI et AD (Fig. 6B). Elles montrent également que l'ensemble de 18 groupes de sondes tel que défini ci-dessus permet de discriminer les patients AD et Contrôles, c'est-à-dire permet d'identifier, à partir d'une population de sujets ceux qui présentent un AD (Fig. 6A).

### Exemple 6. Protocole de détection

Les étapes du test diagnostique sont les suivantes:

L'échantillon est préparé par une étape d'extraction de l'ARN à partir d'échantillons de sang collectés dans des tubes de prélèvement sanguin Paxgene. Ces tubes contiennent un additif qui stabilise le profil de transcription de gènes in vivo en réduisant la dégradation d'ARN in vitro et en minimisant l'induction de gènes. Lorsqu'ils sont utilisés avec PaxgeneTM Blood RNA Kit, les échantillons prélevés permettent la détection et la quantification exactes du niveau de transcription de gènes. Après extraction, une étape de contrôle qualitatif et quantitatif de l'ARN est réalisée.

L'ARN est retro-transcrit, amplifié linéairement puis fragmenté, marqué à la biotine et enfin hybridé sur des Biopuces regroupant les sondes de l'invention. Les lames sont ensuite lavées, l'hybridation révélée puis et scannées pour mesurer le niveau d'hybridation sur chaque sonde. Les résultats d'hybridations sont importés et analysés dans le logiciel d'analyse statistique partek.

L'application de la signature donne une réponse binaire qui permet de placer le patient testé dans la catégorie AD ou dans la catégorie contrôle.

**Tableau 3**

| **Ensemble de groupes de sondes (probe sets)** | **Séquence de référence (sequence cible ou target sequence (SEQ ID)** | **Sonde spécifique (SEQ ID)** |
|---|---|---|
| Ensemble 3 | 67 | 5770 |
| | 67 | 5771 |
| | 67 | 5772 |
| | 568 | 7210 |
| | 568 | 7211 |
| | 568 | 7212 |
| | 573 | 7225 |
| | 573 | 7226 |
| | 573 | 7227 |
| | 1096 | 8726 |
| | 1096 | 8727 |
| | 1096 | 8728 |
| | 1724 | 10549 |
| | 1724 | 10550 |
| | 1724 | 10551 |
| | 2406 | 12505 |
| | 2406 | 12506 |
| | 2406 | 12507 |
| | 2590 | 13021 |
| | 2590 | 13022 |
| | 2590 | 13023 |
| | 3180 | 14704 |
| | 3180 | 14705 |
| | 3180 | 14706 |
| | 3288 | 15011 |
| | 3288 | 15012 |
| | 3288 | 15013 |
| | 3323 | 15113 |
| | 3323 | 15114 |
| | 3323 | 15115 |
| | 3325 | 15119 |
| | 3325 | 15120 |
| | 3325 | 15121 |
| | 3337 | 15153 |
| | 3337 | 15154 |
| | 3337 | 15155 |
| | 3408 | 15355 |
| | 3408 | 15356 |
| | 3408 | 15357 |
| | 3757 | 16359 |
| | 3757 | 16360 |
| | 3757 | 16361 |
| | 4178 | 17560 |
| | 4178 | 17561 |
| | 4178 | 17562 |
| | 4558 | 18636 |
| | 4558 | 18637 |
| | 4558 | 18638 |
| | 4735 | 19146 |
| | 4735 | 19147 |
| | 4735 | 19148 |
| | 5523 | 21401 |
| | 5523 | 21402 |
| | 5523 | 21403 |
| Ensemble 2 | 53 | 5728 |
| | 53 | 5729 |
| | 53 | 5730 |
| | 613 | 7337 |
| | 613 | 7338 |
| | 613 | 7339 |
| | 697 | 7571 |
| | 697 | 7572 |
| | 697 | 7573 |
| | 709 | 7606 |
| | 709 | 7607 |
| | 1200 | 9033 |
| | 1200 | 9034 |
| | 1200 | 9035 |
| | 1205 | 9046 |
| | 1205 | 9047 |
| | 1205 | 9048 |
| | 1339 | 9433 |
| | 1339 | 9434 |
| | 1339 | 9435 |
| | 1846 | 10897 |
| | 1846 | 10898 |
| | 1846 | 10899 |
| | 2157 | 11798 |
| | 2157 | 11799 |
| | 2157 | 11800 |
| | 2289 | 12184 |
| | 2289 | 12185 |
| | 2289 | 12186 |
| | 2429 | 12573 |
| | 2429 | 12574 |
| | 2429 | 12575 |
| | 2600 | 13049 |
| | 2600 | 13050 |
| | 2600 | 13051 |
| | 2639 | 13160 |
| | 2642 | 13167 |
| | 2712 | 13369 |
| | 2712 | 13370 |
| | 2712 | 13371 |
| | 3503 | 15630 |
| | 3503 | 15631 |
| | 3636 | 16007 |
| | 3636 | 16008 |
| | 3636 | 16009 |
| | 3641 | 16022 |
| | 3641 | 16023 |
| | 3641 | 16024 |
| | 4529 | 18553 |
| | 4529 | 18554 |
| | 4671 | 18961 |
| | 4671 | 18962 |
| | 4671 | 18963 |
| | 5055 | 20051 |
| | 5055 | 20052 |
| | 5055 | 20053 |
| | 5219 | 20522 |
| | 5219 | 20523 |
| | 5219 | 20524 |
| | 5354 | 20912 |
| | 5354 | 20913 |
| | 5354 | 20914 |
| Ensemble 4 | 42 | 5695 |
| | 42 | 5696 |
| | 42 | 5697 |
| | 45 | 5704 |
| | 45 | 5705 |
| | 45 | 5706 |
| | 79 | 5806 |
| | 79 | 5807 |
| | 79 | 5808 |
| | 275 | 6379 |
| | 275 | 6380 |
| | 275 | 6381 |
| | 349 | 6590 |
| | 349 | 6591 |
| | 349 | 6592 |
| | 830 | 7951 |
| | 830 | 7952 |
| | 830 | 7953 |
| | 841 | 7984 |
| | 841 | 7985 |
| | 841 | 7986 |
| | 894 | 8131 |
| | 894 | 8132 |
| | 894 | 8133 |
| | 895 | 8134 |
| | 895 | 8135 |
| | 895 | 8136 |
| | 902 | 8153 |
| | 902 | 8154 |
| | 902 | 8155 |
| | 1043 | 8568 |
| | 1043 | 8569 |
| | 1043 | 8570 |
| | 1356 | 9484 |
| | 1356 | 9485 |
| | 1356 | 9486 |
| | 1445 | 9746 |
| | 1445 | 9747 |
| | 1445 | 9748 |
| | 1668 | 10392 |
| | 1668 | 10393 |
| | 1668 | 10394 |
| | 1782 | 10714 |
| | 1782 | 10715 |
| | 1782 | 10716 |
| | 1800 | 10765 |
| | 1800 | 10766 |
| | 1800 | 10767 |
| | 1833 | 10859 |
| | 1833 | 10860 |
| | 2542 | 12889 |
| | 2542 | 12890 |
| | 2542 | 12891 |
| | 2698 | 13327 |
| | 2698 | 13328 |
| | 2698 | 13329 |
| | 3156 | 14634 |
| | 3156 | 14635 |
| | 3156 | 14636 |
| | 3158 | 14640 |
| | 3158 | 14641 |
| | 3158 | 14642 |
| | 3160 | 14646 |
| | 3160 | 14647 |
| | 3160 | 14648 |
| | 3278 | 14983 |
| | 3278 | 14984 |
| | 3278 | 14985 |
| | 3606 | 15919 |
| | 3606 | 15920 |
| | 3606 | 15921 |
| | 3826 | 16550 |
| | 3826 | 16551 |
| | 3826 | 16552 |
| | 3967 | 16956 |
| | 3967 | 16957 |
| | 3967 | 16958 |
| | 3972 | 16971 |
| | 3972 | 16972 |
| | 3972 | 16973 |
| | 3976 | 16983 |
| | 3976 | 16984 |
| | 3976 | 16985 |
| | 4810 | 19364 |
| | 4810 | 19365 |
| | 4810 | 19366 |
| | 4864 | 19519 |
| | 4864 | 19520 |
| | 4864 | 19521 |
| | 4865 | 19522 |
| | 4865 | 19523 |
| | 4865 | 19524 |
| | 4867 | 19528 |
| | 4867 | 19529 |
| | 4867 | 19530 |
| | 4869 | 19534 |
| | 4869 | 19535 |
| | 4869 | 19536 |
| Ensemble 1 | 2363 | 12390 |
| | 2363 | 12391 |
| | 2363 | 12392 |
| | 2364 | 12393 |
| | 4218 | 17670 |
| | 5063 | 20074 |
| | 5063 | 20075 |
| | 5063 | 20076 |
| | 5307 | 20777 |
| | 5307 | 20778 |
| | 5307 | 20779 |
| | 5426 | 21121 |
| | 5426 | 21122 |
| | 5426 | 21123 |

## Revendications

1. Méthode pour détecter la maladie d'Alzheimer chez un mammifère, comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.
une « partie » de séquence désignant une région de 15 à 50 nucléotides consécutifs de cette séquence,
pour obtenir un profil d'hybridation, le profil d'hybridation étant caractéristique de la maladie d'Alzheimer chez ce mammifère.

2. Méthode pour discriminer les patients atteints d'Alzheimer de sujets présentant des troubles cognitifs légers comprenant la mise en contact, dans des conditions permettant une hybridation entre séquences complémentaires, des acides nucléiques issus d'un échantillon de sang du mammifère et d'un ensemble de sondes, l'ensemble de sondes comprenant au moins :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.
une « partie » de séquence désignant une région de 15 à 50 nucléotides consécutifs de cette séquence,
pour obtenir un profil d'hybridation, le profil d'hybridation étant caractéristique de la maladie d'Alzheimer chez ce mammifère.

3. Méthode selon la revendication 1 ou 2, **caractérisée en ce que** l'ensemble de sondes comprend:
- de 1 à 3 sondes, chevauchantes ou non, comprenant chacune tout ou une partie de la séquence SEQ ID NO : 2363 ou de sa séquence complémentaire ;
- de 1 à 3 sondes, chevauchantes ou non, comprenant chacune tout ou une partie de la séquence SEQ ID NO : 2364 ou de sa séquence complémentaire ;
- de 1 à 3 sondes, chevauchantes ou non, comprenant chacune tout ou une partie de la séquence SEQ ID NO : 5307 ou de sa séquence complémentaire ;
- de 1 à 3 sondes, chevauchantes ou non, comprenant chacune tout ou une partie de la séquence SEQ ID NO : 5426 ou de sa séquence complémentaire ;
- de 1 à 3 sondes, chevauchantes ou non, comprenant chacune tout ou une partie de la séquence SEQ ID NO : 4218 ou de sa séquence complémentaire ; et
- de 1 à 3 sondes, chevauchantes ou non, comprenant chacune tout ou une partie de la séquence SEQ ID NO : 5063 ou de sa séquence complémentaire,
une « partie » de séquence désignant une région de 15 à 50 nucléotides consécutifs de cette séquence.

4. Méthode selon l'une quelconque des revendications 1 à 3, **caractérisée en ce que** l'échantillon est mis au contact d'amorces nucléiques spécifiques de chacune des séquences SEQ ID Nos : 2363, 2364, 5307, 5426, 4218 et 5063, et la présence ou le risque de développer la maladie d'Alzheimer est déterminé par amplification sélective.

5. Méthode selon l'une quelconque des revendications précédentes, **caractérisée en ce que** l'échantillon est un échantillon issu de sang, de préférence un échantillon de sang total.

6. Méthode selon la revendication 1, pour différentier la présence de la maladie d'Alzheimer d'autres types de démences telles que les démences à corps de Lewy, les démences fronto-temporales, les démences dues à la maladie de Parkinson, les démences vasculaires et les démences mixtes.

7. Produit comprenant un support sur lequel est immobilisé un ensemble de (groupes de) sondes comprenant au moins :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.
- une « partie » de séquence désignant une région de 15 à 50 nucléotides consécutifs de cette séquence.

8. Produit selon la revendication 7, **caractérisé en ce que** la séquence des sondes est telle que définie dans le tableau 3.

9. Kit comprenant un compartiment ou conteneur comprenant au moins :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.
une « partie » de séquence désignant une région de 15 à 50 nucléotides consécutifs de cette séquence.

10. Kit comprenant un produit selon l'une des revendications 7 ou 8 et des réactifs pour une réaction d'hybridation.

11. Utilisation d'un produit ou kit selon l'une des revendications 7 à 10 pour la détection in vitro ou ex vivo de la présence de la maladie d'Alzheimer chez un sujet.

12. Utilisation d'un ensemble de (groupes de) sondes comprenant au moins :
- une sonde comprenant tout ou partie de la séquence SEQ ID NO : 2363 ou de sa séquence complémentaire ;
- une sonde comprenant tout ou partie de la séquence SEQ ID NO : 2364 ou de sa séquence complémentaire ;
- une sonde comprenant tout ou partie de la séquence SEQ ID NO : 5307 ou de sa séquence complémentaire ;
- une sonde comprenant tout ou partie de la séquence SEQ ID NO : 5426 ou de sa séquence complémentaire ;
- une sonde comprenant tout ou partie de la séquence SEQ ID NO : 4218 ou de sa séquence complémentaire ; et
- une sonde comprenant tout ou partie de la séquence SEQ ID NO : 5063 ou de sa séquence complémentaire.
une « partie » de séquence désignant une région de 15 à 50 nucléotides consécutifs de cette séquence,
- pour détecter in vitro ou ex vivo la présence de la maladie d'Alzheimer chez un sujet ou pour discriminer les patients AD des patients atteints de troubles cognitifs légers.

## Claims

1. Method for detecting Alzheimer's disease in a mammal, comprising contacting, under conditions allowing hybridization between complementary sequences, the nucleic acids from a mammalian blood sample and a set of probes, the set of probes comprising at least:
- a probe comprising all or part of SEQ ID NO: 2363 or its complementary sequence;
- a probe comprising all or part of SEQ ID NO: 2364 or its complementary sequence;
- a probe comprising all or part of SEQ ID NO: 5307 or its complementary sequence;
- a probe comprising all or part of SEQ ID NO: 5426 or its complementary sequence;
- a probe comprising all or part of SEQ ID NO: 4218 or its complementary sequence; and
- a probe comprising all or part of SEQ ID NO: 5063 or its complementary sequence,
a "part of" a sequence designating a region of 15 to 50 consecutive nucleotides of said sequence,
in order to obtain a hybridization profile, the hybridization profile being characteristic of Alzheimer's disease in this mammal.

2. Method for discriminating patients with Alzheimer's from subjects with mild cognitive impairment, comprising contacting, under conditions allowing hybridization between complementary sequences, the nucleic acids from a mammalian blood sample and a set of probes, the set of probes comprising at least:
- a probe comprising all or part of SEQ ID NO: 2363 or its complementary sequence;
- a probe comprising all or part of SEQ ID NO: 2364 or its complementary sequence;
- a probe comprising all or part of SEQ ID NO: 5307 or its complementary sequence;
- a probe comprising all or part of SEQ ID NO: 5426 or its complementary sequence;
- a probe comprising all or part of SEQ ID NO: 4218 or its complementary sequence; and
- a probe comprising all or part of SEQ ID NO: 5063 or its complementary sequence,
a "part of" a sequence designating a region of 15 to 50 consecutive nucleotides of said sequence,
in order to obtain a hybridization profile, the hybridization profile being characteristic of Alzheimer's disease in this mammal.

3. Method according to claim 1 or 2, **characterized in that** the set of probes comprises:
- from 1 to 3 probes, overlapping or not, each comprising all or part of SEQ ID NO: 2363 or its complementary sequence;
- from 1 to 3 probes, overlapping or not, each comprising all or part of SEQ ID NO: 2364 or its complementary sequence;
- from 1 to 3 probes, overlapping or not, each comprising all or part of SEQ ID NO: 5307 or its complementary sequence;
- from 1 to 3 probes, overlapping or not, each comprising all or part of SEQ ID NO: 5426 or its complementary sequence;
- from 1 to 3 probes, overlapping or not, each comprising all or part of SEQ ID NO: 4218 or its complementary sequence; and
- from 1 to 3 probes, overlapping or not, each comprising all or part of SEQ ID NO: 5063 or its complementary sequence,
a "part of" a sequence designating a region of 15 to 50 consecutive nucleotides of said sequence.

4. Method according to one of claims 1 to 3, **characterized in that** the sample is contacted with nucleic acid primers specific of each of the sequences SEQ ID NOs: 2363, 2364, 5307, 5426, 4218 and 5063, and the presence or risk of developing Alzheimer's disease is determined by selective amplification.

5. Method according to any one of the preceding claims, **characterized in that** the sample is a blood sample, preferably a whole blood sample.

6. Method according to claim 1, to differentiate the presence of Alzheimer's disease from other types of dementia such as dementia with Lewy bodies, frontotemporal dementia, dementia due to Parkinson's disease, vascular dementia and mixed dementia.

7. A product comprising a support on which is immobilized a set of (groups of) probes comprising at least:
- a probe comprising all or part of SEQ ID NO: 2363 or its complementary sequence;
- a probe comprising all or part of SEQ ID NO: 2364 or its complementary sequence;
- a probe comprising all or part of SEQ ID NO: 5307 or its complementary sequence;
- a probe comprising all or part of SEQ ID NO: 5426 or its complementary sequence;
- a probe comprising all or part of SEQ ID NO: 4218 or its complementary sequence; and
- a probe comprising all or part of SEQ ID NO: 5063 or its complementary sequence,
a "part of" a sequence designating a region of 15 to 50 consecutive nucleotides of said sequence.

8. Product according to claim 7, **characterized in that** the sequence of the probes is as defined in Table 3.

9. Kit comprising a compartment or container comprising at least :
- a probe comprising all or part of SEQ ID NO: 2363 or its complementary sequence;
- a probe comprising all or part of SEQ ID NO: 2364 or its complementary sequence;
- a probe comprising all or part of SEQ ID NO: 5307 or its complementary sequence;
- a probe comprising all or part of SEQ ID NO: 5426 or its complementary sequence;
- a probe comprising all or part of SEQ ID NO: 4218 or its complementary sequence; and
- a probe comprising all or part of SEQ ID NO: 5063 or its complementary sequence,
a "part of" a sequence designating a region of 15 to 50 consecutive nucleotides of said sequence.

10. Kit comprising a product according to any one of claims 7 or 8 and reagents for a hybridization reaction.

11. Use of a product or kit according to one of claims 7 to 10 for the *in vitro* or *ex vivo* detection of the presence of Alzheimer's disease in a subject.

12. Use of a set of (groups of) probes, comprising at least:
- a probe comprising all or part of SEQ ID NO: 2363 or its complementary sequence;
- a probe comprising all or part of SEQ ID NO: 2364 or its complementary sequence;
- a probe comprising all or part of SEQ ID NO: 5307 or its complementary sequence;
- a probe comprising all or part of SEQ ID NO: 5426 or its complementary sequence;
- a probe comprising all or part of SEQ ID NO: 4218 or its complementary sequence; and
- a probe comprising all or part of SEQ ID NO: 5063 or its complementary sequence,
a "part of" a sequence designating a region of 15 to 50 consecutive nucleotides of said sequence,
to detect *in vitro* or *ex vivo* the presence of Alzheimer's disease in a subject or to discriminate AD patients from patients with mild cognitive impairment.

## Patentansprüche

1. Verfahren zum Nachweis der Alzheimer-Krankheit bei einem Säuger, umfassend Inkontaktbringen von Nukleinsäuren, die von einer Blutprobe des Säugers stammen, und einem Sondensatz unter Bedingungen, die eine Hybridisierung von komplementären Sequenzen erlauben, wobei der Sondensatz wenigstens umfasst:
- eine Sonde, umfassend die gesamte oder einen Abschnitt der Sequenz SEQ ID NR: 2363 oder die komplementäre Sequenz davon;
- eine Sonde, umfassend die gesamte oder einen Abschnitt der Sequenz SEQ ID NR: 2364 oder die komplementäre Sequenz davon;
- eine Sonde, umfassend die gesamte oder einen Abschnitt der Sequenz SEQ ID NR: 5307 oder die komplementäre Sequenz davon;
- eine Sonde, umfassend die gesamte oder einen Abschnitt der Sequenz SEQ ID NR: 5426 oder die komplementäre Sequenz davon;
- eine Sonde, umfassend die gesamte oder einen Abschnitt der Sequenz SEQ ID NR: 4218 oder die komplementäre Sequenz davon; und
- eine Sonde, umfassend die gesamte oder einen Abschnitt der Sequenz SEQ ID NR: 5063 oder die komplementäre Sequenz davon;
wobei ein "Abschnitt" der Sequenz einen Bereich von 15 bis 50 aufeinanderfolgenden Nukleotiden dieser Sequenz bezeichnet,
um ein Hybridisierungsprofil zu erhalten, wobei das Hybridisierungsprofil für die Alzheimer-Krankheit bei diesem Säuger charakteristisch ist.

2. Verfahren zur Unterscheidung der Patienten, die an der Alzheimer-Krankheit leiden, von Personen, die leichte kognitive Störungen aufweisen, umfassend Inkontaktbringen von Nukleinsäuren, die von einer Blutprobe des Säugers stammen, und einem Sondensatz unter Bedingungen, die eine Hybridisierung von komplementären Sequenzen erlauben, wobei der Sondensatz wenigstens umfasst:
- eine Sonde, umfassend die gesamte oder einen Abschnitt der Sequenz SEQ ID NR: 2363 oder die komplementäre Sequenz davon;
- eine Sonde, umfassend die gesamte oder einen Abschnitt der Sequenz SEQ ID NR: 2364 oder die komplementäre Sequenz davon;
- eine Sonde, umfassend die gesamte oder einen Abschnitt der Sequenz SEQ ID NR: 5307 oder die komplementäre Sequenz davon;
- eine Sonde, umfassend die gesamte oder einen Abschnitt der Sequenz SEQ ID NR: 5426 oder die komplementäre Sequenz davon;
- eine Sonde, umfassend die gesamte oder einen Abschnitt der Sequenz SEQ ID NR: 4218 oder die komplementäre Sequenz davon; und
- eine Sonde, umfassend die gesamte oder einen Abschnitt der Sequenz SEQ ID NR: 5063 oder die komplementäre Sequenz davon;
wobei ein "Abschnitt" der Sequenz einen Bereich von 15 bis 50 aufeinanderfolgenden Nukleotiden dieser Sequenz bezeichnet,
um ein Hybridisierungsprofil zu erhalten, wobei das Hybridisierungsprofil für die Alzheimer-Krankheit bei diesem Säuger charakteristisch ist.

3. Verfahren gemäß Anspruch 1 oder 2, **dadurch gekennzeichnet, dass** der Sondensatz umfasst:
- 1 bis 3 Sonden, eventuell überlappend, umfassend jeweils die gesamte oder einen Abschnitt der Sequenz SEQ ID NR: 2363 oder die komplementäre Sequenz davon;
- 1 bis 3 Sonden, eventuell überlappend, umfassend jeweils die gesamte oder einen Abschnitt der Sequenz SEQ ID NR: 2364 oder die komplementäre Sequenz davon;
- 1 bis 3 Sonden, eventuell überlappend, umfassend jeweils die gesamte oder einen Abschnitt der Sequenz SEQ ID NR: 5307 oder die komplementäre Sequenz davon;
- 1 bis 3 Sonden, eventuell überlappend, umfassend jeweils die gesamte oder einen Abschnitt der Sequenz SEQ ID NR: 5426 oder die komplementäre Sequenz davon;
- 1 bis 3 Sonden, eventuell überlappend, umfassend jeweils die gesamte oder einen Abschnitt der Sequenz SEQ ID NR: 4218 oder die komplementäre Sequenz davon; und
- 1 bis 3 Sonden, eventuell überlappend, umfassend jeweils die gesamte oder einen Abschnitt der Sequenz SEQ ID NR: 5063 oder die komplementäre Sequenz davon;
wobei ein "Abschnitt" der Sequenz einen Bereich von 15 bis 50 aufeinanderfolgenden Nukleotiden dieser Sequenz bezeichnet.

4. Verfahren gemäß einem der Ansprüche 1 bis 3, **dadurch gekennzeichnet, dass** die Probe mit Nukleinsäure-Primem in Kontakt gebracht wird, die für jede der Sequenzen SEQ ID NR: 2363, 2364, 5307, 5426, 4218 und 5063 spezifisch sind, und das Vorliegen oder das Risiko einer Entwicklung der Alzheimer-Krankheit durch eine selektive Amplifikation bestimmt wird.

5. Verfahren gemäß einem der vorhergehenden Ansprüche, **dadurch gekennzeichnet, dass** die Probe eine aus Blut gewonnene Probe, vorzugsweise aus Gesamtblut gewonnene Probe, ist.

6. Verfahren gemäß Anspruch 1, um das Vorliegen der Alzheimer-Krankheit von anderen Demenz-Typen, wie Lewy-Körperchen-Demenzen, fronto-temporalen Demenzen, Demenzen, die auf die Parkinson-Krankheit zurückzuführen sind, vaskulären Demenzen und gemischten Demenzen, zu unterscheiden.

7. Produkt, umfassend einen Träger, auf dem ein Satz von (Gruppen von) Sonden immobilisiert ist, umfassend wenigstens:
- eine Sonde, umfassend die gesamte oder einen Abschnitt der Sequenz SEQ ID NR: 2363 oder die komplementäre Sequenz davon;
- eine Sonde, umfassend die gesamte oder einen Abschnitt der Sequenz SEQ ID NR: 2364 oder die komplementäre Sequenz davon;
- eine Sonde, umfassend die gesamte oder einen Abschnitt der Sequenz SEQ ID NR: 5307 oder die komplementäre Sequenz davon;
- eine Sonde, umfassend die gesamte oder einen Abschnitt der Sequenz SEQ ID NR: 5426 oder die komplementäre Sequenz davon;
- eine Sonde, umfassend die gesamte oder einen Abschnitt der Sequenz SEQ ID NR: 4218 oder die komplementäre Sequenz davon; und
- eine Sonde, umfassend die gesamte oder einen Abschnitt der Sequenz SEQ ID NR: 5063 oder die komplementäre Sequenz davon;
- wobei ein "Abschnitt" der Sequenz einen Bereich von 15 bis 50 aufeinanderfolgenden Nukleotiden dieser Sequenz bezeichnet.

8. Produkt gemäß Anspruch 7, **dadurch gekennzeichnet, dass** die Sequenz der Sonden wie in Tabelle 3 definiert ist.

9. Kit, umfassend eine Kammer oder einen Behälter, umfassend wenigstens:
- eine Sonde, umfassend die gesamte oder einen Abschnitt der Sequenz SEQ ID NR: 2363 oder die komplementäre Sequenz davon;
- eine Sonde, umfassend die gesamte oder einen Abschnitt der Sequenz SEQ ID NR: 2364 oder die komplementäre Sequenz davon;
- eine Sonde, umfassend die gesamte oder einen Abschnitt der Sequenz SEQ ID NR: 5307 oder die komplementäre Sequenz davon;
- eine Sonde, umfassend die gesamte oder einen Abschnitt der Sequenz SEQ ID NR: 5426 oder die komplementäre Sequenz davon;
- eine Sonde, umfassend die gesamte oder einen Abschnitt der Sequenz SEQ ID NR: 4218 oder die komplementäre Sequenz davon; und
- eine Sonde, umfassend die gesamte oder einen Abschnitt der Sequenz SEQ ID NR: 5063 oder die komplementäre Sequenz davon;
wobei ein "Abschnitt" der Sequenz einen Bereich von 15 bis 50 aufeinanderfolgenden Nukleotiden dieser Sequenz bezeichnet.

10. Kit, umfassend ein Produkt gemäß einem der Ansprüche 7 oder 8 und Reagenzien für eine Hybridisierungsreaktion.

11. Verwendung eines Produkts oder Kits gemäß einem der Ansprüche 7 bis 10 zum ex vivo oder in vivo Nachweis des Vorliegens der Alzheimer-Krankheit bei einer Person.

12. Verwendung eines Satzes von (Gruppen von) Sonden, umfassend
- eine Sonde, umfassend die gesamte oder einen Abschnitt der Sequenz SEQ ID NR: 2363 oder die komplementäre Sequenz davon;
- eine Sonde, umfassend die gesamte oder einen Abschnitt der Sequenz SEQ ID NR: 2364 oder die komplementäre Sequenz davon;
- eine Sonde, umfassend die gesamte oder einen Abschnitt der Sequenz SEQ ID NR: 5307 oder die komplementäre Sequenz davon;
- eine Sonde, umfassend die gesamte oder einen Abschnitt der Sequenz SEQ ID NR: 5426 oder die komplementäre Sequenz davon;
- eine Sonde, umfassend die gesamte oder einen Abschnitt der Sequenz SEQ ID NR: 4218 oder die komplementäre Sequenz davon; und
- eine Sonde, umfassend die gesamte oder einen Abschnitt der Sequenz SEQ ID NR: 5063 oder die komplementäre Sequenz davon;
wobei ein "Abschnitt" der Sequenz einen Bereich von 15 bis 50 aufeinanderfolgenden Nukleotiden dieser Sequenz bezeichnet,
- um das Vorliegen der Alzheimer-Krankheit bei einer Person in vitro oder ex vivo nachzuweisen oder um die AD-Patienten von Patienten, die an leichten kognitiven Störungen leiden, zu unterscheiden.
